(19) Europäisches Patentamt

European Patent Office

Office européen des brevets

(11) **EP 1 510 959 A2**

(12) **EUROPEAN PATENT APPLICATION**

(43) Date of publication:
**02.03.2005 Bulletin 2005/09**

(51) Int Cl.$^7$: **G06F 19/00**, G01N 33/68

(21) Application number: **04016539.1**

(22) Date of filing: **12.08.2002**

| | |
|---|---|
| (84) Designated Contracting States:<br>**AT BE BG CH CY CZ DE DK EE ES FI FR GB GR IE IT LI LU MC NL PT SE SK TR**<br>Designated Extension States:<br>**AL LT LV MK**<br><br>(30) Priority: **10.08.2001 US 311545 P**<br>          **10.08.2001 US 927790**<br>          **25.09.2001 US 324899 P**<br>          **25.01.2002 US 351937 P**<br>          **25.01.2002 US 352103 P**<br><br>(62) Document number(s) of the earlier application(s) in accordance with Art. 76 EPC:<br>**02763433.6 / 1 432 980**<br><br>(71) Applicant: **Xencor**<br>**Monrovia, CA 91016 (US)**<br><br>(72) Inventors:<br> &bull; **Bentzien, Joerg**<br>  **White Plains, NY 10603 (US)** | &bull; **Dahiyat, Bassil I.**<br>  **Altadena, CA 91001 (US)**<br>&bull; **Desjarlais, John**<br>  **Pasadena, CA 91104 (US)**<br>&bull; **Hayes, Robert, J.**<br>  **Radnor, PA 19087 (US)**<br>&bull; **Vielmetter, Jost**<br>  **Altadena, CA 91001 (US)**<br><br>(74) Representative: **Kiddle, Simon John et al**<br>  **Mewburn Ellis LLP**<br>  **York House,**<br>  **23 Kingsway**<br>  **London WC2B 6HP (GB)**<br><br>Remarks:<br>  This application was filed on 14 - 07 - 2004 as a divisional application to the application mentioned under INID code 62. |

(54) **Protein design automation for protein libraries**

(57)    The invention relates to the use of protein design automation (PDA™) to generate computationally pre-screened secondary libraries of proteins, and to methods and compositions utilizing the libraries.

EP 1 510 959 A2

**Description**

This application is a continuing application of Serial No. 09/927,790, filed on August 10, 2001 and claims the benefit of the filing dates of Serial Nos. 60/311,545, filed on August 10, 2001, 60/324,899, filed on September 25, 2001, 60/351,937, filed on January 25, 2002, and 60/352,103, filed on January 25, 2002.

FIELD OF THE INVENTION

The invention relates to the use of a variety of computation methods, including protein design automation (PDA™) technology to generate computationally prescreened secondary libraries of proteins, and to methods of making and methods and compositions utilizing the libraries.

BACKGROUND OF THE INVENTION

Directed molecular evolution may be used to create proteins and enzymes with novel functions and properties. Starting with a known natural protein, several rounds of mutagenesis, functional screening, and/or selection and propagation of successful sequences are performed. The advantage of this process is that it may be used to rapidly evolve any protein without knowledge of its structure. Several different mutagenesis strategies exist, including point mutagenesis by error-prone PCR, cassette mutagenesis, and DNA shuffling. These techniques have had many successes; however, they are all handicapped by their inability to produce more than a tiny fraction of the potential changes and their ability to effectively explore all possible sequences. For example, there are $20^{500}$ possible amino acid changes for an average protein approximately 500 amino acids long. Clearly, the mutagenesis and functional screening of so many mutants is impossible; directed evolution provides a very sparse sampling of the possible sequences and hence examines only a small portion of possible improved proteins, typically point mutants or recombinations of existing sequences. By sampling randomly from the vast number of possible sequences, directed evolution is unbiased and broadly applicable, but inherently inefficient because it ignores all structural and biophysical knowledge of proteins.

In contrast, computational methods may be used to screen enormous sequence libraries (up to or more than $10^{80}$ in a single calculation) overcoming the key limitation of experimental library screeni ng methods such as directed molecular evolution. There are a wide variety of methods known for generating and evaluating sequences. These include, but are not limited to, sequence profiling (Bowie and Eisenberg, Science 253(5016): 164-70, (1991)), rotamer library selections (Dahiyat and Mayo, Protein Sci 5(5): 895-903 (1996); Dahiyat and Mayo, Science 278(5335): 82-7 (1997); Desjarlais and Handel, Protein Science 4: 2006-2018 (1995); Harbury et al, PNAS USA 92(18): 8408-8412 (1995); Kono et al., Proteins: Structure, Function and Genetics 19: 244-255 (1994); Hellinga and Richards, PNAS USA 91: 5803-5807 (1994)); and residue pair potentials (Jones, Protein Science 3: 567-574, (1994)). (see Altschul and Koonin, Trends Biochem Sci 23(11): 444-447. (1998); (see Altschul et al., J. Mol. Biol. 215(3): 403 (1990) and Lockless and Ranganathan, Science 286:295-299 (1999), Pattern discovery in Biomolecular Data: Tools, Techniques, and Applications; edited by Jason T.L. Wang, Bruce A. Shapiro, Dennis Shasha. New York: Oxford University, 1999.) Directed evolution is a random technique. Currently, there is no comprehensive rational design approach that allows efficient exploration of all possible sequence space.

**SUMMARY OF THE INVENTION**

The present invention provides methods for generating a secondary library of scaffold protein variants comprising providing a primary library comprising a rank-ordered list or filtered set of scaffold protein primary variant sequences. A list of primary variant positions in the primary library is then generated, and a plurality of the primary variant positions is then combined to generate a secondary library of secondary sequences.

It is an object of the present invention to provide computational methods for prescreening sequence libraries to generate and select secondary libraries, which may then be made and evaluated experimentally.

In an additional object, the invention provides methods for generating a secondary library of scaffold protein variants comprising providing a primary library comprising a rank-ordered list or filtered set of scaffold protein primary variant sequences, and generating a probability distribution of amino acid residues in a plurality of variant positions. The plurality of the amino acid residues is combined to generate a secondary library of secondary sequences. These sequences may then be optionally synthesized and tested, in a variety of ways, inducing muitiplexing PCR with pooled oligonucleotides, error prone PCR, gene shuffling, etc.

In a further object, the invention provides compositions comprising a plurality of secondary variant proteins or nucleic acids encoding the proteins, wherein the plurality comprises all or a subset of the secondary library. The invention further provides cells comprising the library, particularly mammalian cells.

In an additional object, the invention provides methods for generating a secondary library of scaffold protein variants comprising providing a first library rank-ordered list or filtered set of scaffold protei n primary variants, generating a probability distribution of amino acid residues in a plurality of variant positions; and synthesizing a plurality of scaffold protein secondary variants comprising a plurality of the amino acid residues to form a secondary library. At least one of the secondary variants is different from the primary variants.

It is a further object of the invention to provide a method for receiving a scaffold protein structure with residue positions; selecting a collection of variable residue positions from said residue positions; establishing a group of potential rotamers for each of said variable residue positions, and wherein a first group for a first variable residue position has a first set of rotamers from at least two different amino acid side chains, and wherein a second group for a second variable residue position has a second set of rotamers from at least two different amino acid side chains; and, analyzing the interaction of each of said rotamers in each group with all or part of the remainder of said protein to generate a set of optimized protein sequences.

It is a further object of the invention to provide a method for receiving a scaffold protein with residue positions; selecting a collection of variable residue positions fro m said residue positions; establishing a group of potential amino acids for each of said variable residue positions, wherein a first group for a first variable residue position has a first set of at least two amino acid side chains, and wherein a second group for a second variable residue position has a second set of at least two different amino acid side chains; and, analyzing the interaction of each of said amino acids with all or part of the remainder of said protein to generate a set of optimized protein sequences.

It is a further object of the invention to provide a method for receiving a scaffold protein with residue positions; selecting a set of variable residue positions from said residue positions; establishing a group of potential rotamers for each of said variable residue positions; analyzing the interaction of each of said rotamers with all or part of the remainder of said protein to generate a set of optimized protein sequences, wherein said analyzing step includes the use of at least one scoring function; and, generating a library of said optimized protein sequences.

It is a further object of the invention to provide a method for receiving a scaffold protein with residue positions; selecting a set of variable residue positions from said residue positions; classifying each variable residue position as either a core, surface or boundary position; establishing a group of potential amino acids for each of said variable residue positions, wherein the group for at least one variable residue position has at least two different amino acid side chains; and, analyzing the interaction of each of said amino acids with all or part of the remainder of said protein to generate a set of optimized protein sequences, wherein said analyzing step includes the use of at least one scoring function.

It is a further object of the invention to provide a method for receiving a scaffold protein with residue positions; selecting a set of variable residue positions from said residue positions; establishing a group of potential rotamers for each of said variable residue positions, wherein the group for at least one variable residue position has rotamers of at least two different amino acid side chains, and wherein at least one of said amino acid side chains is from a hydrophilic amino acid and, analyzing the interaction of each of said rotamers with all or part of the remainder of said protein to generate a set of optimized protein sequences, wherein said analyzing step includes the use of at least one scoring function.

It is a further object of the invention to provide a computational method for receiving a scaffold protein with residue positions; selecting a collection of variable residue positions from said residue positions; providing a sequence alignment of a plurality of related proteins; generating a frequency of occurrence for individual amino acids in at least a plurality of positions with said alignments; creating a pseudo-energy scoring function using said frequencies; using said pseudo-energy scoring function and at least one additional scoring function to generate a set of optimized protein sequences.

It is a further object of the invention to provide a computational method comprising receiving a scaffold protein with residue positions; selecting a collection of variable residue positions from said residue positions; providing a sequence alignment of a plurality of related proteins; generating a frequency of occurrence for individual amino acids in at least a plurality of positions with said proteins; selecting a group of potential amino acids for each of said variable residue positions, wherein a first group for a first variable residue position has a first set of at least two amino acid side chains, and wherein a second group for a second variable residue position has a second set of at least two different amino acid side chains according to their frequency of occurrence; and, analyzing the interaction of each of said amino acids at each variable residue position with all or part of the remainder of said protein using at least one scoring function to generate a set of optimized protein sequences.

It is a further object of the invention to provide a method computational method for receiving a scaffold protein with residue positions; selecting a collection of variable residue positions from said residue positions; providing an amino acid substitution matrix; creating a pseudo-energy scoring function using said matrix; using said pseudo-energy scoring function and at least one additional scoring function to generate a set of optimized protein sequences.

It is a further object of the invention to provide a method for receiving a scaffold protein with residue positions; selecting a collection of at least one variable residue position from said residue positions; importing a set of coordinates for a scaffold protein, said scaffold protein comprising amino add positions; analyzing the interaction of each of said amino acids with all or part of the remainder of said protein; utilizing a plurality of scoring functions, at least a first a scoring function having a first weight and a second scoring function having a second weight, to generate at least one variable decoy sequence; and, comparing the scores from said scoring functions of said variable decoy sequence to the scores of a reference state to generate modified weights, wherein each weight is increased if the corresponding score of the decoy is higher than the corresponding score of the reference state and each weight is decreased if the corresponding score of the decoy is lower than the corresponding score of the reference state and, wherein the extent of increase or decrease is based on the relative individual and total scores of the decoy and reference states.

It is a further object of the invention to provide a method for receiving a scaffold protein with residue positions; selecting a collection of variable residue positions from said residue positions; importing a set of coordinates for a scaffold protein, said scaffold protein comprising amino acid positions; generating a variable protein sequence comprising a defined energy state for each amino acid position; applying an energy increase to at least one of said defined energy states for a least one of said amino acid positions; and, generating at least one alternate variable protein sequence.

It is a further object of the invention to provide a method for receiving a scaffold protein with residue positions; selecting a collection of variable residue positions from said residue positions; importing a set of coordinates for a scaffold protein, said scaffold protein comprising amino acid positions; generating a variable protein sequence comprising a defined energy state for each amino acid position; applying a probability parameter to at least one of said amino acid positions; and generating at least one alternate variable protein sequence.

It is a further object of the invention to provide a method for receiving a scaffold protein with residue positions; selecting a collection of variable residue positions from said residue positions; importing a set of coordinates for a scaffold protein, said scaffold protein comprising amino acid positions; generating a set of optimized variant protein sequences comprising one or more variant amino acids; and, applying a clustering algorithm to cluster said set into a plurality of subsets.

It is a further object of the invention to provide a method for receiving at least one scaffold protein structure with variable residue positions of a target protein; computationally generating a set of primary variant amino acid sequences that adopt a conformation similar to the conformation of said target protein; and, identifying at least one protein sequence that is similar to at least one member of said set of primary variants, but is dissimilar to said target protein amino acid sequence.

It is an additional object of the invention to provide a method for generating variant protein sequence libraries comprising providing populations of at least two double stranded donor fragments corresponding to a nucleic acid template; adding polymerase primers capable of hybridizing to end regions of each of said population of donor fragments; generating a population of hybrid double stranded molecules wherein one strand comprises a 5'-purification tag and the other strand comprises a 5'-phosphorylated overhang; enriching for variant strands by removing strands comprising a 5'-biotin moiety; annealing said variant strands to form at least two double stranded ligation substrates; and, ligating said ligation substrates to form a double stranded ligation product wherein said ligation product encodes a variant protein.

These and other objects of the invention are to provide computational protein design and optimization techniques via an objective, quantitative design technique implemented in connection with a general purpose computer.

## BRIEF DESCRIPTION OF THE DRAWINGS

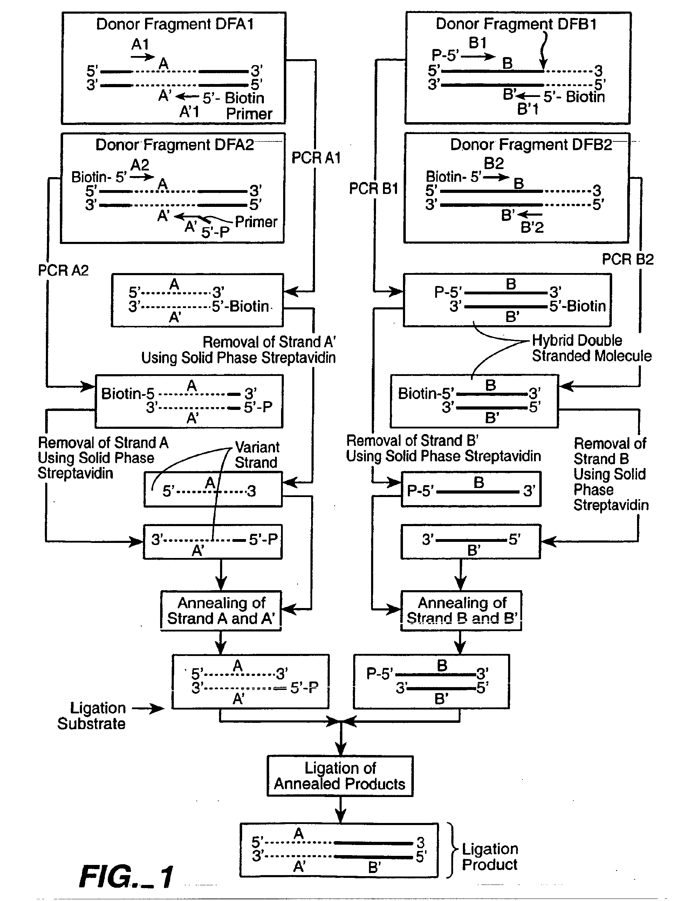

Figure 1 depicts a gene assembly scheme.

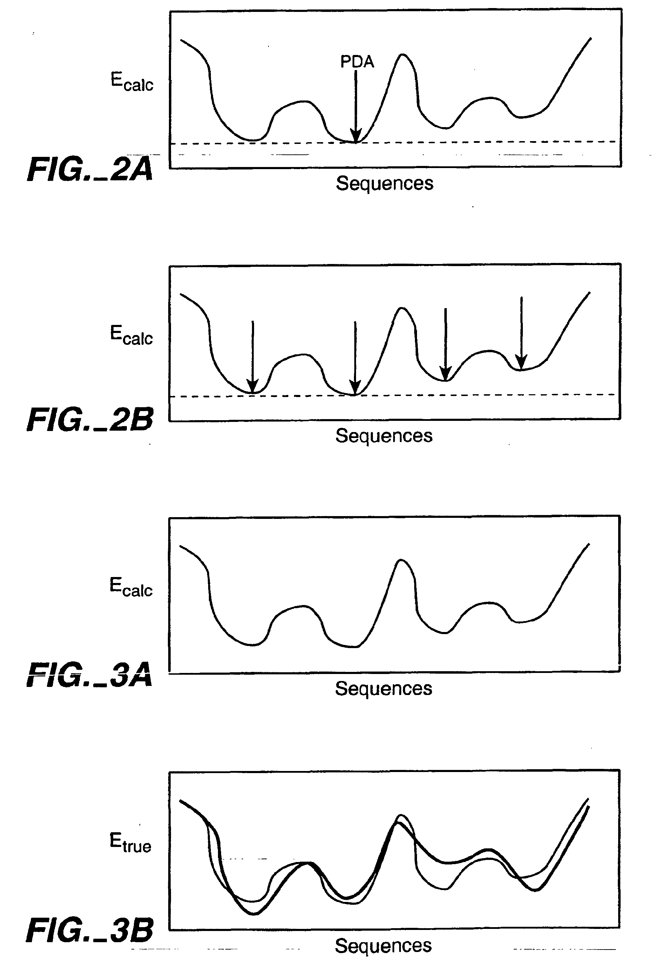

Figure 2 illustrates that most protein design simulations do not sufficiently may sequence space. As shown in the upper graph, most protein design simulations only map the lowest energy basin; thereby omitting other low energy basins that could provide viable sequences for computationally generated protein sequences.

Figure 3 illustrates the point that the alternate low energy basins can represent equally good sequences for incorporation into a protein template. This is because the force field representation of the energy (i.e., $E_{calc}$) is not necessarily identical to the actual energy (i.e., $E_{true}$) associated with a native protein structure.

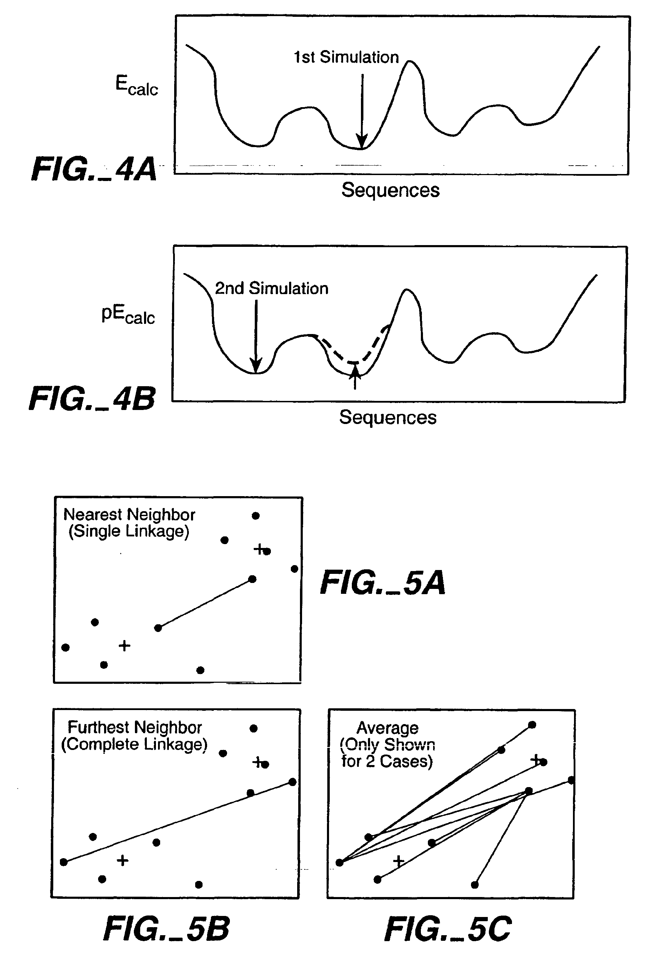

Figure 4 illustrates the application of taboo for mapping sequence space. The calculated energy surface is manipulated based on previous solutions to discourage repeated convergence to the same local minimum.

Figure 5 illustrates clustering algorithms that may be used in the methods of the present invention.

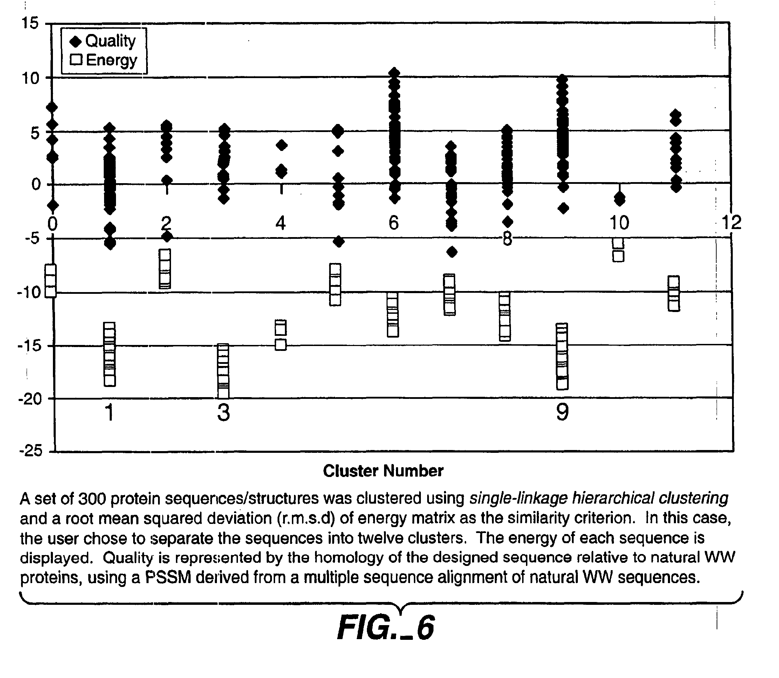

Figure 6 depicts an example of energy matrix clustering of designed WVV domain proteins using a single linkage clustering algorithm.

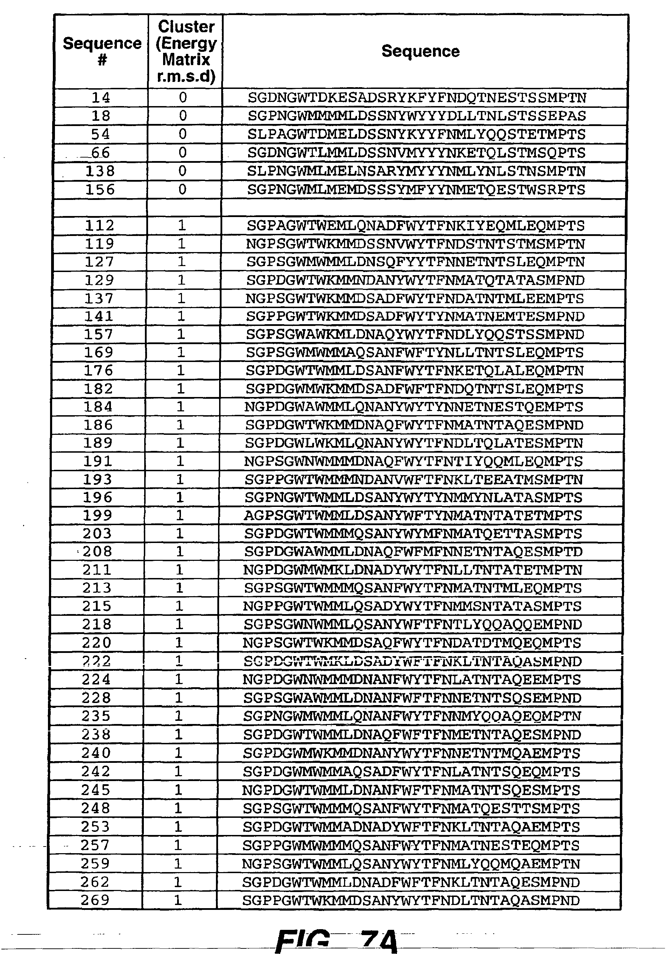

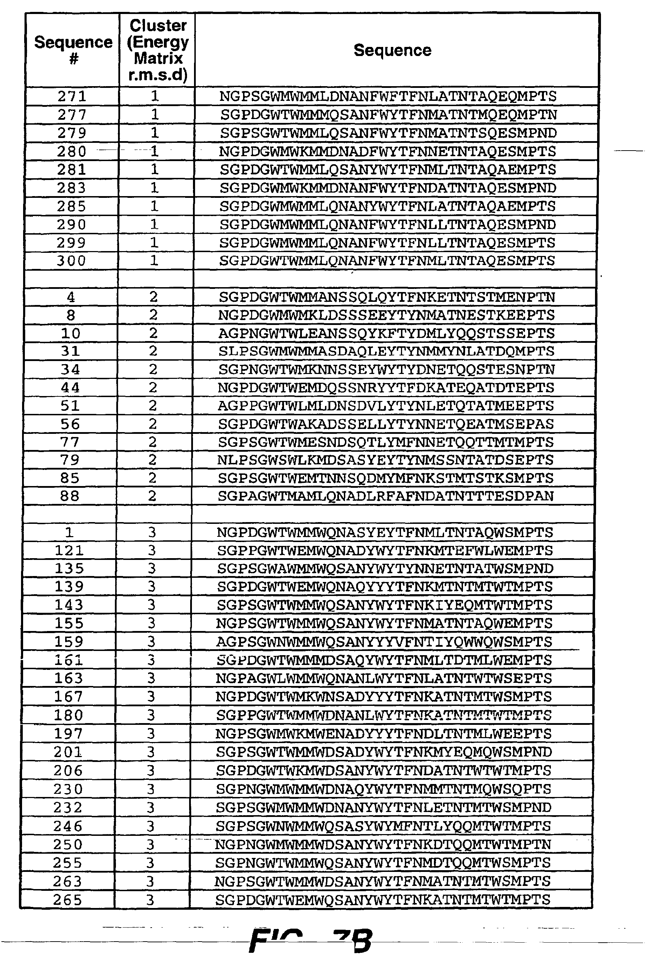

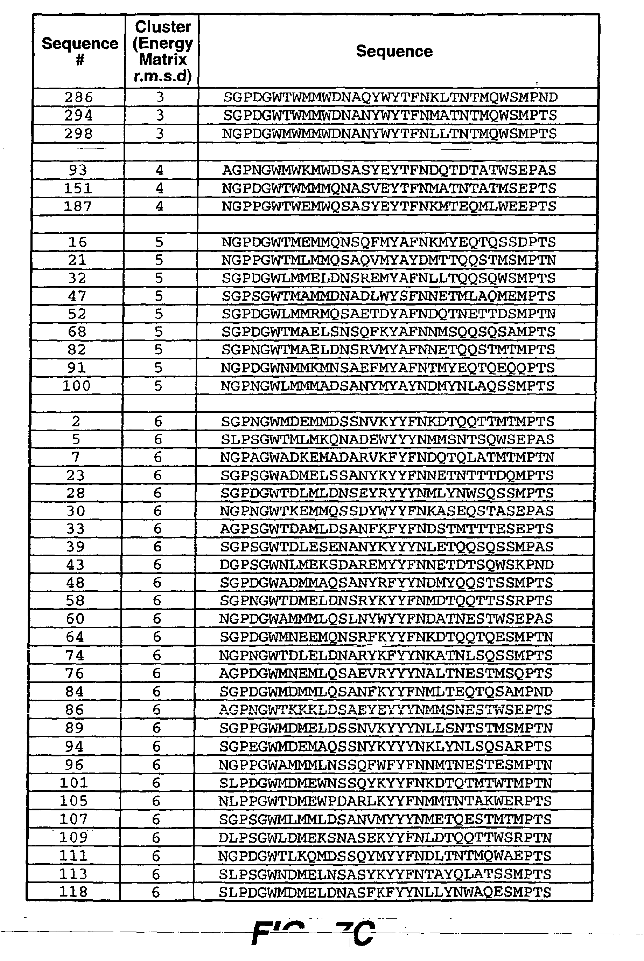

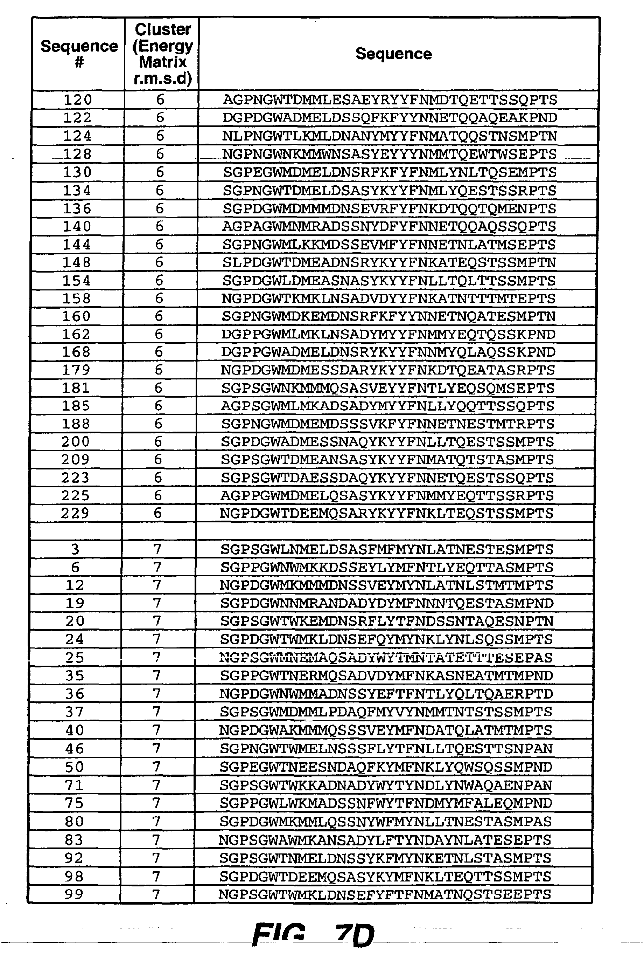

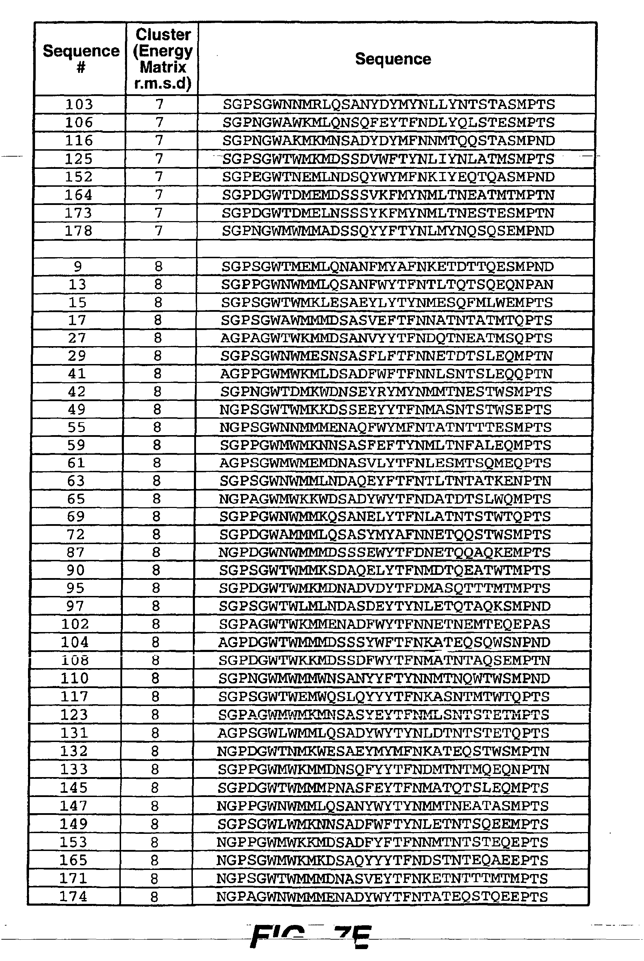

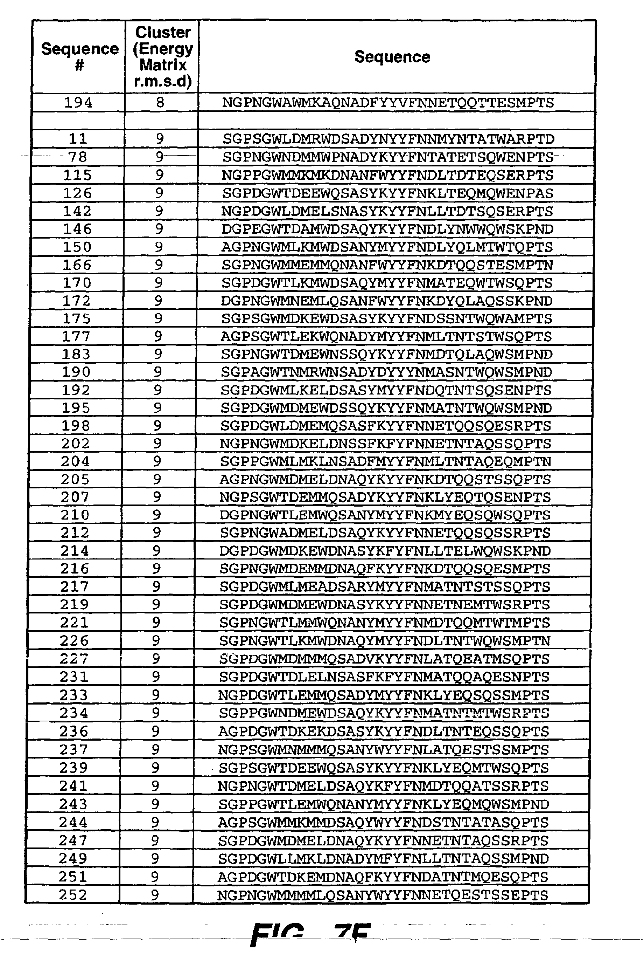

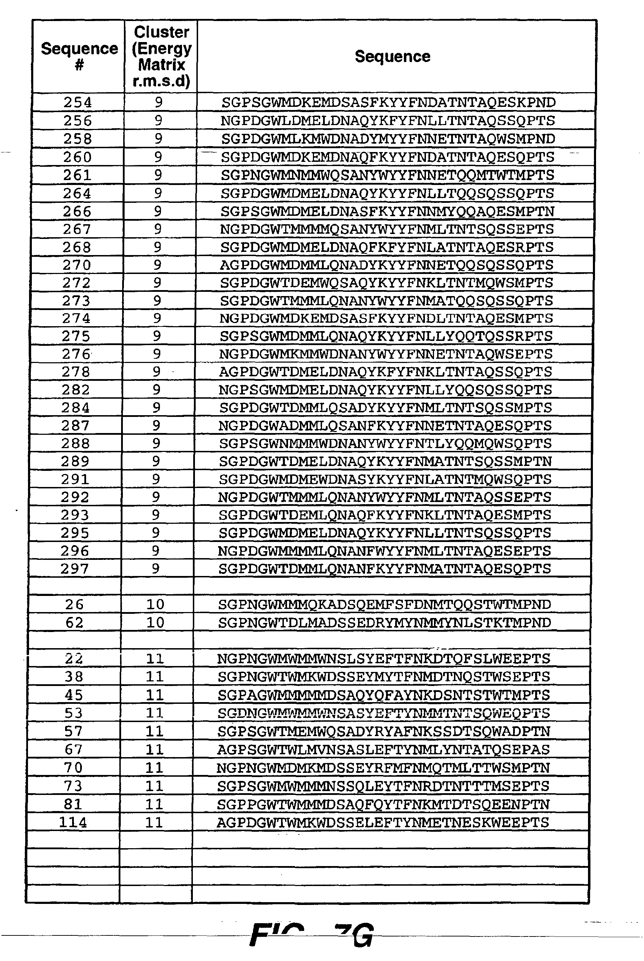

Figure 7 depicts the data used to generate Figure 7.

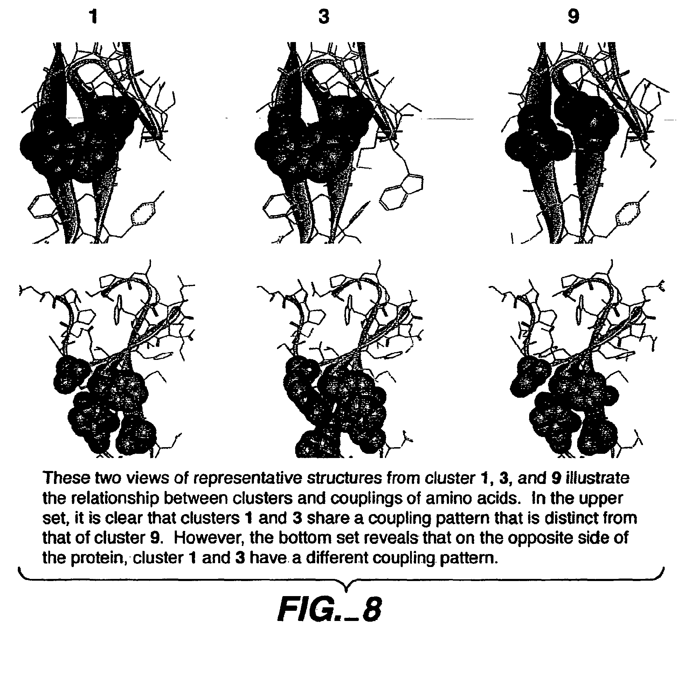

Figure 8 depicts representative structures from cluster 1, 3, and 9.

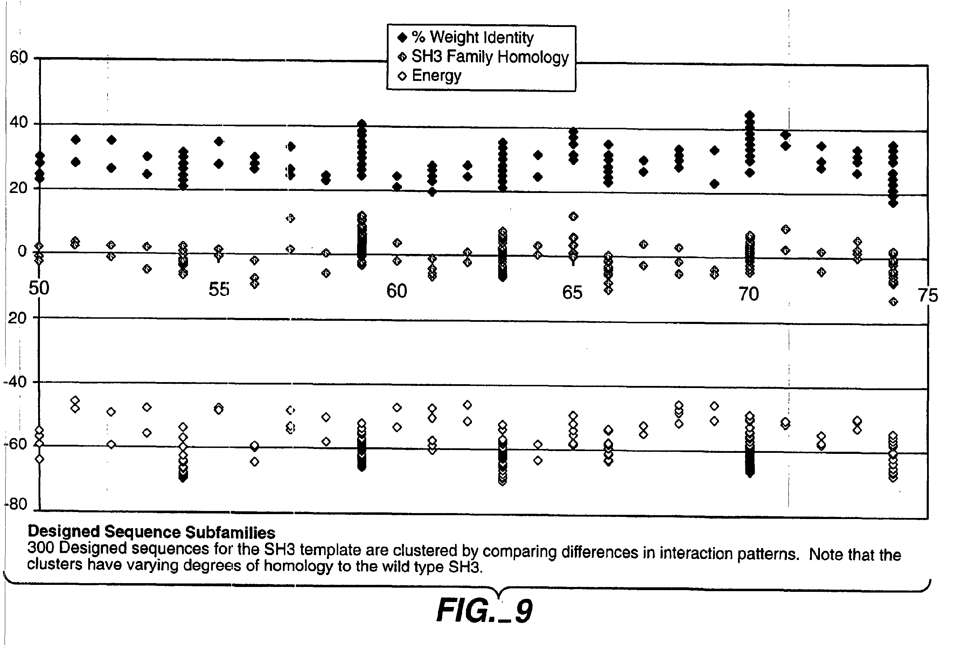

Figure 9 depicts an example of energy matrix clustering of designed SH3 proteins.

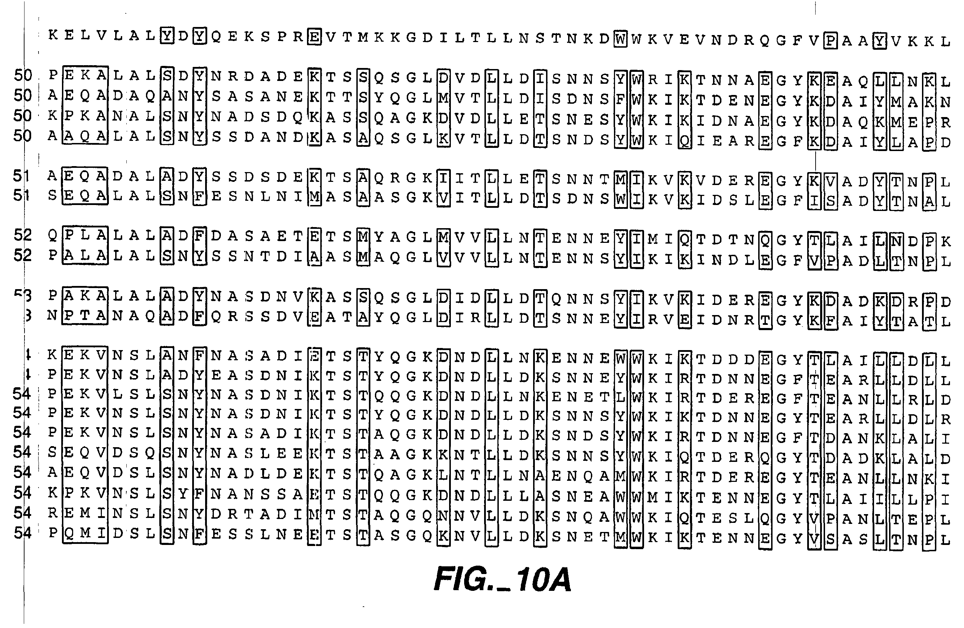

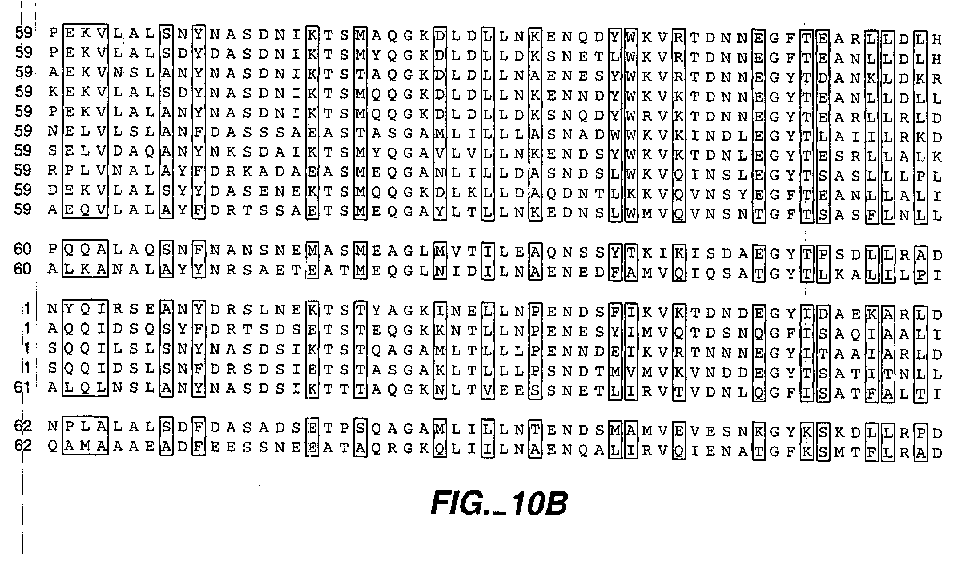

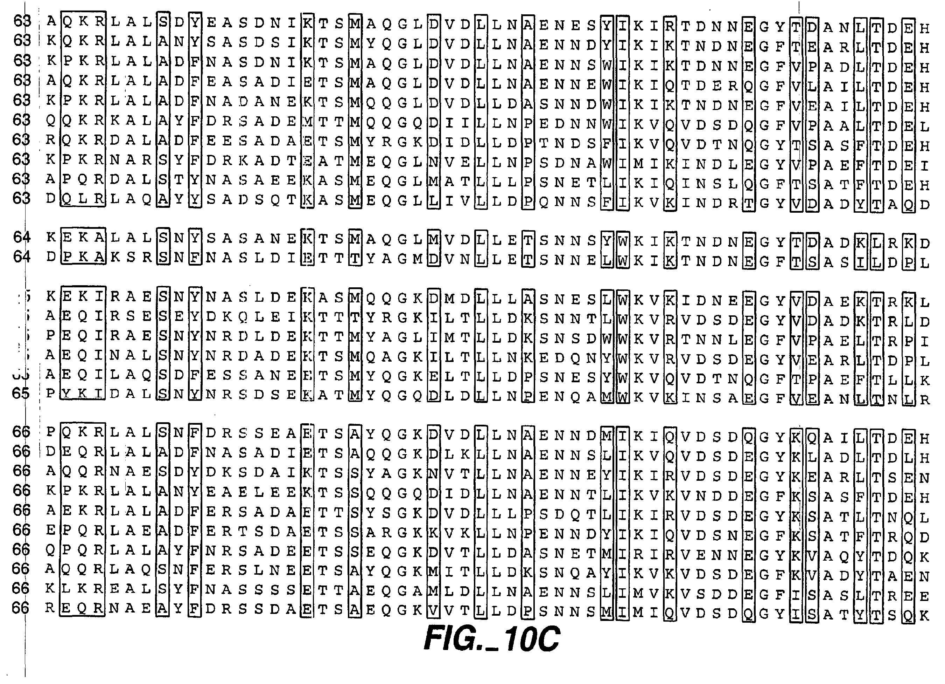

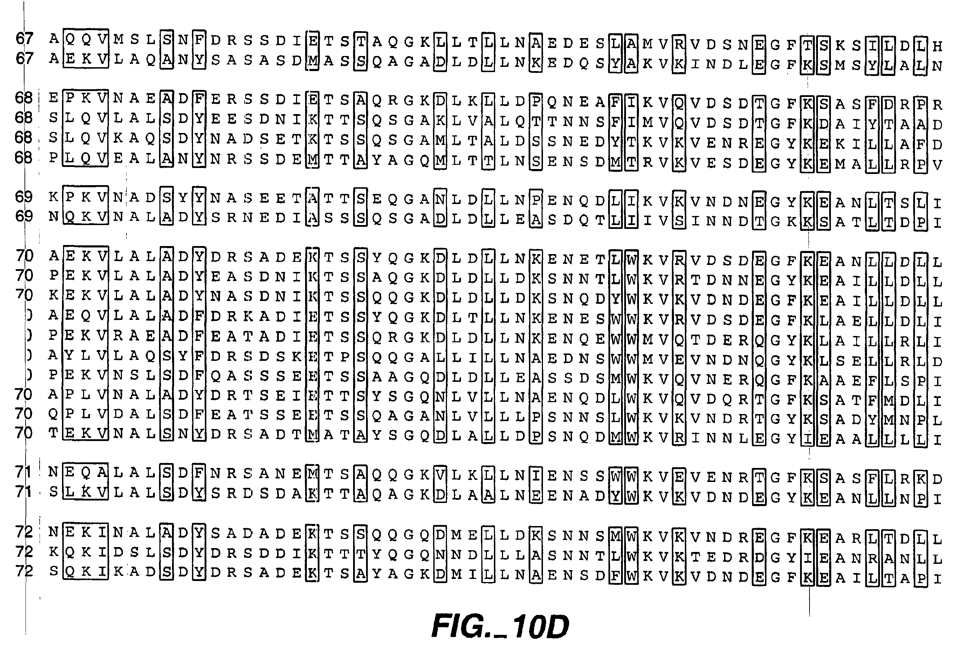

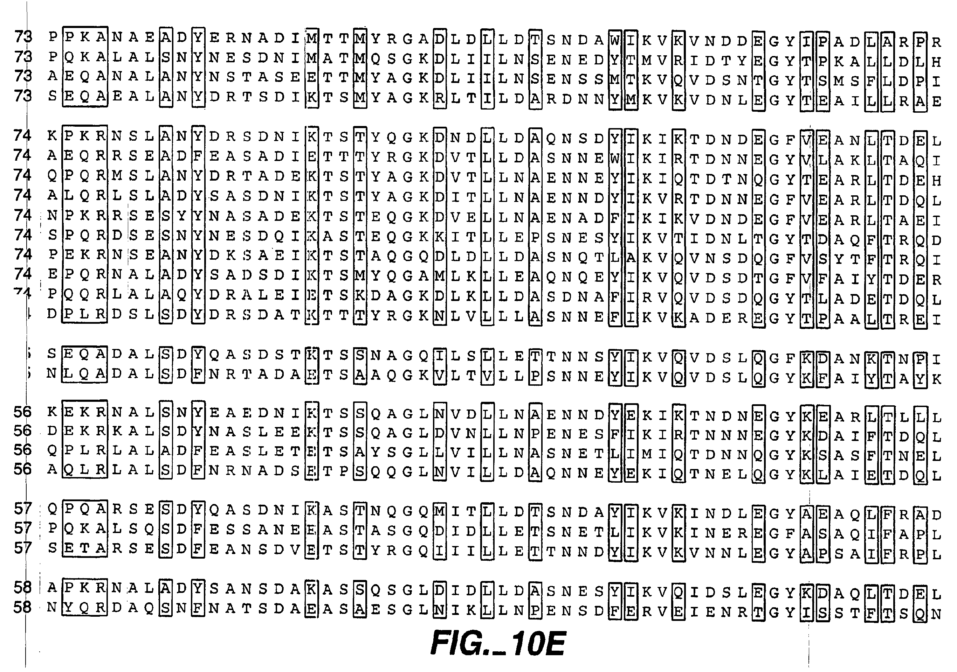

Figure 10 depicts the superfamily of sequences designed for SH3. As shown in Figure 6, the virtual superfamily of sequences designed using an SH3 backbone structure have significant homology to the template sequence and other members of the natural SH3 family. Identities with the native sequence are highlighted in dark grey. Functional positions are shaded in light grey. Note that although the simulations did not include a functional constraint, the native functional residue usually appears with low frequency in the alignment.

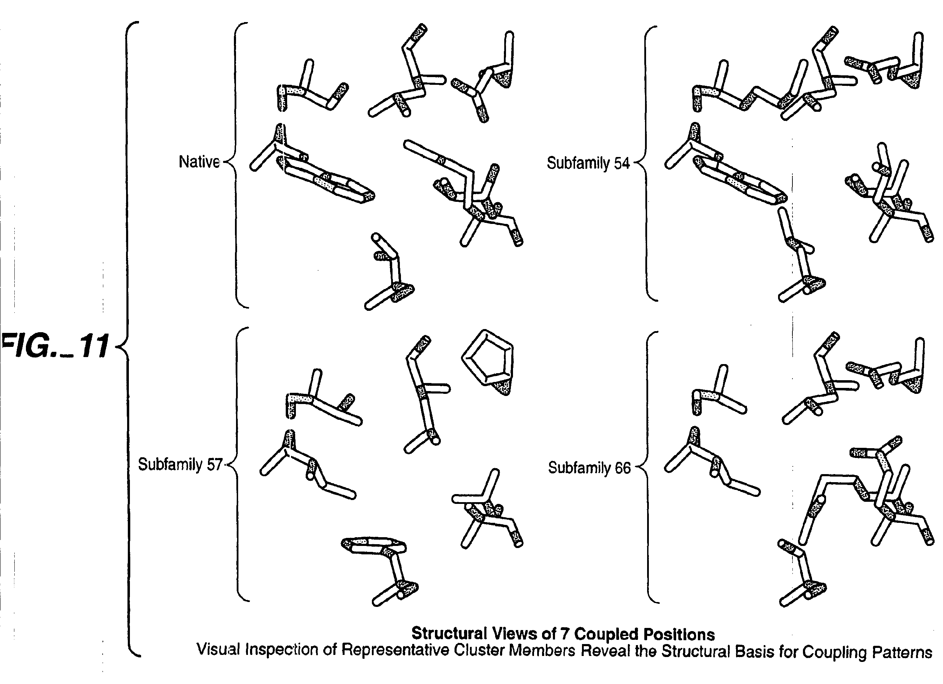

Figure 11 illustrates coupling patterns in SH3 subfamilies. Interaction-based clustering reveals a series of virtual sequence subfamilies that contain various combinations of coupled amino acids (highlighted in different shades of grey. Note that some subfamilies differ by amino acids coupled at 7 positions (medium intensity shading). The amino acid couplings lead to multiple low energy solutions in different sequence subspaces. As a result, some subfamilies have more similarity to the wild type sequence than others.

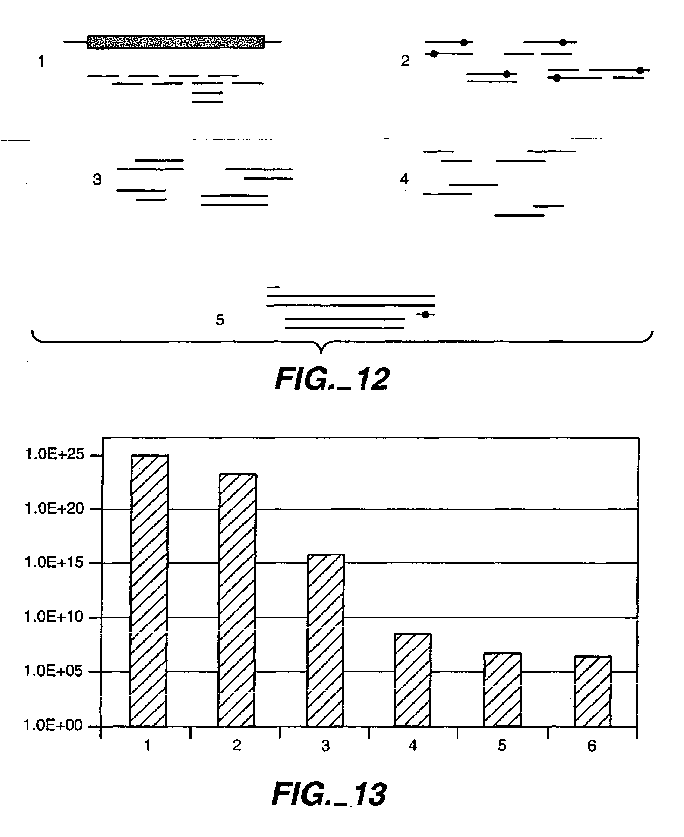

Figure 12 depicts the synthesis of a full-length gene and all possible mutations by PCR. Overlapping oligonucleotides corresponding to the full-length gene (black bar, Step 1) are synthesized, heated and annealed. Addition of *Pfu* DNA polymerase to the annealed oligonucleotides results in the 5' → 3' synthesis of DNA (Step 2) to produce longer DNA fragments (Step 3). Repeated cycles of heating, annealing (Step 4) results In the production of longer DNA, including some full-length molecules. These may be selected by a second round of PCR using primers (arrowed) corresponding to the end of the full-length gene (Step 5).

Figure 13 depicts the reduction of the dimensionality of sequence space by PDA™ technology screening. From left to right, 1: without PDA™ technology; 2: without PDA™ technology not counting Cysteine, Proline, Glycine; 3: with PDA™ technology using the 1% criterion, modeling free enzyme; 4: with PDA™ technology using the 1% criterion, modeling enzyme-substrate complex; 5: with PDA™ technology using the 5% criterion modeling free enzyme; 6: with PDA™ technology using the 5% criterion modeling enzyme-substrate complex.

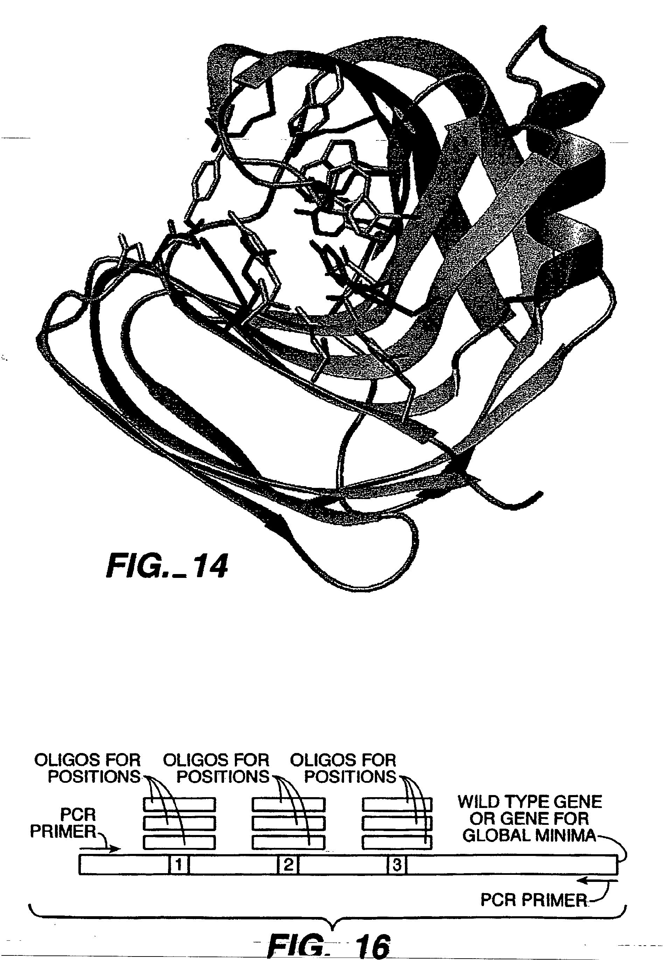

Figure 14 depicts the active site of B. circulans xylanase. Those positions included in the PDA™ technology design are shown by their side chain representation.

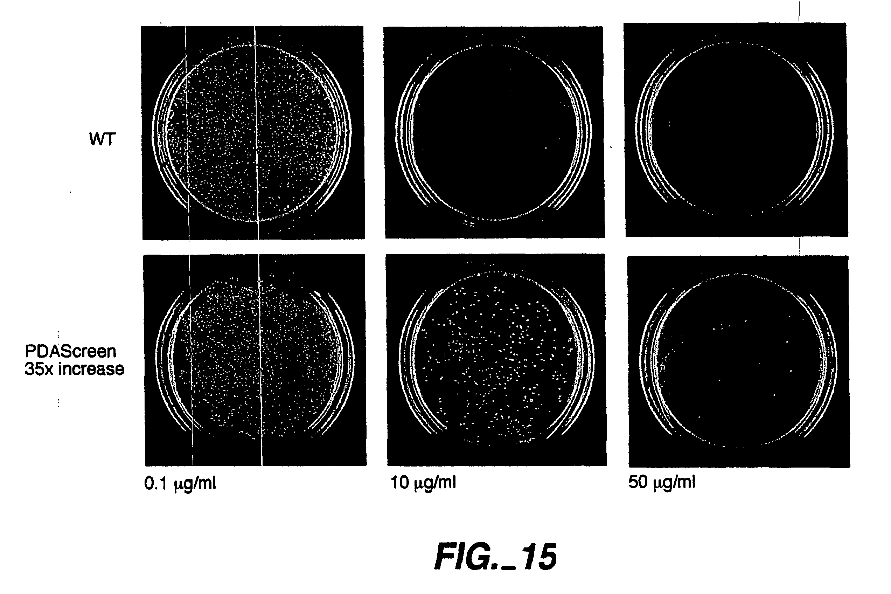

Figure 15 depcits cefotaxime resistance of E. coli expressing wild-type (WT) and PDA™ technology.

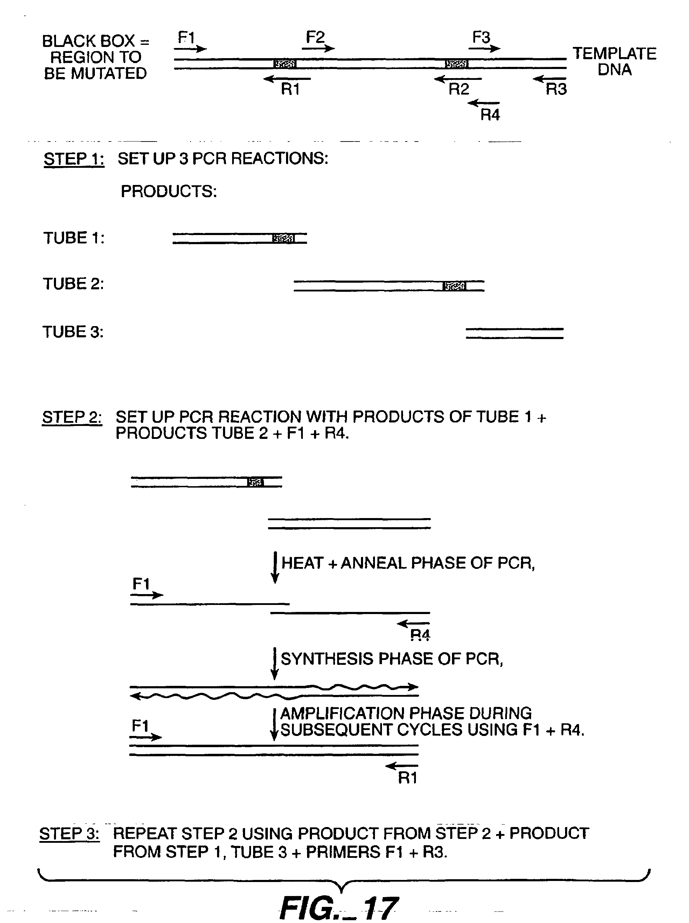

Figure 16 depicts a preferred scheme for synthesizing a library of the invention. The wild-type gene, or any starting gene, such as the gene for the global minima gene, may be used. Oligonucleotides comprising different amino acids at the different variant positions may be used during PCR using standard primers. This generally requires fewer oligonucleotides and may result in fewer errors.

Figure 17 depicts and overlapping extension method. At the top of Figure 6 is the template DNA showing the locations of the regions to be mutated (black boxes) and the binding sites of the relevant primers (arrows). The primers R1 and R2 represent a pool of primers, each containing a different mutation; as described herein, this may be done using different ratios of primers if desired. The variant position is flanked by regions of homology sufficient to get hybridization. In this example, three separate PCR reactions are done for step 1. The first reaction contains the template plus oligos F1 and R1. The second reaction contains template plus F2 and R2, and the third contains the template and F3 and R3. The reaction products are shown. In Step 2, the products from Step 1 tube 1 and Step 1 tube 2 are taken. After purification away from the primers, these are added to a fresh PCR reaction together with F1 and R4. During the Denaturation phase of the PCR, the overlapping regions anneal and the second strand is synthesized. The product is then amplified by the outside primers. In Step 3, the purified product from Step 2 is used in a third PCR reaction, together with the product of Step 1, tube 3 and the primers F1 and R3. The final product corresponds to the full-length gene and contains the required mutations.

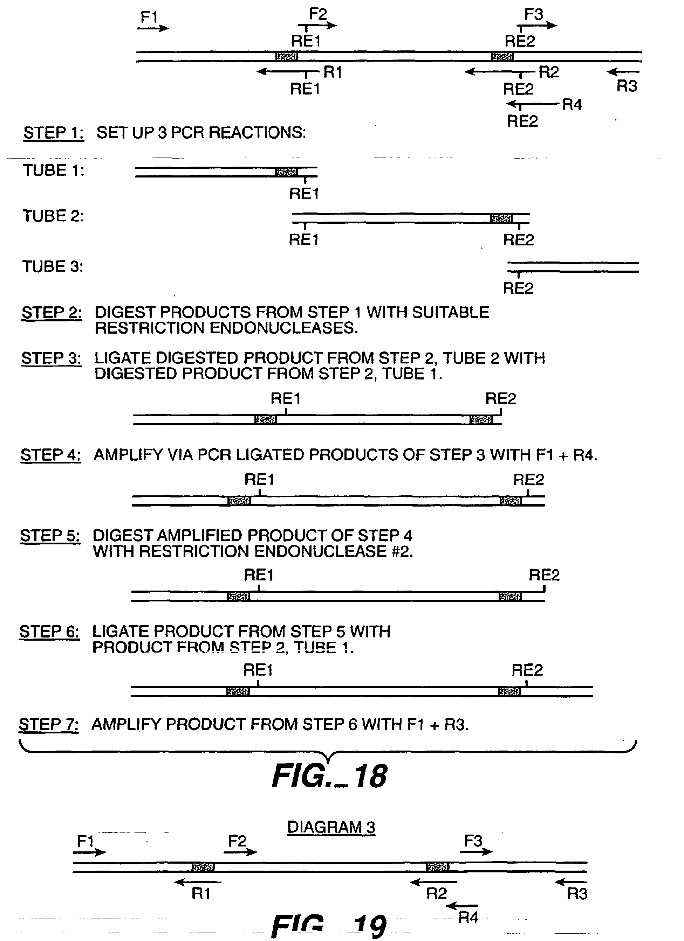

Figure 18 depicts a ligation of PCR reaction products to synthesize the libraries of the invention. In this technique, the primers also contain an endonuclease restriction site (RE), eith er blunt, 5' overhanging or 3' overhanging. We set up three separate PCR reactions for Step 1. The first reaction .contains the template plus oligos F1 and R1. The second reaction contains template plus F2 and R2, and the third contains the template and F3 and R3. The reaction products are shown. In Step 2, the products of step 1 are purified and then digested with the appropriate restriction endonuclease. The digestion products from Step 2, tube 1 and Step 2, tube 2 and ligate them together with DNA ligase (step 3). The products are then amplified in Step 4 using primer F1 and R4. The whole process is then repeated by digesting the amplified products, ligating them to the digested products of Step 2, tube 3, and then amplifying the final product by primers F1 and R3. It would also be possible to ligate all three PCR products from Step 1 together in one reaction, providing the two restriction sites (RE1 and RE2) were different

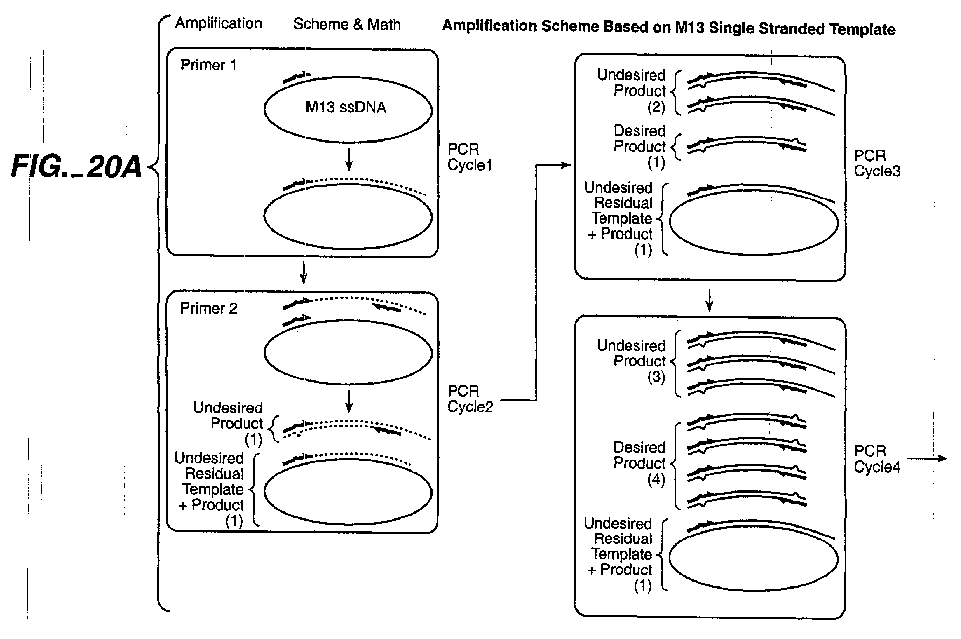

Figure 19 depicts blunt end ligation of PCR products. In this technique, the primers such as F1 and R1 do not overlap, but they abut. Again three separate PCR reactions are performed. The products from tube 1 and tube 2 are ligated, and then amplified with outside primers F1 and R4. This product is then ligated with the product from Step 1, tube 3. The final products are then amplified with primers F1 and R3.

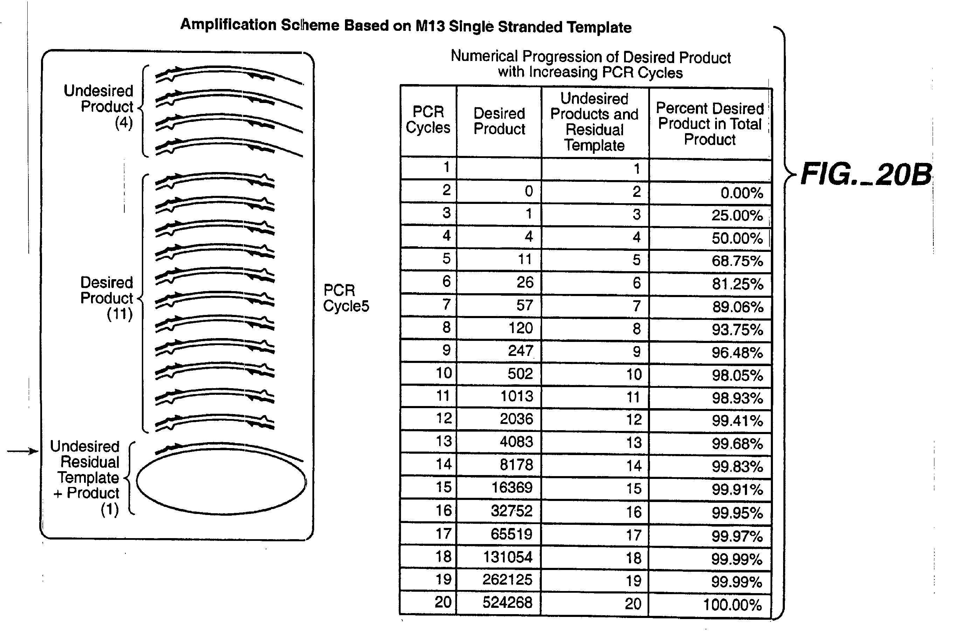

Figure 20A and B depicts M13 single stranded template production of mutated PCR products. Primer1 and Primer2 (each representing a pool of primers corresponding to desired mutations) are mixed with the M13 template containing the wild type gene or any starting gene. PCR produces the desired product (11) containing the combinations of the desired mutations incorporated in Primer1 and Primer2. This scheme may be used to produce a gene with mutations, or fragments of a gene with mutations that are then linked together via ligation or PCR for example.

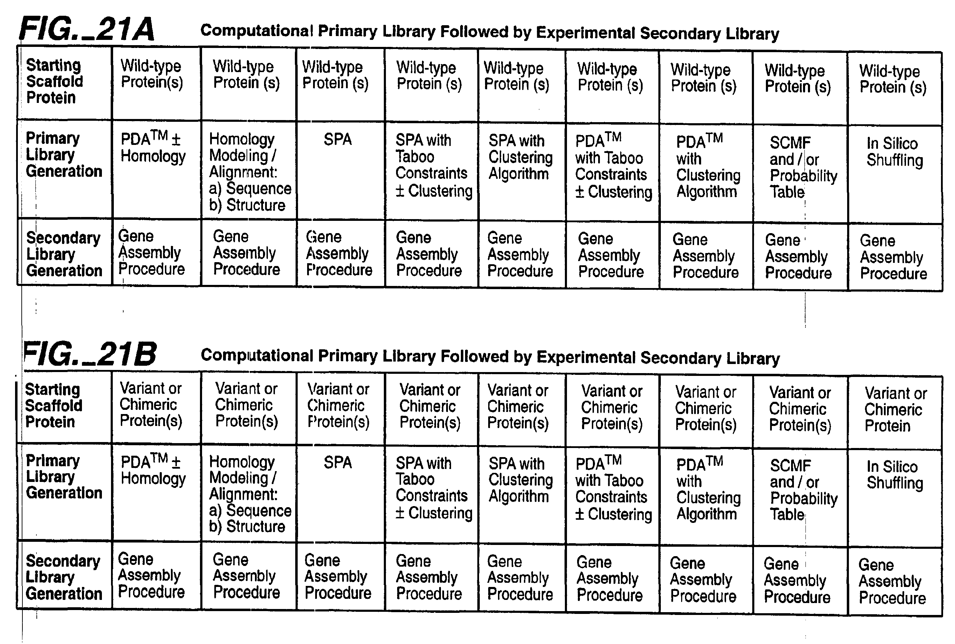

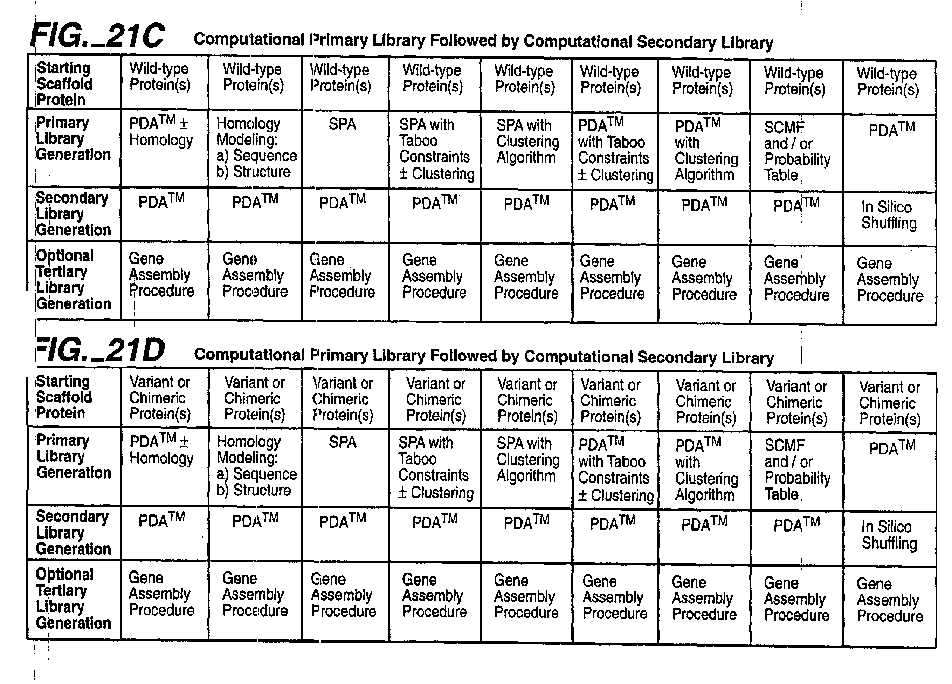

Figure 21A-E depict examples of some preferred combinations.

## DETAILED DESCRIPTION OF THE INVENTION

As used herein, the following terms shall have the meaning as described below.

By "altered phenotype" or "changed physiology" or other grammatical equivalents herein is meant that the phenotype of the cell containing a variable amino acid sequence (preferably an optimized sequence) is altered in some way, preferably in some detectable, observable and/or measurable way. Examples of phenotypic changes Include, but are not limited to: gross physical changes such as changes in cell morphology, cell growth, cell viability, adhesion to substrates or other cells, and cellular density; changes in the expression of one or more RNAs, proteins, lipids, hormones, cytokines, or other molecules; changes in the equilibrium state (i.e. half-life) or one or more RNAs, proteins, lipids, hormones, cytokines, or other molecules; changes in the localization of one or more RNAs, proteins, lipids, hormones, cytokines, or other molecules; changes in the bioactivity or specific activity of one or more RNAs, proteins, lipids, hormones, cytokines, receptors, or other molecules; changes in phosphorylation; changes in the secretion of ions, cytokines, hormones, growth factors, or other molecules; alterations in cellular membrane potential, polarization, integrity or transport; changes in infectivity, susceptibility, latency, adhesion, and uptake of viruses and bacterial pathogens; etc. By "capable of altering the phenotype" herein is meant that the library member (e.g. the variable amino acid sequence and/or the variable nucleic acid sequence) may change the phenotype of the cell in some detectable and/or measurable way.

By "alternate amino acid" as used herein is meant an amino acid state that differs from the amino acid defined by the starting amino acid sequence in the protein design cycle. As outlined below, this starting amino acid sequence (e.g. the scaffold protein) may be a wild-type sequence or a variant sequence.

By "amino acid identity" as used herein is meant the identity of an amino acid at a specified position; e.g. when the position of an amino acid is specified, which one of the 20 naturally occurring or non-natural analogs is present at that position.

By "boundary residues" as used herein is meant, residue positions that are not clearly in the protein core or on the protein surface. Methods for determining boundary residues are outlined below. The solvent accessibility of side chains in boundary positions is determined by the conformation and identities of the residues surrounding it. In a preferred embodiment, both hydrophobic and polar amino acids can be considered as possible replacement residues at boundary positions.

By "candidate bioactive agent" or "candidate drugs" or grammatical equivalents herein is meant any molecule, e.g. proteins (which herein includes proteins, polypeptides, and peptides), small organic or inorganic molecules, polysaccharides, polynucleotides, etc. which are to be tested against a particular target. Candidate agents encompass numerous chemical classes. In a preferred embodiment, the candidate agents are organic molecules, particularly small organic molecules, comprising functional groups necessary for structural interaction with proteins, particularly hydrogen bonding, and typically include at least an amine, carbonyl, hydroxyl or carboxyl group, preferably at least two of the functional chemical groups. The candidate agents often comprise cyclical carbon or heterocyclic structures and/or aromatic or polyaromatic structures substituted with one or more chemical functional groups. A preferred embodiment is a protein where the uses include therapeutic, veterinary, agricultural, and industrial applications.

By a "cellular library" herein is meant a plurality of cells wherein generally each cell within the library contains at least one member of the library. Ideally each cell contains a single and different library member, although as will be appreciated by those in the art, some cells within the library may not contain a library member and some may contain more than one library member. When methods other than retroviral infection are used to introduce the library members into a plurality of cells, the distribution of library members within the individual cell members of the cellular library may vary widely, as it is generally difficult to control the number of nucleic acids which enter a cell during electroporation and other transformation methods. Suitable cell types for cellular libraries are included below. In addition, as will be appreciated by those in the art, a cellular library generally includes a single cell type, although in some embodiments, a cellular library may contain two or more cell types.

By "chemically modified" as used herein is meant to include modification via chemical reactions as well as enzymatic reactions. The substrates in these reactions generally include, but are not limited to, alkyl groups (including but not limited to straight and branched alkanes, alkenes, and alkynes), aryl groups (including but not limited to arenes and heteroaryl), alcohols, ethers, amines, aldehydes, ketones, carboxylic acids, esters, amides, heterocyclic compounds (including, but not limited to, piperidines, pyrrolidines, purines, pyrimidines, benzodiazepins, and carbohydrates), steroids (including but not limited to estrogens, androgens, cortisone, ecodysone, etc.), secondary metabolites (including, but not limited to, terpenoids, alkaloids, polyketides, beta-lactams, polyether antibiotics, and aminoglycosides), organometallic compounds, lipids, amino acids, and nucleosides. The reactions generally include, but are not limited to, hydrolysis, reduction, oxidation, alkylation, aromatic substitutions, electrocyclizations, dipolar cyclizations, radical anion, radical cation, metal mediated couplings, and polymerization.

By "clustering algorithm" herein is meant an algorithm that may be used to separate a large selection or set of computationally generated sequences into subsets that represent various sub-regions of sequence space. Clustering algorithms are well known in the art, and representative examples are outlined below.

By "control sequences" or "regulatory sequences" as used herein refers to DNA sequences necessary for the expression of a gene in a particular host organism. The control sequences that are suitable for prokaryotes, for example, include a promoter, optionally an operator sequence, and a ribosome binding site. Eukaryotic cells utilize control sequences including, but not limited to, promoters, polyadenylation signals, and enhancers.

By "core positions" as used herein is meant, positions that are in the interior of a protein or which are inaccessible or nearly inaccessible to solvent. Methods for determining which position comprise core positions are outlined below. As more fully outlined below, in a preferred embodiment, for design purposes, only hydrophobic amino acids are considered for incorporation into variable positions at core variable positions. As more fully outlined below, in an alternate preferred embodiment, polar amino acids are considered at core positions only if they form favorable electrostatic or hydrogen bond interactions with other polar groups, or if disruption of the scaffold is desired.

By "coupling" as used herein is meant the non-additive contribution (e.g. synergistic) of two or more amino acids to an interaction involving said amino acids. Coupling can be positive (the interaction is more favorable than the sum of the individual contributions), neutral, or negative (the interaction is less favorable than the sum of the individual contributions). Such coupling typically occurs for amino acids located very close in space.

By "decoy state," "decoy structure," or "decoy sequence" as used herein is meant a protein sequence and structure that is different from a specified reference state, and that serves as a comparison state for use in various parameter optimization methods. Decoy structures are more fully described below.

By "donor fragment" or "donor nucleic acid fragment" as used herein is meant nucleic acid fragments generated from or corresponding to a template nucleic acid molecule. Preferably, the donor fragments are generated using modified primers and a polymerase, although fragments may be generated using enzymatic, chemical or physical cleavage (e.g. shearing) of template nucleic acid molecules. Any DNA/RNA polymerase is suitable; however thermophilic polymerases are preferred.

An "energy matrix" is defined for the present purposes as follows. A protein design cycle simulation is performed to yield a single protein sequence/structure. In the context of this state, all amino acids (in all rotamer states) are sampled at each position or at each variable position. Alternatively, less than all rotamer states, or less than all amino acids, are sampled at some or all of the positions. Suita ble sampling techniques to generate the energies are outlined herein. The context-dependent energy of each amino acid is stored. An energy matrix is defined by the listing of the context-dependent energy of each amino acid at each position of the structure. The similarity of two energy matrices (from two different simulations) may be defined as the root-mean-squared-deviation of two energy matrices. It should be noted that in some cases, energy matrices comprising less than all of the possible interactions can be constructed.

By "filtered set" herein is meant the optimized protein sequences that are generated using some sort of selection criteria. Although in some cases, the set may comprise an arbitrary or random selection of a subset of the primary sequences. In a preferred embodiment, the filtered set comprises a rank ordered list of sequences. As outlined herein, this may be done in a variety of ways, including an arbitrary cutoff (for example, the top 10,000 sequences are chosen, or the top 1000 and the bottom 1000), an energy limitation (e.g. anything with a total energy calculation below X), or when a certain number of residue positions have been varied (e.g. the set is complete when 10 variable positions is achieved, etc). As is outlined more fully below, filtering can be used as all or part of the primary, secondary, tertiary, etc. library generation; that is, filtering can be the sole computational analysis or part of a larger analysis, at one or more of the steps of the invention. For example, a primary library may be computationally generated using PDA, and a filtering step applied to define the set for secondary library generation, etc.

By "fixed position" herein is meant, residue positions at which the amino acid identity will be held constant in a protein design calculation, in some embodiments, fixed positions may be floated, as defined below. That is, in some embodiments, an amino acid identity is kept fixed, but its rotameric state is allowed to change. In other embodiments, the amino acid identity and rotameric state are held constant. The conformation and amino acid identity may be that observed in the scaffold structure or the conformation and/or amino acid identity may be different than that observed in the scaffold structure.

By "floated position" herein is meant, a position at which the amino acid conformation but not the amino acid identity is allowed to vary in a protein design calculation. The floated position may be fixed as a non-wild type residue. For example, when known site-directed mutagenesis techniques have shown that a particular amino acid is desirable (for example, to eliminate a proteolytic site or alter the substrate specificity of an enzyme), the position may be constrained to allow only that amino acid, Alternatively, the methods of the present invention may be used to evaluate specific mutations *de novo.*

By "gene assembly procedures" as used herein is meant either enzymatic or chemical methods of joining gene fragments. A wide variety of exemplary methods are included herein and described below.

By "global optimum protein sequence" as used herein is meant an amino acid sequence that best fits the mathematical equations of the computational process. As will be appreciated by those in the art, a global optimum sequence is the sequence that has the lowest energy or best score of any possible sequence in the context of the particular computational analysis utilized. That is, the global optimum sequence depends on the scoring or ranking systems used, and may change with different computational parameters. For example, when PDA™ is used, the global optimum will depend on the scoring functions utilized, the weighting factors, etc. In addition, there are any number of sequences that are not the global minimum but that have low energies or favorable scores referred to herein as "optimized sequences", defined below.

By "labeled" herein is meant that nucleic acids, proteins, candidate agents, antibodies or other components of the invention have at least one element, isotope, or chemical compound attached to enable the detection of nucleic acids, proteins and antibodies of the invention.

By "ligation product" as used herein is meant either the single stranded or double stranded nucleic acid molecule resulting when at least two ligation substrates are ligated together.

By "ligation substrate" as used herein is meant either a single or double stranded nucleic acid molecule formed by annealing from two complementary donor fragments in which one donor fragment has a 5'-phosphorylated overhang and the other fragment has a free 3'-terminus (see Figure 1).

By "nucleic acid template" herein is meant a single or double stranded nucleic acid. In a preferred embodiment, the nucleic acid template is used to generate donor fragments, defined above. The donor fragments may be obtained directly from the nucleic acid template or separately obtained, e.g., by nucleic acid synthesis, fragmentation (e.g. enzymatic, chemical or physical) or amplification reactions. A nucleic acid template may comprise an intact gene, or a fragment of a gene encoding functional domains of a protein, such as enzymatic domains, regulatory sequences, binding domains, etc., as well as smaller gene fragments The template nucleic acid may be from any organism, either prokaryotic or eukaryotic. The template sequence may be naturally occurring, a variant, a product of a computational step, etc.

By "nucleoside" as used herein, includes nucleotides, nucleosides and analogs, including modified nucleosides such as amino modified nucleosides and includes non-naturally occurring analog structures, I.e. the individual units of a peptide nucleic acid, each containing a base, are referred to herein as a nucleoside.

By "operably linked" as used herein means two or more nucleic acids linked together such that the desired functionality is achieved. For example, when a first nucleic acid sequence is placed Into a functional relationship with another nucleic acid sequence. For example, DNA for a presequence or secretory leader is operably linked to DNA for a polypeptide if it is expressed as a preprotein that participates in the secretion of the polypeptide; a promoter or enhancer is operably linked to a coding sequence if it affects the transcription of the sequence; or a ribosome binding site is operably linked to a coding sequence if it is positioned so as to facilitate translation. Generally, operably linked DNA sequences are contiguous, and in the case of a secretory leader, contiguous and in reading phase. However, enhancers do not have to be contiguous. Linking can be accomplished by ligation at convenient restriction sites. If such sites do not exist, the synthetic oligonucleotide adaptors or linkers are used in accordance with conventional practice

By "optimized protein sequence" as used herein is meant a sequence with at least one optimized property. For example, in the context of a particular computational analysis, an optimized sequence will exhibit a low energy or favorable score. For example, when PDA™ is used, an optimized sequence is one which has a lower energy than the energy of the starting scaffold protein. Alternatively, an optimized protein sequence may have one or more protein properties, defined below, that are desirably different as compared to the starting scaffold protein. An optimized protein sequence may or may not be the global optimum sequence, however, an optimized protein sequence has at least one amino acid substitution, insertion or deletion as compared to the starting scaffold protein used to generate the optimized sequence.

By a "plurality of cells" herein is meant roughly from about $10^2$ cells to $10^3$, $10^8$ or $10^9$, with from $10^6$ to $10^8$ being preferred.

By "position" as used herein is meant a location In the sequence of a protein. Positions are typically numbered using the protein numbering scheme described below. In the context of a given scaffold protein, each position is associated with the location and/or orientation of its associated backbone atoms in three dimensions. Consequently, positions may be described by their secondary structure and by whether an amino acid located at that position would be solvent exposed or buried in the protein core.

By "presentation scaffold" or "presentation structure" as used herein is meant a protein structure that allows the scaffold protein, generally a peptide, to take on a certain conformation. For example, there are a wide variety of "ministructures" known, sometimes referred to as "presentation structures", that can confer conformational stability or give a random sequence a conformationally restricted form. Proteins interact with each other largely through conformationally constrained domains. Although small peptides with freely rotating amino and carboxyl termini can have potent functions as is known in the art, the conversion of such peptide structures into pharmacologic agents is difficult due to the inability to predict side-chain positions for peptidomimetic synthesis. Therefore the presentation of peptides in conformationally constrained structures will benefit both the later generation of pharmaceuticals and will also likely lead to higher affinity interactions of the peptide with the target protein. This fact has been recognized in the combinatorial library generation systems using biologically generated short peptides in bacterial phage systems. A number of workers have constructed small domain molecules in which one might present randomized peptide structures. Thus, synthetic presentation structures, i.e. artificial polypeptides, are capable of presenting a randomized peptide as a conformationally-restricted domain. In addition, random peptide structures that are not totally random, i.e., that are selected or filtered as described herein may be presented. Preferred presentation structures maximize accessibility to the peptide by presenting it on an exterior loop. Accordingly, suitable presentation structures include, but are not limited to, minibody structures, loops on beta-sheet turns and coiled-coil stem structures in which residues not critical to structure are randomized, zinc-finger domains, cysteine-linked (disulfide) structures, transglutaminase linked structures, cyclic peptides, B-loop structures, helical barrels or bundles, leucine zipper motifs, etc.

By "primary library" as used herein is meant a collection of sequences, preferably optimized and generally, but not always, in the form of a filtered set, a rank-ordered list (e.g. a scored or sampled set), an alignment, a probability distribution table, etc. A primary library is generated as a targeted subset of all or a portion of the sequence space for a particular scaffold protein. That is, a primary library is generated using any number of techniques, either alone or in combination; to reduce the size of the set of sequences likely to take on a particular fold or have a particular protein property. The primary library preferably comprises a set of sequences resulting from computation, which may include energy calculations and/or statistical or knowledge based approaches. In general, it is preferable to have the primary library be large enough to randomly sample a reasonable sequence space to allow for robust secondary libraries. Thus, primary libraries that range from about 50 to about $10^{13}$ are preferred, with from about 1000 to about $10^7$ being particularly preferred, and from about 1000 to about 100,000 being especially preferred.

By "probability parameter" as used herein is meant a parameter that governs the rate at which a given amino acid or rotamer state is sampled during a simulation.

By "protein" as used herein is meant at least two amino acids linked together by a peptide bond. As used herein, protein includes proteins, oligopeptides, polypeptides and peptides. The peptidyl group may comprise naturally occurring amino acids and peptide bonds, or synthetic peptidomimetic structures, i.e. "analogs", such as peptoids (see Simon et al., PNAS USA 89(20):9367 (1992)). The amino acids may either be naturally occurring or non-naturally occurring. The side chains may be in either the (R) or the (S) configuration. In a preferred embodiment, the amino acids are in the (S) or L-configuration.

By "protein numbering scheme" herein is meant, the manner in which, as is known in the art, the residues, or positions, of proteins are generally numbered. The residues, or positions, are generally sequentially numbered starting with the N-terminus of the protein. Thus a protein having a methionine at its N-terminus is said to have a methionine at residue or amino acid position 1, with the next residues as 2, 3, 4, etc. In some embodiments, a set of aligned proteins is numbered together. In such cases, insertions relative to the consensus sequence are denoted by adding a letter after the number; for example, a one-residue insertion between positions 1 and 3 would produce the numbering 1, 2a, 2b, 3. Similarly, deletions relative to the consensus sequence are denoted by skipping a number, for

**EP 1 510 959 A2**

example, a one residue deletion between positions 1 and 3 would produce the numbering 1, 3.

By "protein properties" herein is meant, biological, chemical, and physical properties including, but not limited to, enzymatic activity, specificity (including substrate specificity, kinetic association and dissociation rates, reaction mechanism, and pH profile), stability (including thermal stability, stability as a function of pH or solution conditions, resistance or susceptibility to ubiquitination or proteolytic degradation), solubility, aggregation, structural integrity, the creation of new antibody CDRs, generate new DNA, RNA binding, generate peptide and peptidomimmetic libraries, crystallizability, binding affinity and specificity (to one or more molecules including proteins, nucleic adds, polysaccharides, lipids, and small molecules), oligomerization state, dynamic properties (including conformational changes, allostery, correlated motions, flexibility, rigidity, folding rate), subcellular localization, ability to be secreted, ability to be displayed on the surface of a cell, posttranslational modification (including N- or C-linked glycosylation, lipidation, and phosphorylation), ammenability to synthetic modification (including PEGylation, attachment to other molecules or surfaces), and ability to induce altered phenotype or changed physiology (including cytotoxic activity, immunogenicity, toxicity, ability to signal, ability to stimulate or inhibit cell proliferation, ability to induce apoptosis, and ability to treat disease). As is outlined herein, protein properties may be modulated using the techniques of the invention. When a biological activity is the property, modulation in this context includes both an increase or a decrease in activity.

By "pseudo energy" as used herein is meant an energy-like term derived from non-energetic information. Such pseudo energies are typically used as a mechanism for combining non-energetic information with energy based scoring functions. For example, statistical information arising from structural analysis, sequence alignments, or simulation history may be incorporated into a calculation by their conversion to pseudo energies.

By "recency parameter" as used herein means the application of at least one restraint to the most recent moves of a simulation (see Modern Heuristic Search Methods, edited by V.J. Rayward-Smith, et al., 1996, John Wiley & Sons Ltd., hereby expressly incorporated by reference in its entirety).

By "residue" as used herein is meant an amino acid side chain. A residue may be one of the naturally occurring amino acid side chains or a synthetic analog.

By "scaffold protein" herein is meant a protein for which a library of variants is desired. The scaffold protein is used as input in the protein design calculations, and often is used to facilitate experimental library generation. A scaffold protein may be any protein that has a known structure or for which a structure may be calculated, estimated, modeled or determined experimentally. As outlined more fully below, the scaffold protein may be a wild-type protein from any organism, a variant, a chimeric protein, etc. Preferred embodiments of scaffold proteins are outlined below.

By "secondary library" as used herein is meant a library of amino acid sequences that is derived from a primary library using a variety of approaches discussed further below, including both experimental and computational methods, or combinations thereof. Secondary libraries are generally generated experimentally and analyzed for the presence of members possessing desired protein properties. The secondary library may be either a subset of the primary library, or contain new library members, i.e. sequences that are not found in the primary library. The secondary library typically comprises at least one member sequence that is not found in the primary library, and preferably a plurality of such sequences, although this is not required.

By "selectable gene," "selection gene" or "selectable marker" as used herein Is meant any gene that enables survival and/or reproduction of the cells that express it The marker gene may confer resistance to a selection agent such as an antibiotic, or may provide a protein required for growth.

By "sequence space" herein is meant all sequential combinations of amino acids that are possible for a defined protein and a defined set of positions thereof. For example, the sequence space for all positions of a 100-residue protein is $20^{100}$, and the sequence space for ten selected positions of a protein would be $20^{10}$, if only the twenty naturally occurring amino acids are considered.

By "shuffling", as used herein means recombination of one or more protein, DNA, or RNA sequences. Shuffling may be done experimentally and/or computationally (e.g. "in silico shuffling"). See for example, U.S. Patent 6,319,714; WO 0042559WO 00/42560; and WO 00/42561.

By "solid support" or other grammatical equivalents herein is meant any material that may be modified to contain discrete individual sites appropriate for the attachment or association of beads, other solid support surfaces not in solution, and is amenable to at least one detection method. As will be appreciated by those in the art, the number of possible supports is very large. Possible solid supports include, but are not limited to, glass and modified or functionalized glass, plastics (including acrylics, polystyrene and copolymers of styrene and other materials, polypropylene, polyethylene, polybutylene, polyurethanes, Teflon®, etc.), polysaccharides, nylon or nitrocellulose, resins, silica or silica-based materials including silicon and modified silicon, carbon, metals, inorganic glasses, plastics, optical fiber bundles, and a variety of other polymers. In general, the solid supports allow optical detection and do not themselves appreciably fluoresce.

By "sticky end" as used herein is meant the end of an enzymatically cleaved DNA fragment that has either a 5' or 3' overhang, and has the potential to interact favorably with another sticky end with similar properties.

By "surface positions" as used herein is meant amino acid positions within a scaffold protein (or a variable protein) with a significant degree of solvent accessibility. Methods for the determination of surface positions are outlined below. In a preferred embodiment, only polar amino acids are considered as possible replacement residues at surface positions in protein design calculations.

By "tabu search algorithms" as used herein is meant any algorithms from the class of searching methods in which searching moves are made such that moves already made, or made recently in the history of the search, are either avoided or disfavored.

By "tertiary library" as used herein is meant a library that is generated by computational or experimental modification or manipulation of a secondary library.

By "variant protein sequence" as used herein is meant a protein sequence that differs from another protein sequence. In other words a variant protein sequence has at least one amino acid that differs from the amino acid defined by the starting amino acid sequence in the protein design cycle. As outlined below, this starting amino acid sequence (e.g. the scaffold protein) may be a wild-type sequence or a variant sequence.

By "variable residue position" herein is meant a position at which both the amino acid identity and conformation are allowed to be altered in a protein design calculation. The amino acid identity to which a position may be mutated may be the full set or a subset of the 20 naturally occurring amino acids or may be a set of non-naturally occurring amino acids or synthetic analogs.

By "temperature factor" as used herein is meant a parameter in an optimization algorithm that determines the acceptance criteria for a sampling jump. As will be appreciated by those skilled in the art, high temperature factors allow searches across a broad area of sequence space, and low temperature factors allow searches over a narrow region of sequence space. See Metropolis et al., J. Chem Phys v21, pp 1087, 1953, hereby expressly incorporated by reference.

By "variant strand" as used herein is meant a nucleic acid strand generated using the gene assembly methods outlined herein to differ from the corresponding template nucleic acid sequence by at least one nucleotide or its complement.

All references cited herein are expressly incorporated by reference.

**Introduction**

The present invention is directed to methods of using computational screening of protein sequence libraries (that may comprise up to $10^{80}$ or more members) to select smaller libraries of protein sequences (that may comprise up to $10^{13}$ members), which may then be used in a number of ways. For example, the proteins may actually synthesized and experimentally tested in the desired assay to identify proteins that possess desired properties. Similarly, the library may be subjected to additional computational manipulation in order to create a new library, which may be experimentally tested.

As may be appreciated by those skilled in the art, a variety of user interfaces may be utilized in the present invention. In a preferred embodiment, the interface is designed to maximize usability and efficiency. Furthermore, any or all of the computational methods described below may be automated for increased usability and efficiency.

Computational screening to enrich libraries with proteins possessing desired properties

By computationally screening very large libraries of variant proteins, a greater diversity of protein sequences may be screened (i.e. a larger sampling of sequence space) than is possible using experimental methods alone. Consequently, the probability of identifying proteins with desired properties is increased and greater improvements may be realized compared to the results of purely experimental methods.

The number of possible protein sequences grows exponentially with the number of positions that are randomized. Generally, only up to $10^{12}$ - $10^{15}$ sequences may be contained in a physical library because of experimental and physical constraints (e.g. transformation efflciency, instrumentation limits, the cost of producing large numbers of biopolymers, and, for larger libraries, the number of carbon atoms in the universe, etc.) Often, practical considerations may limit the library size to $10^6$ or fewer. These limits are reached for only 10 amino acid positions. In contrast, using the automated protein design techniques outlined below, virtual libraries of protein sequences that are vastly larger than experimental libraries may be generated and analyzed: up to $10^{80}$ or more candidate sequences may be screened computationally.

Using experimental methods alone, only a sparse sampling of sequences is possible in the search for proteins or peptides with desired properties, lowering the chance of success (both finding any proteins that possess the desired properties, and finding proteins that surpass the minimum acceptable criteria) and almost certainly missing desirable candidates. Because of the random nature of the mutations in experimental libraries, most of the candidates in the library are not suitable (for example, a large fraction of sequence space encodes unfolded, misfoided, incompletely folded, partially folded, or aggregated proteins), resulting in an enormous waste of the time and resources required to produce the library. In effect, when experimental methods alone are used, the screened library is composed of a large amount of "wasted sequence space".

Computational pre-screening may be used to generate and/or enrich libraries of variant proteins that possess desired protein properties. An experimental library consisting of the favorable candidates found in the virtual library screening may then be generated, resulting In a much more efficient use of the time, money and effort required to construct and screen an experimental library. In effect, when computational pre-screening is used the screened library is composed of primarily productive sequence space. As a result, computational pre-screening increases the chances of identifying one or more proteins that possess the desired protein properties.

Computational pre-screening may also be beneficial when the library of mutants is sufficiently small to be screened experimentally (that is, a library size of less than $10^{15}$). It reduces the number of mutants that must be tested experimentally, thereby reducing the cost and difficulty associated with protein engineering and experimental screening.

While experimental methods are typically limited to $10^7$ -$10^{13}$ sequences, computational methods have the unique ability to screen $10^{80}$ sequences or more. However, purely computational methods are limited by an incomplete knowledge of the structure-function relationship in proteins. In contrast, experimental methods are capable of identifying sequences with desired protein properties, even in cases where the causative link between sequence and observed protein properties is not understood. Thus, computational pre-screening followed by experimental screening of the most promising constructs combines the best features of computational and experimental methods.

Computational screening for target identification

In a preferred embodiment, the present invention finds use in the screening of random peptide libraries for the purpose of target identification. In this application, random peptides are screened for the ability to cause a phenotypic alteration. Following identification of the active peptides, their interaction partners, which will typically be other proteins, may be determined. These proteins are likely to be involved in the biochemical pathway associated with a given phenotypic alteration, and therefore could potentially serve as new drug targets. This approach is analogous to the chemical genetics methods that have been developed for small molecule libraries (Chen et al. 6:221-235 (1999), Knockaert et al. Chem. Biol. 7(6):411-22, (2000)).

Screening small molecule libraries for compounds that are capable of inducing specific alterations in cellular physiology or phenotype has led to the discovery of proteins that function in a variety of biochemical and signal transduction pathways. Cyclosporin A (CsA) and FK506, for example, were selected in standard pharmaceutical screens for inhibition of T-cell activation. It is noteworthy that while these two drugs bind completely different cellular proteins, cyclophilin and FK5O6 binding protein (FKBP), respectively, the effect of either drug is virtually the same: profound and specific suppression of T-cell activation, phenotypically observable in T cells as inhibition of mRNA production dependent on transcription factors such as NF-AT and NF-KB.

Chemical genetics approaches have typically used libraries of small molecules; however, libraries of peptides or proteins could be used instead. Computational pre-screening of the peptide libraries could be used to maximize the diversity of properties in the library and to select structured peptides that are especially likely to bind other molecules with high affinity.

Computational screening for fold identification

The present invention also finds use in fold identification. Structural and functional properties of protein sequences, such as those deriving from various genome projects, may often be inferred from sequence similarity to proteins whose structural and/or functional properties have been characterized. One limitation of this approach is that many newly discovered sequences lack sufficient sequence similarity with any of the better characterized proteins.

In a preferred embodiment, a three-dimensional database is created by modifying a known protein structure to incorporate particular amino acid residues required for a characteristic property or function, as is described in WO 00/23474, expressly incorporated herein by reference. This allows the creation of a database that can be used in a manner similar to other "structural alignment" programs. That is, by using the protein design cycle systems outlined herein, a variety of amino acid sequences that will take on a particular structural fold are generated. These sequences represent a set of artificial sequences that will take on a particular conformation. This database may be searched against protein databases to identify new proteins having structural similarity with the known protein. Thus, proteins can be identified that make take on a particular fold but do not have enough sequence homology to a naturally occurring protein to be chosen using known alignment programs. In some cases, this will allow the assignment of putative functional information as well; for example, by identifying proteins with structural homology to a particular class of enzyme or ligand, the new protein can be assigned similar function. This finds particular use in identifying proteins that have been sequenced but to which no structure and/or function has been assigned.

In addition, the database could contain additional computationally generated sequences that are predicted to be compatible with a given structure and/or function. Computationally supplemented d databases may contain a significant greater diversity and total number of sequences than databases that rely solely on experimental results. Consequently, the fraction of sequences that may be classified into a protein family will be larger using a computationally supplemented database than using a purely experimental database. Fold identification using PDA™ technology and bioinformatics tools (e.g. dynamic programming algorithms, BLAST search), may then be used to identify new drug targets and antidotes to biological weapons.

As an example of this concept, the sequencing of new genomes will reveal proteins, structural motifs, and domains that are unique to certain genomes. For example, there may be some domains that are unique to bacterial or viral genomes and do not exist in eukaryotic genomes. PDA™ technology and/or the other computational methods outlined herein may be used to identify sequences that are compatible with these structures. Bacterial and viral genomes may then be searched to identify additional proteins that are likely to fold to the structures, but could not be identified as homologs using traditional methods. The resulting proteins may serve as novel drug targets that could be used to discover new classes of antibiotics and antiviral drugs.

Approach to library generation

The invention describes novel methods to create secondary libraries derived from very large computational mutant libraries. These methods allow the rapid experimental and/or computational testing of large numbers of computationally designed sequences.

As more fully outlined below, the invention may take on a wide variety of configurations. In general, primary libraries are generated computationally. This may be done in a wide variety of ways, including, but not limited to, sequence alignments of related proteins, structural alignments, structural prediction models, SCMF methods, or preferably protein design automation™ (PDA™) technology computational analysis.

Once the primary library is generated, it may be manipulated in a variety of ways. in one embodiment, a different type of computational analysis may be done; for example, a new type of ranking may be performed. In a preferred embodiment, some subset of the primary library is then experimentally generated to form a secondary library. Alternatively, some or all of the primary library members are recombined to form a secondary library, resulting in a secondary library that contains sequences not included in the primary library. Again, this may be done either computationally or experimentally or both.

Accordingly, the present invention provides computational and experimental methods for generating secondary libraries of scaffold protein variants.

Overview of PDA™ Technology Methodology

In a preferred embodiment, the computational method used to generate the primary library is Protein Design Automation™ (PDA™) technology, as is described in U.S.S.N.s 60/061,097, 60/043,464, 60/054,678, 09/127,926 and PCT US98/07254, all of which are expressly Incorporated herein by reference. Briefly, PDA™ technology may be described as follows. A known, generated or homologous protein structure is used as the starting point The residues to be optimized are then identified, which may be the entire sequence or subset(s) thereof. The side chains of any positions to be modified are then removed. The amino acids that will be considered at each position are selected. (for example, core residues generally will be selected from the set of hydrophobic residues, surface residues generally will be selected from the hydrophilic residues, and boundary residues may be either). Each amino acid residue may be represented by a discrete set of allowed conformations, called rotamers. Interaction energies are calculated between each residue in a given rotamer and the backbone and between each pair of residues in each of their rotamers at different positions. Combinatorial search algorithms, typically DEE and Monte Carlo, are used to identify the optimum amino acid sequence and additional low energy sequences which will comprise the primary library.

PDA™ technology, viewed broadly, has four components that may be varied to alter the output (i.e. the primary library): generation of the template or templates, choice of amino acid identities and conformations considered at each position, the scoring functions used in the process; and the optimization strategy.

**Selection and preparation of the scaffold protein**

Source of Three-dimensional Structure

The scaffold protein may be any protein for which a three dimensional structure (that is, three dimensional coordinates for each atom of the protein) is known or may be generated. The three dimensional structures of proteins may be determined using X-ray crystallographic techniques, NMR techniques, *de novo* modeling, homology modeling, etc. In general, if X-ray structures are used, structures at 2 A resolution or better are preferred, but not required. Suitable protein structures include, but are not limited to, all of those found in the Protein Data Base compiled and serviced by the Research Collaboratory for Structural Bioinformatics (RCSB, formerly the Brookhaven National Lab).

Scope of Scaffold

The scaffold used in protein design calculations may comprise an entire protein or peptide, a subset of a protein such as a domain (including functional domains such as enzymatic domains, substrate-binding domains, regulatory domains, dimerization domains, etc.), motif, site, or loop. The scaffold protein may comprise more than one protein chain. That is, the scaffold may be an oligomer (including but not limited to dimers, trimers, hexamers, 60-mers such as viral coats, and long protein chains such as actin filaments) or a multi-protein complex (including but not limited to ligand-receptor pairs, antibody-antigen pairs, ribosome complexes, proteosome complexes, transcription complexes, chaperone complexes, the splicesome, molecular motors, focal adhesion complexes, multi-protein signaling complexes, etc.). The scaffold may additionally contain non-protein components, including but not limited to small molecules, substrates, cofactors, metals, water molecules, prosthetic groups, nucleic acids such as DNA and RNA, sugars, and lipids.

Source of the scaffold protein

The scaffold proteins may be from any organism, including prokaryotes and eukaryotes, with proteins from bacteria, fungi, viruses, extremophiles such as the archaebacteria, insects, fish, animals (particularly mammals and particularly human) and birds all possible. The scaffold protein does not necessarily need to be naturally occurring, for example the scaffold protein could be a designed protein, or a protein selected by a variety of methods including but not limited to directed evolution (Farinas et al. Current Opinion in Biotechnology 12:545-551 (2001) Morawski et al. Biotechnology and Bioengineering 76:99-107 (2001), Stemmer Nature 370(6488): 389-91 (1994) Ness et al. Adv. Protein. Chem. 55:261-92 (2000)), DNA shuffling (Maxygen, Enchira, Diversa) or ribosome display (Hanes et al. Methods in Enzymology 328:404-430 (2000); Hanes and Pluckthun, Proc. Natl. Acad. Sci. USA 94:4937-4942 (1997); Roberts and Szostak, Proc. Natl. Acad. Sci. USA 94, 12297-302 (1997).

Examples of suitable scaffolds

As will be appreciated by those skilled in the art, any number of scaffold proteins find use in the present invention. Suitable proteins include, but are not limited to, industrial and pharmaceutical proteins, including ligands, cell surface receptors, antigens, antibodies, cytokines, hormones, transcription factors, signaling modules, cytoskeletal proteins and enzymes.

Specifically, preferred scaffold proteins include, but are not limited to, those with known or predictable structures (including variants):

- cytokines (IL-1ra (+receptor complex), IL-1 (receptor alone), IL-1a, IL-1b (including variants and or receptor complex), IL-2, IL-3, IL-4, IL-5, IL-6, IL-8, IL-10, IFN-$\beta$, INF-$\gamma$, IFN-$\alpha$-2a; IFN-$\alpha$-2B, TNF-$\alpha$; CD40 ligand (chk), Human Obesity Protein Leptin, Granulocyte Colony-Stimulating Factor, Bone Morphogenetic Protein-7, Ciliary Neurotrophic Factor, Granulocyte-Macrophage Colony-Stimulating Factor, Monocyte Chemoattractant Protein 1, Macrophage Migration Inhibitory Factor, Human Glycosylation-Inhibiting Factor, Human Rantes, Human Macrophage Inflammatory Protein 1 Beta, human growth hormone, Leukemia Inhibitory Factor, Human Melanoma Growth Stimulatory Activity, neutrophil activating peptide-2, Cc-Chemokine Mcp-3, Platelet Factor M2, Neutrophil Activating Peptide 2, Eotaxin, Stromal Cell-Derived Factor-1, Insulin, Insulin-like Growth Factor I, Insulin-like Growth Factor II, Transforming Growth Factor B1, Transforming Growth Factor B2, Transforming Growth Factor B3, Transforming Growth Factor A, Vascular Endothelial growth factor (VEGF), acidic Fibroblast growth factor, basic Fibroblast growth factor, Endothelial growth factor, Nerve growth factor, Brain Derived Neurotrophic Factor, Ciliary Neurotrophic Factor, Platelet Derived Growth Factor, Human Hepatocyte Growth Factor, Fibroblast Growth Factor (including but not limited to alternative splice variants , abundant variants, and the like), Glial Cell-Derived Neurotrophic Factor, and haemopoietic receptor cytokines (including but not limited to erythropoietin, thrombopoietin, and prolactin), APM1 (including, but not limited to adipose most abundant gene transript 1), and the like.
- other extracellular signaling moieties, including, but not limited to, Sonic hedgehog, protein hormones such as chorionic gonadotrophin and leutenizing hormone.
- blood clotting and coagulation factors including, but not limited to, TPA and Factor VIIa; coagulation factor IX; coagulation factor X ; PROTEIN S protein; Fibrinogen and Thrombin; ANTITHROMBIN III; streptokinase and urokinase, retevase, and the like.
- transcription factors and other DNA binding proteins, including but not limited to, histones, p53; myc; PIT1; NFkB;

AP1;JUN; KD domain, homeodomain, heat shock transcription factors, stat, zinc finger proteins (e.g. zif268).

- Antibodies, antigens, and trojan horse antigens, including, but not limited to, immunoglobulin super family proteins, including but not limited to CD4 and CD8, Fc receptors, T-cell receptors, MHC-I, MHC-II, CD3, and the like. Also, immunoglobulin-like proteins, including but not limited to fibronectin, pkd domain, integrin domains, cadhrin, invasins, cell surface receptors with Ig-like domains, and the like. Intrabodies, and the like; Anti-Her/2 neu antibody (e.g. Herceptin); Anti-VEGF; Anti-CD20 (Rituxan), among others.
- intracellular signaling modules, including, but not limited to, kinase s, phosphatases, G-proteins Phosphatidylinositol 3-kinase (PI3-kinase) kinase, Phosphatidylinositol 4-kinase, wnt family members including but not limited to wnt-1 through wnt 15, EF hand proteins including calmodulin, troponin C, S100B, calbindin and D9k; NOTCH; MEK; MAPK; ubitquitin and ubiquitin like proteins, including UBL1, UBL5, UBL3 and UBL4, and the like.
- viral proteins, including, but not limited to, hemagglutinin trimerization domain and HIV Gp41 ectodomain (fusion domain); viral coat proteins, viral receptors, integrases, proteases, reverse transcriptases.
- receptors, including, but not limited to, the extracellular region of human tissue factor cytokine-binding region Of Gp130, G-CSF receptor, erythropoietin receptor, Fibroblast Growth Factor receptor, TNF receptor, IL-1 receptor, IL-1 receptor/IL1ra complex, IL-4 receptor, INF-γ receptor alpha chain, MHC Class I, MHC Class II , T Cell Receptor, Insulin receptor, insulin receptor tyrosine kinase and human growth hormone receptor, Lectins; GPCRs, including but not limited to G-Protein coupled receptors; ABC Transporters/ Multidrug resistance proteins; Na and K channels; Nuclear Hormone Receptors; Aquaporins; Transporters, RAGE (receptor for advanced glycan end points), TRK -A, -B, -C, and the like, and haemopoietic receptors.
- enzymes including, but not limited to, hydrolases such as proteases/proteinases, synthases/synthetases/ligases, decarboxylases/lyases, peroxidases, ATPases, carbohrases, lipases; isomerases such as racemases, epimerases, tautomerases, or mutases; transferases, hydrolases, kinases, reductases/oxidoreductases, hydrogenases, polymerases, phophatases, and proteasomes anti-proteasomes, (e.g., MLN341). Suitable enzymes include, but limited to, those listed in the Swiss-Prot enzyme database.
- Additional proteins including but not limited to heat shock proteins, ribosomal proteins, glycoproteins, motor proteins, transporters, drug resistance proteins, kinetoplasts and chaperonins.
- Antimicrobial peptides
- small proteins including but not limited to metal ligand and disulfide-bridged proteins such as metallothionein, Kunitiz-type inhibitors, crambin, snake and scorpion toxins, and trefoil proteins; antimicrobial peptides such as defensins, thoredoixn, fereodoxin, transferetin, and the like.
- protein domains and motifs including, but not limited to, SH-2 domains, SH-3 domains, Pleckstrin homology domains, WW domains, SAM domains, kinase domains, death domains, RING finger domains, Kringle domains, heparin-binding domains, cysteine-rich domains, leucine zipper domains, zinc finger domains, nucleotide binding motifs, transmembrane helices, and helix-turn-helix motifs. Additionally, ATP/GTP-binding site motif A; Ankyrin repeats; fibronectin domain; Frizzled (fz) domain; GTPase binding domain; C-type lectin domain; PDZ domain; 'Homeobox' domain; Krüeppel-associated box (KRAB); Leucine zipper; DEAD and DEAH box families; ATP-dependent helicases; HMG1/2 signature; DNA mismatch repair proteins mutL / hexB / PMS1 signature; Thioredoxin family active site; Thioredoxins; Annexins repeated domain signature; Clathrin light chains signatures; Myotoxins signature; Staphylococcal enterotoxins / Streptococcal pyrogenic exotoxins signatures; Serpins signature; Cysteine proteases inhibitors signature; Chaperonins; Heat shock; WD domains; EGF-like domains; Immunoglobulin domains, Immunoglobulin-like proteins and the like.
- specific protein sites or other subsets of residues, including but not limited to protease cleavage/ recognition sites, phosphorylation sites, metal binding sites, and signal sequences. Additionally, proteins having post-translational modifications include, but are not limited to: N-glycosylation site; O-glycosylation site; Glycosaminoglycan attachment site; Tyrosine sulfation site; cAMP- and cGMP; dependent protein kinase phosphorylation site; Protein kinase C phosphorylation site; Casein kinase II phosphorylation site; Tyrosine kinase phosphorylation site; N-myristoylation site; Amidation site; Aspartic acid and asparagine hydroxylation site; Vitamin K-dependent carboxylation domain; Phosphopantetheine attachment site; Prokaryotic membrane lipoprotein lipid attachment site; Prokaryotic N-terminal methylation site; Prenyl group binding site (CAAX box); Intein N- and C-terminal splicing motif profiles, and the like.
- Proteins involved in motility, including but not limited to chemokines, S100 family proteins (including but not limited to NRAGE).
- Peptides - defensins
- peptide ligands including, but not limited to, a short region from the HIV-1 envelope cytoplasmic domain (shown to block the action of cellular calmodulin), regions of the Fas cytoplasmic domain (death-inducing apoptotic or G protein inducing functions), magainin, a natural peptide derived from Xenopus (anti-tumor and anti-microbial activity), short peptide fragments of a protein kinase C isozyme, βPKC (blocks nuclear translocation of full-length βPKC in Xenopus oocytes following stimulation), SH-3 target peptides, naturitic peptides (AMP, BMP, and CMP), and fibrinopeptides and neuropeptides.

- presentation scaffolds or "ministructures" including, but are not limited to, minibody structures (see for example Bianchi et al., J. Mol. Biol. 236(2):649-59 (1994), and references cited therein, all of which are incorporated by reference), maquettes (Grosset et al. Biochemistry 40:5474-5487 (2001)), loops on beta-sheet turns and coiled-coil stem structures (see, for example, Myszka et al., Biochem. 33:2362-2373 (1994) and Martin et al., EMBO J. 13(22):5303-5309 (1994), incorporated by reference), zinc-finger domains, transglutaminase linked structures, cyclic peptides, B-loop structures, coiled coils, helical bundies, helical hairpins, and beta hairpins.
- Ion channel protein domains, including but not limited to sodium, calcium, potassium, and chloride, including their component subunit Examples of extracellular ligand-gated ion channels include nAChR receptors, GABA and glycine, 5H-T, MOD-1, P(2X), glutamate, NMDA, AMPA, Kainate receptors, GluR-B, ORCC, P2X3, Inward rectifying channels, ROMK, IRK, BIR, and the like. Examples of voltage-gated ion channels, Examples of intracellular ligand-gated ion channels, Mechanosensative and cell volume-regulated ion channels, and the like.

In addition, a preferred embodiment utilizes scaffold proteins such as random peptides. That is, there is a significant amount of work being done in the area of utilizing random peptides in high throughput screening techniques to identify biologically relevant (particularly disease states) proteins. The methods of the invention are particularly relevant for computationally prescreening random peptide libraries to drastically reduce the amount of wet chemistry that must be done, by removing sequences that are unlikely to be successful. Different design criteria can be used to produce candidate sets that are biased for properties such as charge, solubility, or active site characteristics (polarity, size), are biased to have certain amino acids at certain positions or to take on certain folds. That is, the peptides (which may be the scaffold protein or the candidate agents, as outlined below) are randomized, either fully randomized or they are biased in their randomization, e.g. in nucleotide/residue frequency generally or per position. By "randomized" or grammatical equivalents herein is meant that each nucleic acid and peptide consists of essentially random nucleotides and amino acids, respectively. Thus, any amino acid residue may be incorporated at any position. The synthetic process can be designed to generate randomized peptides and/or nucleic acids, to allow the formation of all or most of the possible combinations over the length of the nucleic acid, thus forming a library of randomized candidate nucleic acids.

In one embodiment, the library is fully randomized, with no sequence preferences or constants at any position. In a preferred embodiment, the library is biased. That is, some positions within the sequence are either held constant, or are selected from a limited number of possibilities. For example, in a preferred embodiment, the nucleotides or amino acid residues are randomized within a defined class, for example, of hydrophobic amino acids, hydrophilic residues, sterically biased (either small or large) residues, towards the creation of cysteines, for cross-linking, prolines for SH-3 domains, serines, threonines, tyrosines or histidines for phosphorylation sites, etc., or to purines, etc. In a preferred embodiment, the bias is towards peptides or nucleic acids that interact with known classes of molecules. For example, it is known that much of intracellular signaling is carried out via short regions of polypeptides interacting with other polypeptides through small peptide domains. In addition, agonists and antagonists of any number of molecules may be used as the basis of biased randomization of candidate bioactive agents as well.

In general, the generation of a prescreened random peptide libraries may be described as follows. Any structure, whether a known structure, for example a portion of a known protein, a known peptide, etc., or a synthetic structure, can be used as the backbone for computational screening. For example, structures from X-ray crystallographic techniques, NMR techniques, de novo modelling, homology modelling, etc. may all be used to pick a backbone for which sequences are desired. Similarly, a number of molecules or protein domains are suitable as starting points for the generation of biased randomized candidate bioactive agents. A large number of small molecule domains are known, that confer a common function, structure or affinity. In addition, as is appreciated in the art, areas of weak amino acid homology may have strong structural homology. A number of these molecules, domains, and/or corresponding consensus sequences, are known, including, but are not limited to, SH-2 domains, SH-3 domains, Pleckstrin, death domains, protease cleavage/recognition sites, enzyme inhibitors, enzyme substrates, Traf, etc. Similarly, there are a number of known nucleic acid binding proteins containing domains suitable for use in the invention. For example, leucine zipper consensus sequences are known. Thus, in general, known peptide ligands can be used as the starting scaffold backbone for the generation of the primary library.

In a preferred embodiment, the scaffold protein is a variant protein, including, but not limited to, mutant proteins comprising one or a plurality of substitutions, insertions or deletions, including chimeric genes, and genes that have been optimized in any number of ways, including experimentally or computationally.

In a preferred embodiment, the scaffold protein is a chimeric protein. A chimeric protein (sometimes referred to as a "fusion protein") in this context means a protein that has sequences from at least two different sequences operably linked or fused. The chimeric protein may be made using either a single linkage point or a plurality of linkage points. In addition, the source of the parent protein sequences may be as listed above for scaffold proteins, e.g. prokaryotes, eukaryotes, including archebacteria and viruses, etc.

As will be appreciated by those in the art, chimeric proteins may be made from different naturally occurring proteins in a gene family (e.g. one with recognizable sequence or structural homology) or by artificially joining two or more distinct genes. For example, the binding domain of a human protein may be fused with the activation domain of a mouse gene, etc

The sequence of the chimeric gene may be been constructed synthetically (e.g. arbitrary or targeted portions of two or more genes are crossed over randomly or purposely), experimentally (e.g. through homologous recombination or shuffling techniques) or computational (e.g. using genetic annealing programs, "in silico shuffling", alignment programs, etc.). For the purposes of the invention, these techniques can be done at the protein or nucleic acid level.

In a preferred embodiment, the scaffold protein is actually a product of a computational design cycle and/or screening process. That is, a first round of the methods of the invention may produce one or more sequences for which further analysis is desired.

Although several classes of proteins have been stated herein, this should not be construed as an exhaustive list, but rather some examples of proteins that may be optimized using the computational methodologies outlined herein, including PDA™ technology.

Preparation of Protein Backbone for Calculations

The protein scaffold may be modified or altered at the beginning (and optionally, but not preferably, in the middle or end) of a protein design calculation, or the unaltered scaffold may be used. It is also possible to use methods in which the protein scaffold is modified during later steps of a design calculation, including during the energy calculation and optimization steps.

In a preferred embodiment, protein scaffold backbone (comprising, the nitrogen, the carbonyl carbon, the $\alpha$-carbon, and the carbonyl oxygen, along with the direction of the vector from the $\alpha$-carbon to the $\beta$-carbon) may be altered prior to the computational analysis, for example by varying a set of parameters called supersecondary structure parameters. See for example U.S. Patent Nos. 6,269,312, 6,188,965, and 6,403,312, all of which are herein expressly incorporated by reference. Alternatively, the protein scaffold is altered using other methods, such as manually, including directed or random perturbations

Most protein structures contain loop regions that are flexible or conformationally heterogeneous. The protein backbone may be modified in the loop regions using methods such as molecular dynamics simulations and analysis of databases of known loop structures. In addition, loops may be modified in order to incorporate new structural or functional properties such as new binding sites.

In a preferred embodiment, the design cycle is done using a plurality or set of scaffold proteins. That is, the scaffold may be a set of protein structures created by perturbing the starting structure. This may be done using any number of techniques, including molecular dynamics and Monte Carlo analysis, that after the protein structure (including changing the backbone and side chain torsion angles.) Alternatively, an ensemble of structures such as those obtained from NMR may be used as the scaffold. These backbone modifications are particularly useful for enhancing the diversity of sequences derived from protein design simulations. Similarly, other useful ensembles include sets of related proteins, sets of related structures, artificial created ensembles, etc.

In a preferred embodiment, once a protein structure backbone is generated (with alterations, as outlined above), explicit hydrogens are added if not included within the structure. For example, if the structure was determined using X-ray crystallography, hydrogens are typically added.

In a preferred embodiment, energy minimization of the structure is run to relax strain, including strain due to van der Waals clashes, unfavorable bond angles, and unfavorable bond lengths. In an especially preferred embodiment, this is done by doing a number of steps of conjugate gradient minimization (see Mayo et al., J. Phys. Chem. 94: 8897 (1990)) of atomic coordinate positions to minimize the Dreiding force field with no electrostatics. Generally from 10 to 250 steps is preferred, with 50 steps being most preferred.

Identification of Variable, Floated, and Fixed Positions

In a preferred embodiment, all of the residue positions of the protein are variable. This is particularly desirable for smaller proteins, although the present methods allow the design of larger proteins as well. In an alternate preferred embodiment, only some of the residue positions of the protein are variable, and the remainder are fixed or floated. In this embodiment, the variable residues may be at least one, or anywhere from 0.001 % to 99.999% of the total number of residues. Thus, for example, it may be possible to change only a few (or one) residues, or most of the residues, with all possibilities in between.

In an alternate embodiment, only one or two residue positions are variable and the residue positions within a small distance of, for example, 4Å to 6Å of the variable residue positions are floated. In this embodiment, it is possible to conduct separate calculations for different positions and then combine the results to yield protein variants with multiple mutations. Using the results from one calculation as a starting point for the next calculation one residue position at a time, the optimization procedure may be iterative. Iteration may be performed until a consistent result is reached.

In a preferred embodiment, residues which may be fixed include, but are not limited to, structurally or biologically functional residues. For example, residues which are known to be important for biological activity, such as the residues which form the active site of an enzyme, the substrate binding site of an enzyme, the binding site for a binding partner (ligand/receptor, antigen/antibody, etc.), phosphorylation or glycosylation sites, or structurally important residues, such as cysteines participating in disulfide bridges, metal binding sites, critical hydrogen bonding residues, residues critical for backbone conformation such as proline or glycine, residues critical for packing interactions, etc. may all be fixed or floated.

Similarly, residues which may be chosen as variable residues may be those that confer undesirable biological attributes, such as susceptibility to proteolytic degradation, unwanted oligomerization or aggregation, glycosylation sites which may lead to unwanted immune responses, unwanted binding activity, unwanted allostery, undesirable enzyme activity, etc.

Alternatively, residues that confer desired protein properties may be specifically targeted for variation. In a preferred embodiment, this design strategy may be used to alter properties such as binding affinity and specificity and catalytic efficiency and mechanism. A region such as a binding site or active site may be defined, for example, to include all residues within a certain distance, for example 4 - 10 Å, or preferably 5 Å, of the residues that are in van der Waals contact with the substrate or ligand. Alternatively, a region such as a binding site or active site may be defined using experimental results, for example, a binding site could include all positions at which mutation has been shown to affect binding.

Select Amino Acids to be Considered at Each Position

A set of amino acid side chains is assigned to each variable position. That is, the set of possible amino acid side chains that will be considered at each particular position is chosen. In one embodiment, variable positions are not classified and all amino acids are considered at each variable position. Alternatively, a subset of amino acids are considered at each variable position. Methods for determining subsets of amino acids include, but are not limited to, those discussed below. Any combination of classification methods, including no classification, may be applied to the different variable positions.

In a preferred embodiment, all amino acid residues are allowed at each variable residue position identified in the primary library. That is, once the variable residue positions are identified, a secondary library comprising every combination of every amino acid at each variable residue position is made.

In a preferred embodiment, subsets of amino acids are chosen to maximize coverage. Additional amino acids with properties similar to those contained within the primary library may be manually added. For example, if the primary library includes three large hydrophobic residues at t given position, the user may chose to include additional large hydrophobic residues at that position when generating the secondary library. In addition, amino acids in the primary library that do not share similar properties with most of the amino acids at a given position may be excluded from the secondary library. Alternatively, subsets of amino acids may be chosen from the primary library such that a maximal diversity of side chain properties is sampled at each position. For example, if the primary library includes three large hydrophobic residues at a given position, the user may chose to include only one of them in the secondary library, in combination with other amino acids that are not large and hydrophobic.

In a preferred embodiment, each variable position is classified as either a core, surface or boundary residue position. The classification of residue positions as core, surface or boundary may be done in several ways, as will be appreciated by those in the art. In a preferred embodiment, the classification is done via a visual scan of the original protein scaffold and assigning a classification based on a subjective evaluation of one skilled in the art of protein modeling. Alternatively, a preferred embodiment, called RESCLASS, utilizes an assessment of the orientation of the $C\alpha$-$C\beta$ vectors relative to a solvent accessible surface computed using only the template Ca atoms, as outlined in U. S. Patent Nos. 6,269,312, 6,188,965, and 6,403,312, and expressly herein incorporated by reference. Alternatively, a surface area calculation may be done. In an alternate embodiment, the results of the RESCLASS calculation are used in conjunction with the results of a surface area calculation in order to classify residue positions.

A core residue will generally be selected from a set of hydrophobic residues consisting of alanine, valine, isoleucine, leucine, phenylalanine, tyrosine, tryptophan, and methionine (in some embodiments, methionine may be removed from the set). Similarly, surface positions are generally selected from a set of hydrophilic residues consisting of alanine, serine, threonine, aspartic acid, asparagine, glutamine, glutamic acid, arginine, lysine and histidine. Finally, boundary positions are generally chosen from alanine, serine, threonine, aspartic acid, asparagine, glutamine, glutamic acid, arginine, lysine histidine, valine, isoleucine, leucine, phenylalanine, tyrosin e, tryptophan, and methionine.

In a preferred embodiment, proline, cysteine and glyclne are not included in the list of possible amino acid side chains, and thus the rotamers for these side chains are not used. However, in an alternate preferred embodiment, when the variable residue position has a φ angle (that is, the dihedral angle defined by 1) the carbonyl carbon of the preceding amino acid; 2) the nitrogen atom of the current residue; 3) the α-carbon of the current residue; and 4) the carbonyl carbon of the current residue) greater than 0 degrees, the position is set to glycine to minimize backbone strain. in an alternate embodiment, cysteine is considered at positions where disulfide bonds are desired. In a nother alternate embodiment, proline is considered at positions whose backbone conformation is allowable for proline.

As will be appreciated by those in the art, there is a computational benefit to classifying the residue positions, as it decreases the combinatorial complexity of the problem. It should also be noted that there may be situations where alternative classification approaches will be applied or where the sets of core, boundary and surface residues are altered from those described above; for example, under some circumstances, one or more amino acids is either added or subtracted from the set of allowed amino acids. For example, hydrophobic residues may be included at solvent exposed positions in order to confer desired oligomerization or ligand binding activity, and polar residues may be included in the core of the protein in order to construct an active site or a binding site. Similarly, in one embodiment, only residues capable of forming N-capping interactions are included at the position immediately preceding each helix, and amino acids that interact unfavorably with the helix dipole are subtracted from the set of polar residues at the three positions at the beginning and end of each helix.

In a preferred embodiment, the set of amino acids allowed at each position is determined using sequence or structure alignment methods. For example, the set of amino acids allowed at each position may comprise the set of amino acids that is observed at that position in the alignment, or the set of amino acids that is observed most frequently in the alignment.

In another preferred embodiment, the set of amino acids allowed at each position comprises the set of amino acids that are known to interact with a particular dass of molecules or to serve a specific function. Possible sets include, but are not limited to, residues that may ligate or coordinate to certain metals (such as zinc, copper, iron, and molybdenum), residues that may undergo posttranslational modification (such as phosphorylation, glycosylation, prenylation, and lipidation), and residues that are amenable to synthetic modification. Synthetic modifications include, but are not limited to, alkylation or acylation which includes but is not limited to PEGylation, biotinylation, fluorophore conjugation, acetylation, oxidative or reductive homo- or heterooligomerization, native ligation, conjugation to synthetic mono- and oligosaccharides, and covalent or non-covalent attachment to a solid support (e.g. glass beads, glass slides, or 96-well plates). Sites of synthetic modifications include, but are not limited to, the amide N-H, the amino acid side chains, the amino or carboxyl terminus of the protein, or any of the various posttranslational modifications.

In a preferred embodiment, the set of allowed amino acids includes one or more non-natural or noncanonical amino acids. Synthetic modifications of the non-natural or non-canonical amino acids are also viable. In addition to the modifications listed above, these synthetic transformations include, but are not limited to intra- and intermolecular metal mediated couplings such as the Heck reaction or Suzuki coupling and conjugation through shiff base formation. In a preferred embodiment, the set of allowed amino acids includes more than one charge state for some or all of the acidic or basic residues (that is, arginine, lysine, histidine, glutamic acid, aspartic acid, cysteine, and tyrosine).

## Select the Set of Rotamers That Will Be Used to Model Each Residue Type

In a preferred embodiment, a set of discrete side chain conformations, called rotamers, are considered for each amino acid. Thus, a set of rotamers will be considered at each variable and floated position. Rotamers may be obtained from published rotamer libraries (see Lovel et al., Proteins: Structure Function and Genetics 40:389-408 (2000) Dunbrack and Cohen Protein Science 6:1661-1681 (1997); DeMaeyer et al., Folding and Design 2:53-66 (1997); Tuffery et al. J. Biomol. Struct. Dyn. 8:1267-1289 (1991), Ponder and Richards, J. Mol. Biol. 193:775-791 (1987)), from molecular mechanics or ab initio calculations, and using other methods. In a preferred embodiment, a flexible rotamer model is used (see Mendes et. al., Proteins: Structure, Function, and Genetics 37:530-543 (1999)) Similarly, artificial generated rotamers may be used, or augment the set chosen for each amino acid and/or variable position. In a preferred embodiment, at least one conformation that is not low in energy is included in the list of rotamers. In an alternative embodiment, the identity of each amino acid, rather than specific conformational states of each amino acid, are used, i.e., use of rotamers is not essential.

## Generating ranks or lists of possible sequences

In n essence, any computational methods that may result in either the relative ranking of the possible sequences of a protein or a list of suitable sequences may be used to generate a primary library. As will be appreciated by those in the art, any of the methods described herein or known in the art may be used. Each method may be used alone, or in combination with other methods. In a preferred embodiment, knowledge-based and statistical methods are used. Alternatively, methods that rely on energy calculations may also be used. Protein design methods use various criteria to screen sequences, resulting in sequences that are likely to possess desired properties. The design criteria may be altered to generate primary libraries that are likely to contain proteins possessing a different set of desired properties.

<u>Knowledge-based and Statistical Methods</u>

In a preferred embodiment, sequence and/or structural alignment programs may be used to generate primary libraries. For example, various alignment methods may be used to create sequence alignments of proteins related to the target structure (see for example Altschul et al., J. Mol. Biol. 215(3): 403 (1990), incorporated by reference). Sequences may be related at the level of primary, secondary, or tertiary structure. Alternatively, sequences may be related by function or activity. These sequence alignments are then examined to determine the observed sequence variations. These sequence variations are tabulated to define a primary library, or used to bias the convergence of a protein design algorithm.

As is known in the art, sequence alignments can be analyzed using statistical methods to calculate the sequence diversity at any position in the alignment, and the occurrence frequency or probability of each amino acid at a position. In the simplest embodiment, these occurrence frequencies are calculated by counting the number of times an amino acid is observed at an alignment position, then dividing by the total number of sequences in the alignment In other embodiments, the contribution of each sequence, position or amino acid to the counting procedure is weighted by a variety of possible mechanisms. For example, sequences may be weighted towards or away from a wild type sequence, towards a human sequence, etc.

Furthermore, the sequence alignments may be analyzed to produce the probability of observing two residues simultaneously at two positions. These probabilities may serve as a measure of the strength of coupling between residues. In one embodiment, the probabilities may then be used to favor selection of sequences that maintain conserved residue pairs and disfavor selection of sequences that contain pairs that are seldom or never observed in sequence homologs.

As is known in the art, there are a number of sequence-based alignment programs; including for example, Smith-Waterman searches, Needleman-Wunsch, Double Affine Smith-Waterman, frame search, Gribskov/GCG profile search, Gribskov/GCG profile scan, profile frame search, Bucher generalized profiles, Hidden Markov models, Hframe, Double Frame, Blast, Psi-Blast, Clustal, GeneWise, and FASTA.

The source of the sequences may vary widely, and include taking sequences from one or more of the known databases, including, but not limited to, SCOP (Hubbard, et al., Nucleic Acids Res 27(1): 254-256. (1999)); PFAM (Bateman, et al., Nucleic Acids Res 27(1): 260-262. (1999) http://www.sanger.ac.uk/Pfam/); TIGRFAM (http://www.tigr.org/TIGRFAMs); VAST (Gibrat, et al., Curr Opin Struct Bioi 6(3): 377-385. (1996)); CATH (Orengo, et al., Structure 5 (8): 1093-1108. (1997)); PhD Predictor (http://www.embl-heidelberg.de/predictprotein/predictprotein.html); Prosite (Hofmann, et al., Nucleic Acids Res 27(1): 215-219. (1999) http://www.expasy.ch/prosite/); SwissProt (http://www.expasy.ch/sprot/); PIR (http://www.mips.biochem.mpg.de/proj/protseqdb/); GenBank (http://www.ncbi.nlm.nih.gov/Genbank/); Entrez (http://www.ncbi.nlm.nih.gov/entrez/); RefSeq (http://www.ncbi.nlm.nih.gov/LocusLink/refseq.html); EMBL Nucleotide Sequence Database (http://www.ebi.ac.uk/embl/); DDBJ (http://www.ddbj.nig.ac.jp/); PDB (www.rcsb.org) and BIND (Bader, et al., Nucleic Acids Res 29(1): 242-245(2001) http://www.bind.ca/). In addition, sequences may be obtained from genome and SNP databases of organisms including, but not limited to, human, mouse, worm, fly, plants, fungi, bacteria, and viruses. These may include public databases, for example The Genome Database of The Human Genome Project (http://gdbwww.gdb.org/), or private databases, for example those of Celera Genomics Corporation (http://www.celera.com/) or incyte Genomics (http://www.incyte.com/).

In a preferred embodiment, the contribution of each aligned sequence to the frequency statistics is weighted according to its diversity weighting relative to other sequences in the alignment A common strategy for accomplishing this is the sequence weighting system recommended by Henikoff and Henikoff (see Henikoff S, Henikoff JG. Amino acid substitution matrices, Adv Protein Chem. 2000; 54:73-97. Review. PMID: 10829225 and Henikoff S, Henikoff JG. Position-based sequence weights. J Mol Biol. 1994 Nov 4; 243(4): 574-8.PMID: 7966282), each are herein expressly incorporated by reference.

In a preferred embodiment, only sequences within a preset level of homology to the template sequence are included in the alignment (> 60% identity, > 70% identity, etc.)

In a preferred embodiment, the contribution of each sequence to the statistics is dependent on its extent of similarity to the target sequence, such that sequences with higher similarity to the target sequence are weighted more highly. Examples of similarity measures include, but are not limited to, sequence identity, BLOSUM similarity score, PAM matrix similarity score, and Blast score.

In a preferred embodiment, the contribution of each sequence to the statistics is dependent on its known physical or functional properties. These properties include, but are not limited to, thermal and chemical stability, contribution to activity, solubility, etc. For example, when optimizing the target sequence for solubility, those sequences in an alignment with high solubility levels will contribute more heavily to the calculated frequencies.

In a preferred embodiment, each of the weighted or unweighted alignment frequencies is converted directly to a pseudo-energy as -log ($f_a$). Thus, amino acids with higher frequency are assigned lower (more favorable) pseudo energies. If a frequency is zero, a constant positive pseudo energy may be applied.

In a preferred embodiment, each of the final alignment frequencies ($f_a$) is divided by the observed frequency ($f_o$) of occurrence of each amino acid in all proteins. The log of this ratio, known to those in the art as the log-odds ratio, $\log(f_a/f_o)$, reflects the extent of natural selection for/against each amino acid at each position in the protein. Positive numbers reflect positive selection while negative numbers reflect negative selection. These log-odds ratios may then be used as pseudo energy terms within a PDA™ technology simulation. In situations where lower energies are favorable, the negative log-odds, $-\log(f_a/f_o)$, is a more appropriate pseudo energy term. If a frequency is zero, a constant positive energy may be applied.

In a preferred embodiment, no pseudo energies are created. Rather, the position-specific alignment information is used directly to generate the list of possible amino acids at a variable residue position in a PDA™ technology simulation. Lehmann M, Wyss M. Engineering proteins for thermostability: the use of sequence alignments versus rational design and directed evolution.Curr Opin Biotechnol. 2001 Aug; 12(4): 371-5. Review; Lehmann M, Pasamontes L, Lassen SF, Wyss M. The consensus concept for thermostability engineering of proteins. Biochim Biophys Acta. 2000 De c 29; 1543(2): 408-415. Review; Rath A, Davidson AR. The design of a hyperstable mutant of the Abp1p SH3 domain by sequence alignment analysis. Protein Sci. 2000 Dec;9(12):2457-69; Lehmann M, Kostrewa D, Wyss M, Brugger R, D'Arcy A, Pasamontes L, van Loon AP. From DNA sequence to improved functionality: using protein sequence comparisons to rapidly design a thermostable consensus phytase. Protein Eng. 2000 Jan;13(1):49-57; Desjarlais JR, Berg JM. Use of a zinc-finger consensus sequence framework and specificity rules todesign specific DNA binding proteins. Proc Natl Acad Sci U S A. 1993 Mar 15;90(6):2256-60; Desjarlais JR, Berg JM. Redesigning the DNA-binding specificity of a zinc finger protein: a database-guided approach. Proteins. 1992 Feb;12(2):101-4; Henikoff S, Henikoff JG. Amino acid substitution matrices. Adv Protein Chem. 2000; 54:73-97. Review. PMID: 10829225; Henikoff S, Henikoff JG. Position-based sequence weights. J Mol Biol. 1994 Nov 4; 243(4):574-8.PMID: 7966282.

Similarly, structural alignment of structurally related proteins may be done to generate sequence alignments. There are a wide variety of such structural alignment programs known. See for example VAST from the NCBI (http://www.ncbi.nlm.nih.gov: 80/Structure/VAST/vast.shtml); SSAP (Orengo and Taylor, Methods Enzymol 266(617-635 (1996)) SARF2 (Alexandrov, Protein Eng 9(9): 727-732. (1996)) CE (Shindyalov and Bourne, Protein Eng 11(9): 739-747. (1998)); (Orengo et al., Structure 5(8): 1093-108 (1997); Dali (Holm et al., Nucleic Acid Res. 26(1 316-9 (1998), all of which are incorporated by reference). These structurally-generated sequence alignments may then be examined to determine the observed sequence variations.

In a preferred embodiment, residue pair potentials may be used to score sequences (Mlyazawa et ai., Macromolecules 18(3):534-552 (1985) Jones, Protein Science 3: 567-574, (1994); PROSA (Heindlich et al., J. Mol. Biol. 216:167-180 (1990); THREADER (Jones et al., Nature 358:86-89 (1992), expressly incorporated by reference) during computational screening.

In a preferred embodiment, sequence profile scores (see Bowie et al., Science 253(5016): 164-70 (1991), incorporated by reference) and/or potentials of mean force (see Hendlich et al., J. Mol. Biol. 216(1): 167-180 (1990), also incorporated by reference) are calculated to score sequences. Weighting using these methods determines the structural homology between the sequence and the three-dimensional structure of a reference sequence. These methods assess the match between a sequence and a three-dimensional protein structure and hence may act to screen sequences for fidelity to the protein structure. In particular, U.S. Patent Nos. 6,269,312, 6,188,965, and 6,403,312, and herein expressly incorporated by reference, describe a method termed "Protein Design Automation", or PDA™ technology, that utilizes a number of scoring functions to evaluate sequence stability.

Primary libraries may be generated by predicting tertiary structure from sequence, and then selecting sequences that are compatible with the predicted tertiary structure. There are a number of tertiary structure prediction methods, including, but not limited to, threading (Bryant and Altschul, Curr Opin Struct Biol 5(2): 236-244. (1995)), Profile 3D (Bowie, et al., Methods Enzymol 266(598-616 (1996); MONSSTER (Skolnick, et al., J Mol Biol 265(2): 217-241. (1997); Rosetta (Simons, et al., Proteins 37(S3): 171-176 (1999); PSI-BLAST (Altschul and Koonin, Trends Biochem Sci 23(11): 444-447. (1998)); Impala (Schaffer, et al., Bioinformatics 15(12): 1000-1011. (1999)); HMMER (McClure, et al., Proc Int Conf Intell Syst Mol Biol 4(155-164 (1996)); Clustal W (http://www.ebi.ac.uk/clustalw/); ), helix-coil transition theory (Munoz and Serrano, Biopolymers 41:495, 1997), neural networks, local structure alignment and others (e.g., see in Selbig et al., Bioinformatics 15:1039, 1999).

In an alternate embodiment, the primary library consists of all sequences whose binary pattern, or arrangement of hydrophobic and polar residues, is predicted to be compatible with formation of the desired protein structure (Kamtekar et al., Science 262(5140): 1680-5 (1993). In an alternate embodiment, two profile methods (Gribskov et al. PNAS 84:4355-4358 (1987) and Fischer and Eisenberg, Protein Sci. 5:947-955 (1996), Rice and Eisenberg J. Mol. Biol. 267:1026-1038(1997)), all of which are expressly incorporated by reference) are used to generate the primary library.

In a further embodiment, a knowledge-based amino acid substitution matrix can be used to guide the convergence of a protein design cycie. Exampies of such matrices include, but are not limited to: BLOSUM matrices (e. g. 62, 90, etc.), PAM matrices (e.g. 250, etc.), and Dayhoff matrices.

Energy Calculation Methods

Force field calculations that may be used to optimize the conformation of a sequence within a computational method, such as molecular dynamics and rotamer placement methods, or to generate *de novo* optimized sequences as outlined herein. These methods can be used in any step of the methods of the invention, including their use to generate a primary or secondary library.

Force fields include, but are not limited to, ab *initio* or quantum mechanical force fields, semi-empirical force fields, and molecular mechanics force fields. Examples of force fields include OPLS-AA (Jorgensen, et al., J. Am. Chem. Soc. (1996), v 118, pp 11225-11236; Jorgensen, W.L.; BOSS, Version 4.1; Yale University: New Haven, CT (1999)); OPLS (Jorgensen, et al., J. Am. Chem. Soc. (1988), v 110, pp 1657ff; Jorgensen, et al., J Am. Chem. Soc. (1990), v 112, pp 4768ff); UNRES (United Residue Forcefield; Liwo, et al., Protein Science (1993), v 2, pp1697-1714; Liwo, et al., Protein Science (1993), v 2, pp1715-1731; Liwo, et al., J. Comp. Chem. (1997), v 18, pp849-873; Liwo, et al., J. Comp. Chem. (1997), v 18, pp874-884; Liwo, et al., J. Comp. Chem. (1998), v 19, pp259-276; Forcefield for Protein Structure Prediction (Liwo, et al., Proc. Natl. Acad. Sci. USA (1999), v 96, pp5482-5485); ECEPP/3 (Liwo et al., J Protein Chem 1994 May; 13(4): 375-80); AMBER 1.1 force field (Weiner, et al., J. Am. Chem. Soc. v106, pp765-784); AMBER 3.0 force field (U.C. Singh et al., Proc. Natl. Acad. Scl. USA. 82:755-759); CHARMM and CHARMM22 (Brooks, et al., J. Comp. Chem. v4, pp 187-217); cvff3.0 (Dauber-Osguthorpe, et al.,(1988) Proteins: Structure, Function and Genetics, v4,pp31-47); cff91 (Maple, et al., J. Comp. Chem. v15, 162-182); also, the DISCOVER (cvff and cff91) and AMBER forcefields are used in the INSIGHT molecular modeling package (Biosym/MSI, San Diego California) and HARMM is used in the QUANTA molecular modeling package (Biosym/MSI, San Diego California). HF, UHF, MCSCF, CI, MPx, MNDO, AM1, and MINDO are techniques known to those skilled in the art and which may be used to perform computational site directed mutagenesis for protein design. (see Szabó et al, Modem Quantum Chemistry: Introduction to Advanced Electronic Structure Theory, Macmillan, New York, (c1982) and Hehre, Ab Initio Molecular Orbital Theory, Wiley, New York (c1986) all of which are expressly incorporated by reference.)

In a preferred embodiment, the scaffold protein is an enzyme and highly accurate electrostatic models may be used for enzyme active site residue scoring to improve enzyme active site libraries (see Warshel, Computer Modeling of Chemical Reactions in Enzymes and Solutions, Wiley & Sons, New York, 1991, hereby expressly incorporated by reference). These accurate models may assess the relative energies of sequences with high precision, but are computationally intensive. Highly accurate electrostatic models may also be used in the design of binding sites.

Furthermore, scoring functions may be used to screen for sequences that would create metal or cofactor binding sites in the protein (Hellinga, Fold Des. 3(1): R1-8 (1998), hereby expressly incorporated by reference). Similarly, scoring functions may be used to screen for sequences that would create disulfide bonds in the protein.

In a preferred embodiment. rotamer librarv selection methods are used to generate the primary library. (Dahiyat and Mayo, Protein Sci 5(5): 895-903 (1996); Dahiyat and Mayo, Science 278(5335): 82-7 (1997); Desjarlais and Handel, Protein Science 4: 2006-2018 (1995); Harbury et al, PNAS USA 92(18): 8408-8412 (1995); Kono et al., Proteins: Structure, Function and Genetics 19: 244-255 (1994); Hellinga and Richards, PNAS USA 91: 5803-5807 (1994).

In a preferred embodiment, a sequence prediction algorithm (SPA) is used to design proteins that are compatible with a known protein backbone structure as is described in Raha, K., et al. (2000) Protein Sci., 9: 1106-1119, U.S.S.N. 09/877,695; USSN to be determined for a continuation-in-part application filed on February 6, 2002, entitled APPARATUS AND METHOD FOR DESIGNING PROTEINS AND PROTEIN LIBRARIES, with John R. Desjarlais as inventor. expressly incorporated herein by reference.

In an alternate embodiment, other inverse folding methods such as those described by Simons et al. (Proteins, 34:535-543, 1999), Levitt and Gerstein (PNAS USA, 95:5913-5920, 1998), Godzik et al., PNAS, V89, PP 12098-102; Godzik and Skolnick (PNAS USA, 89:12098-102, 1992), Godzik et al. (J. Mol. Biol. 227:227-38, 1992) may be used.

In an alternate embodiment, molecular dynamics calculations may be used to computationally screen sequences by individually calculating mutant sequence scores and compiling a rank ordered list

In addition, other computational methods such as those described by Koehl and Levitt (J. Mol. Biol. 293: 1161-1181 (1999); J. Mol. Biol. 293:1183-1193 (1999); expressly incorporated by reference) may be used to create a primary library.

PDA™ Technology Calculations

In an especially preferred embodiment, the primary library is generated and processed as outlined in U.S. Patent Nos. 6,269,312, 6,188,965, and 6,403,312, and are herein expressly incorporated by reference. This processing step entails analyzing interactions of the rotamers with each other and with the protein backbone to generate optimized protein sequences. Simplistically, the processing initially comprises the use of a number of scoring functions to calculate energies of interactions of the rotamers, with the backbone and with other rotamers. Preferred PDA™ technology scoring functions include, but are not limited to, a van der Waals potential scoring function, a hydrogen bond potential scoring function, an atomic solvation scoring function, a secondary structure propensity scoring function and an electrostatic scoring function. As Is further described below, at least one scoring function is used to score each variable or floated position, although the scoring functions may differ depending on the position classification or other considerations.

As will be appreciated by those skilled in the art, a variety of force fields that may be used in the PDA™ technology calculations. These include, but are not limited to, those listed previously. As outlined in U.S. Patent Nos. 6,269,312, 6,188,965, and 6,403,312, which are herein expressly incorporated by reference, any combination of the preferred scoring functions, either alone or in combination, may be used. For example, in an alternate embodiment, rotamer internal energies are included. In additional embodiments, energies or scores that are a function of the conformation and/or identity of three or more amino acids are included.

In further embodiments, additional terms are included to influence the energy of each rotamer state, including but not limited to, reference energies, psuedo energies based on rotamer statistics, and sequence biases derived from multiple sequence alignments. Because sequence alignment information and rational methods have demonstrated utility for protein optimization, the invention is an improvement via its combination of information from both methods. Sequence alignment information alone may sometimes be misleading because of unfavorable couplings between amino acids that occur commonly in a multiple sequence alignment. Rational methods alone, may have limitations, for example, are subject to systematic errors due to improper parameterization of force field components and weights.

In a preferred embodiment, the scoring functions may be altered. Additional scoring functions may be used. Additional scoring functions include, but are not limited to torsional potentials, entropy potentials, additional solvation models including contact models, solvent exclusion models (see Lazaridis and Karplus, Proteins 35(2): 133-52 (1999)), and the like; and models for immunogenicity, (see U.S.S.N.s 09/903,378, 10/039,170, and PCT/US02/00165, herein expressly incorporated by reference) such as functions derived from data on binding of peptides to MHC (Major Histocompatibility Complex), that may be used to identify potentially immunogenic sequences. Such additional scoring functions may be used alone, or as functions for processing the library after it is initially scored.

Altered scoring functions may also be obtained from analysis of experimental data. For example, if the presence of certain residues at certain positions are correlated with the presence of desired protein properties, a scoring function may be generated which favor these certain residues.

In addition, other methods may be used to "train" scoring functions by comparing designed sequences and their properties to natural sequences and their properties. That is, the relative importance, or weight, given to individual scoring functions can be optimized in a variety of ways. Although a variety of useful scoring functions exist that represent van der Waals, electrostatics, solvation, and other terms, an important aspect of a force field is the contribution (or weight) of each scoring function to the total score. In a preferred embodiment, computational sequence screening may be used to identify force field parameters such that properties of natural proteins are mimicked in computationally designed sequences. Wang Y, Zhang, H, Scott, RA. A new computational model for protein folding based on atomic solvation. Protein Sci. 1995 Jul;4(7):1402-11.; Kuhlman B, Baker, D. Native protein sequences are close to optimal for their structures. Proc Natl Acad Sci U S A. 2000 Sep 12; 97(19): 10383-8; Street AG, Datta, D, Gordon, DB, Mayo, SL. Designing protein beta-sheet surfaces by Z-score optimization. Phys Rev Lett 2000 May 22; 84(21):5010-3.; Gordon, DB, Marshall, SA, Mayo, SL. Energy functions for protein design. Curr Opin Struct Biol. 1999 Aug; 9(4): 509-13. Review.

In a preferred embodiment, one or more scoring functions are optimized or "trained" during the computational analysis, and then the analysis re-run using the optimized system. For example, the results of PDA™ technology calculations, described below, performed on decoy structures, may be used to obtain optimal sets of scoring function weights. First, the various components of a force field are factorized within the computer algorithm. A starting set of parameters, or weights, is defined based on best guess or previous knowledge of the parameter space. The current parameter set is used in conjunction with a protein design algorithm to design one or more protein sequences and structures. These generated structures are then treated as decoy structures. The optimal set of parameters is considered to be that which predicts that decoys with properties very different from the reference structure (native structure or prototype structure) are high in energy. In a preferred embodiment of the invention, a set of equations, relating the calculated energies of each decoy and comparison of each of its energy components to the reference, is used to iteratively optimize the parameters.

In a preferred embodiment of the Invention, the parameterization simulation begins with the creation of a number of decoy structures (e.g. 100-200) using random scoring function weights (within a predefined range), and a computational protein design algorithm.

In a preferred embodiment, parameters are modified at each iteration cycle according to the following equa-

tion:

$$mod_{i,d} = \lambda * \frac{(E_{i,d} - E_{i,n})}{|E_{i,n}|} * P_d$$

which details the modification of weight I based on evaluation of decoy d. $E_{i,d}$ and $E_{i,n}$ represent the values of the ith scoring function component for the decoy and reference structures. $P_d$ represents the normalized Boltzmann probability of the decoy structure according to its total energy using the current weights. The equation may be interpreted as follows. If a decoy structure's iih energy component is higher in value than that of the native ($E_{i,d}$ vs. $E_{i,n}$), the weight of the ith parameter is increased to an extent related to the difference in energy components. The extent of increase is further related to the current probability ($P_d$) of the decoy structure - only high probability (low energy) decoy structures contribute. The change in parameter will thus by definition lead to an Increase in the calculated energy of the decoy relative to the reference (higher energy or score = less favorable). The value of $\lambda$ determines the rate at which the parameters are varied. The equation Is applied to each decoy in the set. Because the probabilities of the decoys are dynamically related to the change in parameters, multiple iterations over the current decoy set (see below) are applied:

$$mod_{i,d} = \lambda * \frac{(E_{i,d} - E_{i,n})}{|E_{i,n}|} * P_d * e^{-\alpha Q_d}$$

In a preferred embodiment, once the parameterization cycle begins, new decoys are generated with the modified parameters. This ensures broad enough coverage of parameter space by creating a broad range of decoy structures, and leads to the creation of ever-Increasing competition between decoys and reference. However, because the actively created decoys will, at some point in the cycle, become high quality structures, an additional term may be added to the parameter modification scheme:

In a preferred embodiment, the parameterization is performed independently on a number of protein target structures. Parameterization using a number of small protein structures has revealed, importantly, that optimal parameters derived from one protein correlate strongly with those derived from different proteins. This result indicates that the invention yields parameter sets that are applicable to a wide variety of proteins. In an additional aspect, the parameter optimization method is applied separately to sets of proteins that exhibit a common desired property (e.g. high solubility, thermostability). In this manner, force field parameters may be specifically trained to design proteins with desired properties, such as thermostability, solubility, and the like.

A key discovery associated with the method is that natural proteins appear to have highly conserved relative amounts of various energy components (polar group contacts, hydrogen bonding energy, etc.). Thus, although the invention is conveniently applied using the native structure as a reference state, analysis of multiple natural proteins has indicated that a prototypical protein may readily be defined and utilized as a reference state.

Finally, a diversity of related scoring function weights may be applied in separate applications of a protein design cycle, such that a diversity of sequence solutions are derived.

In a preferred embodiment, the scoring functions outlined above may be biased or weighted in a variety of ways that does not involve "training". For example, a bias towards or away from a reference sequence or family of sequences may be incorporated; for example, a bias towards wild-type or homologue residues may be used. Similarly, the entire protein or a fragment thereof may be biased; for example, the active site may be biased towards wild-type residues. A bias towards or against increased energy may be generated. Furthermore, biases may be used to design in selectivity. For example, a bias against sequences that bind to one or more unwanted substrates or receptors may be used. Additional scoring function biases include, but are not limited to applying electrostatic potential gradients or hydrophobicity gradients, and biasing towards a desired charge, Isoelectric point, or hydrophobicity. In addition, experimental data, which may include values for any protein property or properties, may be used to generate biases or weights.

Once the scoring functions to be used are identified for each variable position, the preferred first step in the computational analysis is the determination of the interaction of each possible rotamer with all or part of the remainder of the protein. That is, the energy of interaction, as measured by one or more of the scoring functions, of each possible rotamer at each variable position (or each variable and floated position) with the backbone and/or other rotamers, is calculated. In a preferred embodiment, the interaction energy of each rotamer with the entire remainder of the protein, i.e. both the entire template and all other rotamers, is calculated. However, as outlined above, it is also possible to model only a portion of a protein, for example, a domain, motif, or site in a larger protein.

In a preferred embodiment, two sets of interaction energies are calculated for each side chain rotamer at every position: the interaction energy between the rotamer and the template or backbone (the "singles" energy), and the interaction energy between the rotamer and all other possible rotamers at every other position (the "doubles" energy), whether that position is varied or floated. It should be understood that the template in this case includes both the atoms of the protein structure backbone, as well as the atoms of any fixed residues, as well as non-protein atoms in the scaffold. In an alternate embodiment, singles and doubles energies are calculated for fixed positions as well as for variable and floated positions.

Some energy terms, such as the secondary structure propensity scoring function, may be a component of the singles energy only. As will be appreciated by those in the art, many of the doubles energy terms will be close to zero, as many of the energy terms depend on the physical distance between the first rotamer and the second rotamer. That is, the farther apart the two moieties, the lower the energy typically will be. Furthermore, energy terms are not typically calculated for atoms that are separated by less than three, or alternatively less than four, covalent bonds.

Once the singles and doubles energies are calculated and stored, the next step of the computational processing may occur: the identification of one or more sequences that have a low energy or favorable score. Alternatively, energies may be calculated as needed during the optimization steps, although this is often less computationally efficient.

## COMBINATORIAL OPTIMIZATION ALGORITHMS

The discrete nature of rotamer sets allows a simple calculation of the number of possible rotameric sequences for a given design problem. A backbone of length n with m possible rotamers per position will have $m^n$ possible rotamer sequences, a number which grows exponentially with sequence length. For very simple design calculations, it is possible to examine each possible sequence in order to identify the optimal sequence and/or one or more favorable sequences. However, for a typical design problem, the number of possible sequences (up to $10^{80}$ or more) is sufficiently large that examination of each possible sequence is intractable. A variety of combinatorial optimization algorithms may then be used to identify the optimum sequence and/or one or more favorable sequences.

Combinatorial optimization algorithms may be divided into two classes: (1) those that are guaranteed to return the global minimum energy configuration if they converge, and (2) those that are not guaranteed to return the global minimum energy configuration, but which will always return a solution. Examples of the first class of algorithms include, but are not limited to, Dead-End Elimination (DEE) and Branch & Bound (B&B) (including Branch and Terminate) (see Gordon and Mayo, Structure Fold. Des. 7:1089-98, 1999), and examples of the second class of algorithms include, but are not limited to, Monte Carlo (MC), self-consistent mean field (SCMF), Boltzmann sampling, simulated annealing, genetic algorithm (GA) and Fast and Accurate Side-Chain Topology and Energy Refinement (FASTER).

Combinatorial optimization algorithms may be used alone or in conjunction with each other. Strategies for applying combinatorial optimization algorithms to protein design problems include, but are not limited to, (1) Find the global minimum energy configuration, (2) Find one or more low-energy or favorable sequences, and, most preferred, (3) Find the global minimum energy configuration and then find one or more low-energy or favorable sequences. For example, as outlined in U.S.S.N. 09/127,926 and PCT US98/07254, preferred embodiments utilize a Dead End Elimination (DEE) step, and preferably a Monte Carlo step.

In a preferred embodiment when scoring is used, the primary library comprises the optimum sequence. That is, computational processing is run until the simulation program converges on a single sequence which is the global optimum. In a preferred embodiment, the primary library comprises at least two optimized protein sequences. Thus for example, the computational processing step may eliminate a number of disfavored sequences but be stopped prior to convergence, providing a library of sequences of which the global optimum is one. In addition, further computational analysis, for example using a different method, may be run on the library, to further eliminate sequences or rank them differently. Alternatively, as is more fully described in U.S. Patent Nos. 6,263,312, 6,188,965, and 6,403,312, which are herein expressly incorporated by reference, the global optimum may be reached, and then further computational processing may occur, which generates additional optimized sequences.

It should be noted that the preferred methods of the invention result in a rank-ordered list or filtered set of sequences; that is, the sequences are ranked or filtered on the basis of some objective criteria. However, as outlined herein, it is possible to create a set of non-ordered sequences, for example by generating a probability table directly that lists sequences without ranking them.

In a preferred embodiment, an algorithm that is guaranteed to return the global minimum free energy configuration (GMEC) is used. However, such algorithms are not guaranteed to converge to a solution in a tractable amount of time. That is, the algorithm may get stuck. As a result, alternate strategies may be required for some design problems.

The DEE calculation is based on the assumption that if the worst total interaction of a first rotamer is still better than the best total interaction of a second rotamer, then the second rotamer cannot be part of the global minimum energy configuration. An additional aspect of DEE states that if the energy of a rotamer sequence can always be lowered by changing from a first rotamer to a second rotamer, the first rotamer cannot be part of the global minimum. Since the energies of all rotamers have already been calculated, the DEE approach only requires sums over the sequence length to test and eliminate rotamers, which speeds up the calculations considerably. DEE may also include steps in which pairs of rotamers, or combinations of rotamers, are compared in order to identify sets of rotamers that are not compatible with the global minimum free energy configuration. In order to use DEE, the energy or scoring function must be pairwise-decomposable. That is, the energies or scores must be a function of the conformation and/or identity of at most two rotamers.

In the B&B or A* algorithm, a tree is built, where a rotamer is first picked for one position, then a second position, and so on until one complete rotameric sequence is generated. The energy for that rotameric sequence is then calculated or obtained from the results of an earlier energy calculation. The process is then repeated, adding additional branches to the tree. However, in subsequent steps, if at any point the energy of the partially constructed rotameric sequence is worse than the energy of a previously identified complete rotameric sequence, all sequences that contain that partial rotameric sequence may be eliminated. The process may be completed until the GMEC is identified. Additionally, B&B may be used to generate a list of all sequences that are within some energy or score of the GMEC (Gordon and Mayo, Structure Fold. Des. 7:1089-98, 1999) (Leach and Lemon, Proteins 33(2): 227-239, 1998). As for all the techniques listed herein, these algorithms can be used to generate a primary library or a secondary computational library.

In an alternate embodiment, combinatorial search algorithms that are not guaranteed to return the GMEC may be used, either alone or following identification of the GMEC. These algorithms may also be referred to as sampling techniques. Algorithms that do not return the GMEC are typically computationally efficient and converge to a solution or solutions in a tractable, predictable amount of time. However, the quality of the solutions returned using these algorithms is variable, and may sometimes be insufficient. These sampling methods may include the use of amino acid substitutions, insertions or deletions, or recombinations of one or more sequences.

Sampling techniques use a variety of approaches to jump between different points in sequence space (that is, between different possible variant sequences). For all sampling techniques, the kinds of allowable jumps may be altered (for example, jumps to random residues, jumps biased away from the wild type sequence, jumps biased towards similar residues, jumps where multiple residue positions are simultaneously changed, etc). After jumping to a new sequence, the algorithm will choose whether to accept or reject the jump. The acceptance criteria for each sampling jump may be altered, by modifying the temperature factor. As will be appreciated by those skilled in the art, high temperature factors allow searches across a broad area of sequence space, and low temperature factors allow searches over a narrow region of sequence space. See Metropolis et al., J. Chem Phys v21, pp 1087, 1953, hereby expressly incorporated by reference.

A preferred embodiment utilizes a Monte Carlo search, which is a series of biased, systematic, or random jumps in sequence space. Monte Carlo searching may be used to explore sequence space around the global minimum, to find new local minima distant in sequence space, or to find one or more low energy sequences. A Monte Carlo search may be performed to generate a rank-ordered list or filtered set of sequences in the neighborhood of the GMEC. Starting at the GMEC, random positions are changed to other residues or rotamers (that is, the conformation and/or identity is changed), and the energy of the new sequence is calculated. If the new sequence meets the criteria for acceptance, it is used as a starting point for another jump. After a predetermined number of jumps, a rank-ordered list or filtered set of sequences is generated. Monte Carlo searches may also be started at sequences that are not the GMEC, including randomly selected sequences. Such searches may be used to generate a list of favorable sequences when the GMEC is not known.

In another embodiment, self-consistent mean field ("SCMF") methods (see Delarue et al. Pac. Symp. Biocomput. 109-21 (1997), Koehl et al., J. Mol. Bioi. 239:249 (1994); Koehl et al., Nat. Struc. Biol. 2:163 (1995); Koehl et al., Curr. Opin. Struct. Biol. 6:222 (1996); Koehl et al., J. Mol. Bio. 293:1183 (1999); Koehl et al., J. Mol. Biol. 293:1161 (1999); Lee J. Mol. Biol. 236:918 (1994); and Vasquez Biopolymers 36:53-70 (1995); all of which are expressly incorporated by reference.) are used. SCMF works by determining the optimal set of probabilities for all rotamer and residue states in the simulation, using a self-consistency criterion that relates the mean-field energies of the states to their probabilities, and vice versa. The final probabilities may be used to define a list of a favorable sequence combinations that define a combinatorial library of protein sequences. As for all the techniques listed herein, SCMF can be used to generate a primary library or a secondary computational library.

In a preferred embodiment, the sampling technique utilizes genetic algorithms, e.g., such as those described by Holland (Adaptation in Natural and Artificial Systems, 1975, Ann Arbor, U. Michigan Press). Genetic algorithm analysis generally takes generated sequences and recombines them computational, similar to a nucleic acid recombination event, in a manner similar to gene shuffling. Thus the "jumps" of genetic algorithm analysis generally are multiple position jumps. In addition, as outlined below, correlated multiple jumps may also be done. Such jumps may occur with different crossover positions and more than one recombination at a time, and may involve recombination of two or more sequences. Furthermore, deletions or insertions (random or biased) may be done. In addition, as outlined below, genetic algorithm analysis may also be used after the secondary library has been generated.

In a preferred embodiment, Boltzmann sampling is done. As will be appreciated by those in the art, the temperature factor criteria for Boltzmann sampling may be altered to allow broad searches at high temperature factors and narrow searches close to local optima at low temperature factors (see e.g., Metropolis et al., J. Chem. Phys. 21: 1087, 1953).

In a preferred embodiment, the sampling technique utilizes simulated annealing, e.g., such as described by Kirkpatrick et al. (Science, 220:671-680, 1983). Simulated annealing alters the cutoff for accepting good or bad jumps by altering the temperature factor in a systematic manner. That is, slowly decreasing the temperature factor will slowly increase the stringency of the cutoff. This allows broad searches at high temperature factors to new areas of sequence space and narrow searches at low temperature factors to explore regions in detail.

In a preferred embodiment, the FASTER method is used for determination of global optimization of the side chain conformations of proteins. The FASTER method focuses on resolving the combinatorial side chain packing problem, by converging on the near-optimal minima. (see Desmet, et al., Proteins, 48:31-43, 2002).

## TABOO

In a preferred embodiment, a diverse set of low-energy sequences is obtained using a class of algorithms referred to as tabu search algorithms. Traditionally, tabu search algorithms have been used to search for alternative local minima. The present invention presents a novel use of tabu search algorithms by using these algorithms to map amino acid sequence subspaces (see Modem Heuristic Search Methods, edited by V.J. Rayward-Smith, et al., 1996, John Wiley & Sons Ltd., hereby expressly incorporated by reference in its entirety). When used to map sequence space, the tabu search algorithms are referred to herein as "Taboo" search algorithms. A Taboo search assumes that alternative optimization methods, such as protein design algorithms incorporating Dead End Elimination, genetic algorithms, Monte Carlo searches, have been used to provide the location of the global minimum or a local minimum. Thus, a Taboo search is used for finding other regions or subspaces of the search space that contain local minima; preferably those that are reasonably low in energy compared to the global minimum.

Taboo searches are capable of identifying alternative low energy basins because the search incorporates local optima avoidance by recording previously seen solutions by making a list of moves which have been made in the recent past of the search and which are tabu or forbidden for a certain number of iterations. That is, if a move in the search space has been made recently, that move is discouraged for some duration of time during the sampling procedure. The moves may be forbidden for some period of time or search (which can be varied), or weighted against but not forbidden. Such a mechanism helps to avoid cycling and serves to promote the identification of alternative low energy basins. This concept is illustrated in Figure 2. For example, by making the low energy basin identified by PDA™ technology taboo, the search is forced to discover a different low energy basin. This cycle may be repeated until most or all of the alternative low energy basins are identified. These alternative low energy basins or subspaces represent regions of the sequence space of a protein that should be explored experimentally by creation of secondary libraries (see Figure 3).

Once a starting sequence is chosen, a taboo search is done. Preferably, the taboo search is done by applying one or more pseudo energies (pE) and serves to temporarily change the perceived energy landscape of the sequence space (see Figure 4). For example, if a single protein design simulation converges at iteration k to a variable protein sequence and structure that contains amino acid aa in rotamer state **r** at position **i,** then the matrix of side chain-template energies will be modified at iteration k+1 as follows:

$$pE^{k+1}_{aa,r,i} = pE^{k}_{aa,r,i} + \delta E_{taboo}$$

where the pseudo energy at the very first iteration is equivalent to the calculated energy:

$$pE^{0}_{aa,r,i} = E_{aa,r,i}$$

and $\delta E_{taboo}$ is defined by the simulation parameters. In some embodiments, the $\delta E_{taboo}$ magnitude is dynamic (e.g., random and/or slowly decreasing), again as defined by simulation parameters. $E_{aa,r,i}$ represents the energy calculated by the force field or scoring function (i.e., $E_{calc}$ in the Figures).

Application of the pseudo energy increase discourages repeated convergence to solutions containing amino acid aa in rotamer state **r** at position **i** in subsequent design simulations. However, if the current value of pE is insufficient to discourage incorporation of the rotamer, convergence to the same rotamer state in a subsequent simulation will result in additional increase of the pseudo energy.

In a preferred embodiment, calculated and pseudo energies are stored in separate memory locations so that the calculated energy of any solution may be reported directly. This aspect is importa nt for separating the effects of the taboo search from an accurate assessment of protein sequence energies. In a preferred embodiment, the pseudo energy increase is applied to only one rotamer state of a converged **aa** at position **i**. In a preferred embodiment, the pseudo energy increase is applied to all rotamer states of a converged **aa** at position **i.** In a preferred embodiment, the pseudo energy increase is applied to a plurality of amino acid positions, and or a plurality of rotamer states. Thus, a taboo search results in the identification of alternate amino acids/rotamer states for at least one and preferably more than one amino acid position. Alternate amino acids/rotamer states may be reused in a protein design cycle to generate alternate variable protein sequences.

In a preferred embodiment, the taboo search is done by applying a probability parameter to at least one amino acid position. Such biased sampling is generally applied within so-called heuristic or stochastic sampling methods such as Monte Carlo or genetic algorithms. For example, many heuristic methods use the concept of Boltzmann sampling, which is based on the energy difference between two states. In a preferred embodiment, a probability parameter results in a modification of the Boltzmann probability ($P_B$) such that the sampling probability ($P_S$) is reduced:

$$P_B = e^{-(\Delta E)}/RT$$

$$P_S = e^{-(\Delta E + \delta E_{taboo})/RT}$$

In a preferred embodiment, any or all of the methods described herein may utilize a recency parameter. In other words, application of a recency parameter ensures that the most recent moves in sequence space are prohibited for a certain number of iterations. Moves that are considered to be prohibited are derived from a running list, which is an ordered list of all moves performed throughout the search. If the length of the running list is limited, recency may be viewed as the equivalent of short term memory. As will be appreciated by those of skill in the art, one consequence of limiting the length of the running list is that the prohibited moves may be encouraged at a later point in the simulation to allow for the exploration of a sequence space that has not been visited for some defined duration. Thus, recency may be a fixed parameter or allowed to vary dynamically during the search.

In a preferred embodiment, recency is applied to the modified energy matrix by continual application of a damping term to all pseudo energies as follows:

$$pE^K_{aa,r,i} = \lambda * pE^K_{aa,r,i}$$

This continual damping has the simple effect of an exponential decay of the pseudo energy over multiple simulation cycles.

In a preferred embodiment, the damping is applied at every simulation cycle before or after application of additional pseudo energy increases. As will be appreciated by those of skill in the art, this approach mathematically enforces a ceiling or upper limit on the magnitude of the pseudo energy, defined by the combination of $\lambda$ and $\delta E$.

In a preferred embodiment, the application of the damping and pseudo energy terms are reversed. In an alternative embodiment, recency is applied to the modified energy matrix by continual application of a damping term to all pseudo energies as follows:

$$pE^K_{aa,r,i} = \lambda - \gamma$$

In a preferred embodiment, the frequency parameter is applied such that the strength of the taboo energy increase is dependent on the number of times a given amino acid has occurred at a particular position. For example, the pseudo energy equation may be modified to include a frequency bias as follows:

$$pE^{k+1}_{aa,r,i} = pE^{k+1}_{aa,r,i} + f_{aa,r,i} * \delta E_{taboo}$$

In other words, applied in this manner the strength of the taboo energy increase depends on the frequency of occurrence ($f_{aa,r,i}$) of that amino acid or rotamer in previous solutions. In a preferred embodiment, the frequency parameter is biased against the most frequent amino acid residue at a particular position. Any or all of these methods involving recency and frequency parameters may be used reiteratively or combined in any order.

Thus, taboo analysis can be done to generate sequences that are not the GMEC but are local minima (low energy) as well. As for all the computational methods outlined herein, this may be done at any point during the analysis. Thus, for example, a taboo analysis may be done to identify one or more starting scaffolds, e.g. even before a primary library is generated. Alternatively, taboo analysis can be used as the computational analysis for primary and/or library. Alternatively, taboo analysis can be applied in combination with other computational techniques as either part of the primary or secondary library generation. For example, taboo constraints may be added to a Monte Carlo search.

## SELECTING SEQUENCES FOR THE PRIMARY LIBRARY

In general, some subset of all possible sequences is used as the primary library. However, in some instances it may be desirable to include all sequences when a defined number of variable positions are used. It is usually preferable for the primary library to be small enough that a reasonable fraction of the sequence space of a particular sequence may be sampled, allowing for robust generation of secondary libraries. Thus, primary libraries that range from about 50 to $10^{13}$ are preferred, with from 1000 to $10^7$ being particularly preferred, and from 1000 to 100,000 being especially preferred. Thus, in one preferred embodiment, the primary library excludes from 1% to about 90-95% of possible sequence space sequences, with exclusion of at least 1%, 2%, 5%, 10%, 20%, 40%, 50% and 70% being preferred. Alternatively, the library may include 1 in $10^3$, 1 in $10^7$, 1 in $10^{10}$, 1 in $10^{25}$, 1 in $10^{50}$, 1 in $10^{79}$ and 1 in $10^{80}$.

A variety of approaches may be used to select a set of sequences for the primary library, including structure-based methods such as PDA™ technology sequence-based methods, or combinations as outlined herein. In addition, as noted herein, any method used to generate a primary or secondary library may be used as the other step.

It should also be noted that while these methods are described In conjunction with limiting the siz e of the primary library, these same techniques may be used to formulate a cutoff for inclusion in the secondary and tertiary libraries as well.

The set of protein sequences in the primary and secondary libraries are generally, but not always, significantly different from the wild-type sequence from which the backbone was taken, although in some cases the primary or secondary library may contain the wild-type sequence. That is, the range of optimized protein sequences is dependent upon many factors including the size of the protein, properties desired, etc. However, for example, comprises between 0.001% and 100% variant amino acids, with about at least 90%, 70%, 50%, 30%, 10% variant amino acids being preferred.

In a preferred embodiment, the primary library sequences are obtained from a rank-ordered list or filtered set generated using an algorithm such as Monte Carlo, B&B, or SCMF. For example, the top $10^3$ or the top $10^5$ sequences in the rank-ordered list or filtered set may comprise the primary library. Alternatively, all sequences scoring within a certain range of the optimum sequence may be used. For example, all sequences with in 10 kcal/mol of the optimum sequence could be used as the primary library. In addition, as outlined below, any out of a rank-ordered list or a filtered set may be used depending on the conditions, use and additional methodologies of the resulting set; for example, the top X number of sequences may be used, or the top X and the bottom Y number of sequences, for example when a wider range of sequence space is to be explored or when clustering is used. This method has the advantage of using a direct measure of fidelity to a three-dimensional structure to determine inclusion.

Alternatively, the total number of sequences defined by the recombination of all mutations may be used as a cutoff criterion for the primary sequence library. Preferred values for the total number of recombined sequences range from 100 to $10^{20}$, particularly preferred values range from 1000 to $10^{13}$, especially preferred values range from 1000 to $10^7$. Alternatively, a cutoff may be enforced when a predetermined number of mutations per position is reached. As a rank-ordered (or unordered) or filtered set sequence list is lengthened and the library is enlarged, the number of mutations per position will typically increase. Alternatively, the first occurrence in the list of predefined undesirable residues may be used as a cutoff criterion. For example, the first hydrophilic residue occurring in a core position could limit the set of sequences included In the primary library. Alternatively, when multiple related structures are used for the scaffold, the set of optimal sequences for each structure may be used to make the primary library.

In addition, in some embodiments, sequences that do not make the cutoff are included in the primary library. This may be desirable in some situations, for instance to evaluate the primary library generation method, to serve as controls or comparisons, or to sample additional sequence space. For example, in a preferred embodiment, the wild-type sequence is included, even if it did not make the cutoff.

As is further outlined below, it should also be noted that different primary libraries may be combined. For example, positions in a protein that show a great deal of mutational diversity in computational screening may be fixed as outlined below and a different primary library regenerated. A rank-ordered list or filtered set of the same length as the first would now show diversity at positions that were largely conserved in the first library. The variants from a first primary library may be combined with the variants from a second primary library to provide a combined library at lower computational cost than creating a very long rank-ordered list or filtered set. This approach may be particularly useful to sample sequence diversity in both highly mutable and highly conserved positions. In addition, primary libraries may be generated by combining the results of two or more calculations to form one primary library.

## CLUSTERING

Clustering algorithms may be useful for classifying sequences derived by protein design algorithms into representative groups. Clustering can serve a wide variety of purposes. For example, sets of sequences that are close in sequence space can be distinguished from other sets, and thus recombination can be confined within sets. That is, sequences that share a local minima may be recombined, to allow better results, rather than recombine sequences from two local minima that may have quite different sequences. Thus, for example, a primary library can be clustered around local minima ("clustered sets of sequences"), recombination or secondary library generation is within each clustered set, and then each "clustered" secondary library is added to form the secondary library genus.

Clustering algorithms require two key components. First is a metric for comparing the similarity of two entities. Measures of similarity include, but are not limited to sequence identity, sequence similarity, and energetic similarity. Second, clustering algorithms require an algorithm to separate the entities into groups based on relative similarities. Many types of clustering algorithms exist, the most simple and commonly used are single-linkage, complete linkage, and average linkage methods (see Figure 5). These are often applied hierarchically, such that the relationships between entities may be described with a tree structure.

Preferably, clustering algorithms including but not limited to, single linkage clustering algorithms, complete linkage clustering algorithms, and average linkage clustering algorithms are used to analyze the results from computational protein cycles described herein. Clustering algorithms may be used to form subsets using computationally generated energy matrices to measure energetic similarity (see Figure 6). Alternatively, clustering algorithms may be used to form subsets directly from a set of optimized protein sequences.

In a preferred embodiment, a single-linkage clustering algorithm is used to form subsets from computationally generated energy matrices. An example of the use of a single-linkage clustering algorithm to form subsets from a computationally generated energy matrix is shown in Figures 5, 6, and 7.

In alternative embodiments, a single linkage clustering algorithm is used to form subsets directly from a set of optimized protein sequences whereby the measure of similarity between two sequences is the extent of sequence identity. Alternatively, the measure of similarity between two sequences may be based on a standard sequence similarity comparison. As will be appreciated by those skilled in the art, similarity scores include but are not limited to BLOSUM similarity score, Dayhoff similarity score, PAM similarity score, etc. Specific examples of the aforementioned similarity scores include but are not limited to BLOSUM tables, 62 and 90; PAM tables: 250, etc., among others. In a preferred embodiment, subsets of designed protein sequences derived by clustering or related methods may be used to define multiple primary or secondary libraries.

In an alternate embodiment, sets of sequences that may be recombined productively are defined as those that minimize disruption of sets of interacting or correlated residues. Identification of sets of interacting residues may be carried out by a number of ways, e.g. by using known pattern recognition methods, comparing frequencies of occurrence of mutations or by analyzing the calculated energy of interaction among the residues (for example, if the energy of interaction is high, the positions are said to be correlated or interacting). These correlations may be positional correlations (e.g. variable residue positions 1 and 2 always change together or never change together) or sequence correlations (e.g. If there is a residue A at position 1, there is always residue D at position 2). In addition, programs used to search for consensus motifs may be used. See: Lockless and Ranganathan, Science 286:295-299 (1999), Pattern discovery in Blomolecular Data: Tools, Techniques, and Applications, edited by Jason T.L. Wang, Bruce A. Shapiro, Dennis Shasha. New York: Oxford University, 1999; Andrews, Harry C. Introduction to mathematical techniques in patter recognition; New York, Wiley-Interscience (1972); Applications of Pattern Recognition; Editor, K.S. Fu. Boca Raton, Fla. CRC Press, 1982; Genetic Algorithms for Pattern Recognition; edited by Sankar K Pal, Paul P. Wang. Boca Raton: CRC Press, c1996; Pandya, Abhijit S., Pattern recognition with Neural networks in C++/Abhijit S. Pandya, Robert B. Macy. Boca Raton, Fla.: CRC Press, 1996; Handbook of pattern recognition and computer vision / edited by C.H. Chen, L.F. Pau, P.S.P. Wang. 2nd ed. Signapore; River Edge, N.J.: World Scientific, c1999; and Friedman, Introduction to Pattern Recognition: Statistical, Structural, Neural, and Fuzzy Logic Approaches; River Edge, N.J. : Word Scientific, c1999, Series Title: Serien a machine perception and artificial intelligence; vol. 32. All references cited herein are expressly incorporated by reference.

## GENERATION OF SECONDARY LIBRARIES

As described herein, there are a wide variety of methods to generate secondary libraries from primary libraries. The first is a selection step, where some set of primary sequences are chosen to form the secondary library. The second is a computational step, again generally including a selection step, where some subset of the primary library is chosen and then subjected to further computational analysis, including both protein design cycles as well as techniques such as "in silico" shuffling (recombination). The third is an experimental step, where some subset of the primary library is chosen and then recombined experimentally to form a secondary library.

SELECTING SEQUENCES FOR THE SECONDARY LIBRARY

In a preferred embodiment, the primary library of the scaffold protein is used to generate a secondary library. The secondary library may then be generated and tested experimentally or subjected to further computational manipulation. A variety of approaches, including but not limited to those described below, may be used to select sequences for the secondary library. Each approach may be used alone, or any combination of approaches may be used. As will be appreciated by those in the art, the secondary library may be either a subset of the primary library, or contain new library members, i.e. sequences that are not found in the primary library. That is, in general, the variant positions and/ or amino acid residues in the variant positions may be recombined in any number of ways to form a new library that exploits the sequence variations found in the primary library. In such embodiments, the secondary library will contain sequences that were not included in the primary library. In all cases, if the secondary library is generated experimentally, it may optionally comprise one or more "error" sequences, which result from experimental errors, as well as one or more sequences generated intentionally. That is, additional variability can be added to the secondary (or, in fact, to the primary library as well), either experimentally (e.g. through the use of error-prone PCR in secondary library sequences) or computationally (adding an "in silico" variant generation step to sample more sequence space). In the latter case, it is possible to introduce this additional level of variability in a random fashion (as used herein random includes variation introduced in a controlled manner or an uncontrolled manner) or In a directed fashion. For example, directed variability may be introduced by adding certain residues from a particular sequence, e.g. the human sequence.

Selecting a subset of the primary library

As described herein, there are a wide variety of techniques that can be used to generate a secondary library. In a preferred embodiment, a subset of the primary library is used as the secondary library. This subset can be chosen in a variety of ways, as outlined herein. For example, similar to the primary library cut-off, an arbitrary numerical cut-off can be applied: the top X number of sequences forms the basis of the secondary library (or the top X number and the bottom Y number, or any sequences in the top X number plus anything within Z energy of the wild-type sequence, etc. ). As will be appreciated by those in the art, there are a wide variety of relatively simple numerical cutoffs that can be applied.

In a preferred embodiment, all amino acid residues are allowed at each variable residue position identified in the primary library. That is, once the variable residue positions are identified, a secondary library comprising every combination of every amino acid at each variable residue position is made.

In a preferred embodiment, subsets of amino acids are chosen to maximize coverage. Additional amino acids with properties similar to those contained within the primary library may be manually added. For example, if the primary library includes three large hydrophobic residues at a given position, the user may chose to include additional large hydrophobic residues at that position when generating the secondary library. In addition, amino acids in the primary library that do not share similar properties with most of the amino acids at a given position may be excluded from the secondary library. Alternatively, subsets of amino acids may be chosen from the primary library such that a maximal diversity of side chain properties is sampled at each position. For example, if the primary library includes three large hydrophobic residues at a given position, the user may chose to include only one of them in the secondary library, in combination with other amino acids that are not large and hydrophobic.

In a preferred embodiment, the primary library may be analyzed to determine which amino acid positions in the scaffold protein have a high mutational frequency, and which positions have a low mutation frequency. The secondary library may be generated by varying the amino acids at the positions that have high numbers of mutations, while keeping constant the positions that do not have mutations above a certain frequency. For example, if a position has less than 20% and more preferably less than 10% mutations, it may be held invariant

In a preferred embodiment, the secondary library is generated from a probability distribution table. As outlined herein, there are a variety of methods of generating a probability distribution table, including using PDA™ technology output, the results of other energy calculation methods, (e.g. SCMF), and/or the results of knowledge- or sequence-based methods, all described previously. In addition, the probability distribution may be used to generate information entropy scores for each position, as a measure of the mutational frequency observed in the library. In this embodiment, the frequency of each amino acid residue at each variable residue position in the list is identified. Frequencies may be thresholded, wherein any variant frequency lower than a cutoff is set to zero. This cutoff is preferably 1%, 2%, 5%, 10% or 20%, with 10% being particularly preferred. These frequencies may be built into the secondary library, so that the frequency at which each amino acid is present in the primary library is equal, within experimental error, to the frequency at which that amino acid will be present in the secondary library.

Recombination of Some or All Primary Library Sequences to Generate a Secondary Library

In an alternate embodiment, variable residue positions may be recombined to generate novel sequences to form a secondary library. Thus, the secondary library comprises at least one member sequence and preferably a plurality of such member sequences not found in the primary library. Recombination may be performed experimentally and/or computationally using a variety of approaches. For example, a list of naturally occurring sequences may be used to calculate all possible recombinant sequences, with an optional rank ordering or filtering step. Alternatively, once a primary library is generated, one could rank order only those recombinations that occur at cross-over points with at least a threshold of identity over a given window (for example, 100% identity over a contiguous 18 nucleotide sequence, or 80% identity over a contiguous 24 nucleotide sequence). Alternatively, the homology could be considered at the DNA level, by computationally translating the amino acids to their respective DNA codons. Different codon usages could be considered. A preferred embodiment considers only recombinations with crossover points that have DNA sequence identity sufficient for hybridization.

In some embodiments, all possible recombinant sequences are experimentally generated and tested. Alternatively, in a preferred embodiment, the recombinant sequences are scored computationally and a subset of these sequences are experimentally generated and tested. Computational screening of the set of recombinant sequences may be used to reduce the library to an experimentally tractable size and/or to enrich the library in sequences predicted to possess desired properties. The recombinant sequences may be analyzed using methods including, but not restricted to, those methods used to generate and analyze primary library sequences, and by considering the role of clusters of interacting residues, as discussed below.

In a preferred embodiment, the secondary library in generated by using any of the techniques outlined for primary library generation (SPA, PDA™, taboo, clustering, "in silico" recombination, etc.) on the primary library that has been chosen. Particular combinations of computational analyses for primary and secondary libraries are outlined below.

In a preferred embodiment, the secondary library is generated experimentally, using any number of the techniques outlined below, including gene assembly procedures.

It is possible that some recombinant sequences will be inviable, that is, they will fail to fold, aggregate, possess other undesired properties, or lacks desired properties. In certain cases, some algorithms will generate a plurality of local minima, the combination of which may lead to unsatisfactory sequences.

However, computational screening approaches may be used to differentiate and bias or select for viable constructs from inviable constructs. For example, if recombining all library members is predicted to yield an excessive number of unviable sequences, subsets of a library could be recombined instead. Strategies for identifying sets of sequences that may be productively recombined include, but are not limited to, clustering based on sequence identity or similarity, clustering based on similarity of the energy matrix, and identification of sets of interacting residues.

As will be appreciated by those in the art and outlined herein, probability distribution tables can be generated in a variety of ways. In addition to the methods outlined herein, self-consistent mean field (SCMF) methods can be used in the direct generation of probability tables. SCMF is a deterministic computational method that uses a mean field description of rotamer interactions to calculate energies. A probability table generated in this way can be used to create secondary libraries as described herein. SCMF can be used in three ways: the frequencies of amino acids and rotamers for each amino acid are listed at each position; the probabilities are determined directly from SCMF (see Delarue et la. Pac. Symp. Biocomput. 109-21 (1997), expressly incorporated by reference). In addition, highly variable positions and non-variable positions can be identified. Alternatively, another method is used to determine what sequence is jumped to during a search of sequence space; SCMF is used to obtain an accurate energy for that sequence; this energy is then used to rank it and create a rank-ordered list of sequences (similar to a Monte Carlo sequence list). A probability table showing the frequencies of amino acids at each position can then be calculated from this list (Koehl et al., J. Mol. Biol. 239:249 (1994); Koehl et al., Nat Struc. Biol. 2:163 (1995); Koehl et al., Curr. Opin. Struct Biol. 6:222 (1996); Koehl et al., J. Mol. Bio. 293:1183 (1999); Koehl et al., J. Mol. Biol. 293:1161 (1999); Lee J. Mol. Biol. 236:918 (1994); and Vasquez Biopolymers 36:53-70 (1995); all of which are expressly incorporated by reference. Other forcefields that can be used in similar methods are outlined above.

In addition, as outlined herein, a preferred method of generating a probability distribution table is through the use of sequence alignment programs. In addition, the probability table can be obtained by a combination of sequence alignments and computational approaches. For example, one can add amino acids found in the alignment of homologous sequences to the result of the computation. Preferable one can add the wild type amino acid identity to the probability table if it is not found in the computation.

## Generation of Tertiary Libraries

In a preferred embodiment, a variety of additional steps may be done to one or more secondary libraries; for example, further computational processing may occur, secondary libraries may be recombined, or subsets of different secondary libraries may be combined.

In a preferred embodiment, a tertiary library can be generated from combining secondary libraries. For example, a probability distribution table from a secondary library can be generated and recombined, whether computationally or experimentally, as outlined herein. A PDA secondary library may be combined with a sequence alignment secondary library, and either recombined (again, computationally or experimentally) or just the cutoffs from each joined to make a new tertiary library. The top sequences from several libraries can be recombined. Primary and secondary libraries can similarly be combined. Sequences from the top of a library can be combined with sequences from the bottom of the library to more broadly sample sequence space, or only sequences distant from the top of the library can be combined. Primary and/or secondary libraries that analyzed different parts of a protein can be combined to a tertiary library that treats the combined parts of the protein. These combinations can be done to analyze large proteins, especially large multidomain proteins or complete protoesomes.

In a preferred embodiment, a tertiary library can be generated using correlations in the secondary library. That is, a residue at a first variable position may be correlated to a residue at second variable position (or correlated to residues at additional positions as well). For example, two variable positions may sterically or electrostatically interact, such that if the first residue is X, the second residue must be Y. This may be either a positive or negative correlation. This correlation, or "cluster" of residues, may be both detected and used in a variety of ways. (For the generation of correlations, see the earlier cited art).

In addition, primary and secondary libraries can be combined to form new libraries; these can be random combinations or the libraries, combining the "top" sequences, or weighting the combinations (positions or residues from the first library are scored higher than those of the second library).

Additional variability can be added to the tertiary library as well), either experimentally (e.g. through the use of error-prone PCR in tertiary library sequences) or computationally (adding an "in silico" variant generation step to sample more sequence space). In the latter case, it is possible to introduce this additional level of variability in a random fashion (as used herein random includes variation introduced in a controlled manner or an uncontrolled manner) or in a directed fashion. For example, directed variability may be introduced by adding certain residues from a particular sequence, e.g. the human sequence.

In a preferred embodiment, when two computational steps are used (e.g. a PDA™ step to generate a primary library and in silico shuffling or a probability table to generate a secondary library), the experimental generation of the secondary library can result in a tertiary library, that is, a library that contains members not found in the secondary library. Alternatively, the tertiary library may just be a subset of the secondary library as outlined above.

In a preferred embodiment, a secondary library may be computationally remanipulated to form an additional secondary library (sometimes referred to herein as "tertiary libraries"). For example, any of the secondary library sequences may be chosen for a second round of PDA™ technology calculations, by freezing or fixing some or all of the changed positions in the first secondary library. Alternatively, only changes seen in the last probability distribution table would be allowed. Alternatively, the stringency of the probability table may be altered, either by increasing or decreasing the cutoff for inclusion.

In a preferred embodiment, the sequence information derived from experimental screening of a secondary library could be used to guide the design for the tertiary library. In this way, the library generation is an iterative process. In a preferred embodiment, the tertiary library could be derived by computationally screening the secondary library for desired protein properties as previously mentioned.

## Experimentally Making the Library

Once a library is generated using any of the methods outlined herein or combinations thereof, the library (or a tertiary, quaternary, etc. library) is made any number of techniques, including using gene assembly procedures. Accordingly, the present invention provides methods for making protein libraries in any of a variety of different ways.

## Chemical synthesis of proteins

In a preferred embodiment, different protein members of the secondary library may be chemically synthesized. This is particularly useful when the designed proteins are short, preferably less than 150 amino acids in length, with less than 100 amino acids being preferred, and less than 50 amino acids being particularly preferred, although as is known in the art, longer proteins may be made chemically or enzymatically.

These amino acid sequences could then be joined together via chemical ligation to form larger proteins as needed (see Yan, L. and Dawson, P.E, J. Am. Chem. Soc. 123 (2001) 526-533, and Dawson, P.E. and Kent, S.B.H, Ann. Rev. Biochem. 69, (2000) 923-960), hereby expressly incorporated by reference. Furthermore, peptides corresponding to sequences from different library members could be shuffled or randomly ligated together to form a secondary library. For example, one or more peptides with different amino acid sequences from the N-terminal region of the protein could be ligated to one or more peptides with different amino acid sequences from the C-terminal region of the protein. Such an assembly could be repeated for several further rounds of synthesis. Using such a method, a secondary library could be chemically synthesized.

In a preferred embodiment, proteins could be constructed by chemically synthesis of peptides and formed by ligation of the peptides using intein technology (Evans et al. (1999) J. Biol. Chem. 274, 18359-18363; Evans et al. (1999) J. Biol. Chem. 274, 3923-3926; Mathys et al. (1999) Gene 231, 1-13; Evans et al. (1998) Protein Sci. 7,2256-2264; Southworth et al. Biotechniques 27, 110-120).

## Generating nucleic acids that encode single members of a library

In a preferred embodiment, particularly for longer proteins or proteins for which large samples are desired, the secondary library sequences are used to create nucleic acids such as DNA which encode the member sequences and which may then be cloned into host cells, expressed and assayed, if desired. Thus, nucleic acids, and particularly DNA, may be made which encodes each member protein sequence. This is done using well-known procedures. See Maniatis and current protocols. (see Current Protocols in Molecular Biology, Wiley & Sons, and Molecular Cloning - A Laboratory Manual - 3rd Ed., Cold Spring Harbor Laboratory Press, New York (2001)). The choice of codons, suitable expression vectors and suitable host cells will vary depending on a number of factors, and may be easily optimized as needed.

## Gene Assembly Procedures

As will be appreciated by those in the art, the generation of exact sequences for a library comprising a large number of sequences (despite the fact that the set number is much smaller than the original set) is still potentially expensive and time consuming. Accordingly, In a preferred embodiment, there are a variety of gene assembly techniques that may be used to generate the secondary or higher order libraries of the present invention. As discussed herein, these experimentally generated libraries generally recombine sequences within the library, resulting in sequences present in the original library as well as recombined combinations of those sequences.

## Gene Assembly Using Pooled Oligonucleotides

In a preferred embodiment, multiple amplification reactions with pooled oligonucleotides are done, as is generally depicted in Figure 12, comprising variant protein sequences created by the assembly of gene fragments generated from a nucleic acid template. This generally involves generating variant protein sequences created by the assembly of gene fragments generated from a nucleic acid template. They can be full length "overlapping" oligonucleotides, or primers. In one embodiment, overlapping oligonucleotides are synthesized which correspond to the full-length gene. As may be appreciated by one skilled in the art, these oligonucleotides may represent all of the different amino acids at each variant position or subsets. Once these oligonucleotides are made, they are reassembled into a set of variable sequences in any number of ways, outlined below. While the reactions described below focus on PCR as the amplification techniques, others are included as is generally outlined below.

In general, the invention may take on a wide variety of configurations. For example, libraries of nucleic acids encoding all or a subset of possible proteins are generated by assembling nucleic acid fragments. Preferably, the gene fragments are linked together using an enzymatic or non-enzymatic method for the ligation of gene fragments. For example, for each gene fragment, a pair of donor fragments is generated such that the sense strand from one donor fragment complements the antisense strand of the other donor fragment and creates a 5'-phosphorylated overhang when the two strands are hybridized under conditions that allow for the formation of a double stranded molecules. The 5' phosphorylated overhang is located at one of the 5' ends of the resulting double stranded molecule to allow ligation to a free 3'-terminus of an adjacent gene fragment. In some embodiments, 5'-phosphorylated overhangs are generated at both ends, preferably with unique sequences to prevent self-ligation.

Chemically synthesized oligonucleotides are used as primers for the generation of donor fragments. For each pair of donor fragments, one primer is labeled at the 5'-end with a purification tag. The purification tag may be a his, myc, flag, or HA tag or a fusion protein may be used instead, for example gst, thioredoxin, nusA, among others known in the art. Preferably, the purification tag is biotin. The other primer is designed to bind to the other member of the donor fragment pair to create a 5'-phosphorylated overhang, from about 1 to 20 or more base pairs in length.

In a preferred embodiment, at least one of the populations of nucleic acid fragments comprise variant sequences that result in the formation of a variant nucleic acid sequence. In a further embodiment, both the 5'-phosphorylated primer and at least one of the populations of nucleic acid fragments are used to generate variant nucleic acid sequences. In a preferred embodiment, ligation substrates are formed from at least two different donor fragment pairs. The donor fragment pairs may be generated from the same template or from different templates.

In a preferred embodiment, the ligation product is generated using the following steps: (1) generating at least two donor fragments from a template molecule using primer dependent DNA polymerization wherein one strand comprises a purification tag and the other strand comprises a 5'-phosphorylated overhang; (2) removing strands tagged with a purification tag using a suitable capture molecule; (3) annealing the remaining 5'-phosphorylated strand to form first and second ligation substrates; and, (4) ligating said first and second ligation substrates after annealing strands with 5' phosphorylated overhangs to generate nucleic acid molecules encoding variant proteins. (see Kneidinger, Graininger and Messner, Biotechniques 30: 249-252 (2001); Au, Yang, Yand, Lo, and Kao; Biochem Biophys Res Comm 248: 200-203 (1998)). Each of the above-cited references are herein expressly incorporated by reference. This method is more fully described in U.S. Pat. No. 6,110,668 and WO9815567.

In a preferred embodiment, the donor fragments are generated using modified primers and a polymerase. The nucleic acid template may be single stranded (i.e. M13 DNA) or double stranded (i.e., plasmid, genomic, or cDNA). The overall design of the primers will depend on the linkage scheme between the donor fragments. For example, (Figure 20) illustrates the controlled linkage between two neighboring fragments A and B. Initially for each gene fragment, a pair of donor fragments is generated (DFA1/DFA2 and DFB1/DFB2). The donor fragment pairs are designed such that the sense strand from one donor fragment, DFA1 or DFB1, complements the antisense strand of the other donor fragment, DFA2 or DFB2, and creates a 5'-phosphorylated overhang on the hybrid product of the corresponding two strands. The overhang is located on the side where two neighboring gene fragments are to be joined. The sequence of the overhang is a sequence that belongs either to the 3'-end of fragment A or the 5'-end of fragment B (in Figure 10, it belongs to B FIX). The strands not used to form the sticky end hybrid molecule are removed using a purification tag.

In a preferred embodiment, the strands not used to form the sticky end are removed using biotin/streptavidin capture technology as is known in the art. In an alternative embodiment, a 5'-phosphorylated primer is incorporated on the strand to be removed, followed by digestion of this strand with lambda exonuclease. Subsequent 5'-phosphorylation of the remaining strand will allow formation of a hybrid molecule with a phosphorylated overhang.

In a preferred embodiment, equimolar amounts of the corresponding single strands of the donor fragments are combined under conditions suitable to renature double stranded molecules (A/A' and B/B'), with a 5'-phosphorylated overhang. Preferably, these double stranded molecules, also referred to herein as ligation substrates are joined using enzymatic or non enzymatic ligation to form a nucleic acid ligation product that encodes a protein variant. Alternatively, the ligation substrate is not ligated, but instead is used as a source of donor fragments and the process repeated.

The following U.S. patents are incorporated herein in their entirety: U.S. Patent No. 6,188,965; U.S. Patent No. 6,269,312; and U.S. Patent No. 6,403,312. The following U.S. patent applications are incorporated herein in their entirety: U.S.S.N. 09/927,790, filed August 10, 2001 and U.S.S.N. 10/101,499, filed March 18, 2002.

In a preferred embodiment, the oligonucleotides are pooled in equal proportions and multiple PCR reactions are performed to create full length sequences containing the combinations of mutations defined by the secondary library. In addition, this may be done using methods that introduce additional variations, such as error-prone amplification (e.g. PCR) methods or by intentionally introducing other variables.

In a preferred embodiment, the different oligonucleotides are added in relative amounts corresponding to either a probability distribution table or to an arbitrary or computationally derived formula. The multiple PCR reactions thus result in full length sequences with the desired combinations of mutations in the desired proportions.

The total number of oligonucleotides needed is a function of the number of positions being mutated and the number of mutations being considered at these positions:

(number of oligos for constant positions) + M1 + M2 + M3 + Mn = (total number of oligos required),

where Mn is the number of mutations considered at position n in the sequence.

In a preferred embodiment, each overlapping oligonucleotide comprises only one position to be varied; in alternate embodiments, the variant positions are too close together to allow this and multiple variants per oligonucleotide are used to allow complete recombination of all the possibilities. That is, each oligo can contain the codon for a single position being mutated, or for more than one position being mutated. The multiple positions being mutated must be close in sequence to prevent the oligo length from being impractical. For multiple mutating positions on an oligonucleotide, particular combinations of mutations can be included or excluded in the library by including or exduding the oligonucleotide encoding that combination. For example, as discussed herein, there may be correlations between variable regions; that is, when position X is a certain residue, position Y must (or must not) be a particular residue. These sets of variable positions are sometimes referred to herein as a "cluster". When the clusters are comprised of residues close together, and thus can reside on one oligonuclotide primer, the clusters can be set to the "good" correlations, and eliminate the bad combinations that may decrease the effectiveness of the library. However, if the residues of the cluster are far apart in sequence, and thus will reside on different oligonuclotides for synthesis, it may be desirable to either set the residues to the "good" correlation, or eliminate them as variable residues entirely. In an alternative embodiment, the library may be generated in several steps, so that the cluster mutations only appear together. This procedure, i.e., the procedure of identifying mutation clusters and either placing them on the same oligonucleotides or eliminating them from the library or library generation in several steps preserving clusters, can considerably enrich the experimental library with properly folded protein. Identification of clusters can be carried out by a number of ways, e. g. by using known pattern recognition methods, comparisons of frequencies of occurrence of mutations or by using energy analysis of the sequences to be experimentally generated (for example, if the energy of interaction is high, the positions are correlated). these correlations may be positional correlations (e.g. variable positions 1 and 2 always change together or never change together) or sequence correlations (e.g. if there is a residue A at position 1, there is always residue B at position 2). See: Pattern discovery in Biomolecular Data: Tools, Techniques, and Applications; edited by Jason T.L. Wang, Bruce A. Shapiro, Dennis Shasha. New York: Oxford Unviersity, 1999; Andrews, Harry C. Introduction to mathematical techniques in patter recognition; New York, Wiley-Interscience [1972]; Applications of Pattern Recognition; Editor, K.S. Fu. Boca Raton, Fla. CRC Press, 1982; Genetic Algorithms for Pattern Recognition; edited by Sankar K. Pal, Paul P. Wang. Boca Raton : CRC Press, c1996; Pandya, Abhijit S., Pattern recognition with Neural networks in C++/Abhijit S. Pandya, Robert B. Macy. Boca Raton, Fla.: CRC Press, 1996; Handbook of pattern recognition and computer vision / edited by C.H. Chen, L.F. Pau, P.S.P. Wang. 2nd ed. Signapore; River Edge, N.J. : World Scientific, c1999; Friedman, Introduction to Pattern Recognition : Statistical, Structural, Neural, and Fuzzy Logic Approaches; River Edge, N.J. : World Scientific, c1999, Series title; Serien a machine perception and artificial intelligence; vol. 32; all of wh ich are expressly incorporated by reference. In addition programs used to search for consensus motifs can be used as well.

In addition, correlations and shuffling can be fixed or optimized by altering the design of the oligonucleotides; that is, by deciding where the oligonucleotides (primers) start and stop (e.g. where the sequences are "cut"). The start and stop sites of oligos can be set to maximize the number of clusters that appear in single oligonucleotides, thereby enriching the library with higher scoring sequences. Different oligonucleotides start and stop site options can be computationally modeled and ranked according to number of clusters that are represented on single oligos, or the percentage of the resulting sequences consistent with the predicted libarary of sequences.

The total number of oligonucleotides required increases when multiple mutable positions are encoded by a single oligonucleotide. The annealed regions are the ones that remain constant, i.e. have the sequence of the reference sequence.

Oligonucleotides with insertions or deletions of codons can be used to create a library expressing different length proteins. In particular computational sequence screening for insertions or deletions can result in secondary libraries defining different length proteins, which can be expressed by a library of pooled oligonucleotide of different lengths.

Preferably, an individual gene that serves as the template nucleic acid is obtained from at least two different species. In this embodiment, the gene from one species is cloned into a vector to produce a template molecule comprising single stranded nucleic acid molecules. The DNA from the second species is cleaved into fragments. The resulting fragments are added to the template molecule under conditions that permit the fragments to anneal to the template molecule. Unhybridized termini are enzymatically removed. Gaps between hybridized fragments are filled using an appropriate enzyme, such as a polymerase and nicks sealed using a ligase. The chimeric gene can be amplified using suitable primers or other techniques that are well known to those of skill in the art

In a preferred embodiment, sequences derived from introns are used to mediate specific cleavage and ligation of discontinuous nucleic acid molecules to create libraries of novel genes and gene products as described in U.S. Patent Nos. 5,498,531, and 5,780,272, both of which are hereby expressly incorporated by reference in their entirety. In one embodiment, a library of ribonucleic acids encoding a novel gene product or novel gene products is created by mixing splicing constructs comprising an exon and 3' and 5' intron fragments. See U.S. Patent No. 5,498,531.

In another embodiment, DNA sequence libraries are created by mixing DNA/RNA hybrid molecules that contain intron derived sequences that are used to mediate specific cleavage and ligation of the DNA/RNA hybrid molecules such that the DNA sequences are covalently linked to form novel DNA sequences as described in U.S. Patent No. 6,150,141, WO 00/40715 and WO 00/17342, all of which are hereby expressly incorporated by reference in their entirety.

In a preferred embodiment, the secondary library is done by shuffling the family (e.g. a set of variants); that is, some set of the top sequences (if a rank-ordered list is used) can be shuffled, either with or without error-prone PCR. "Shuffling" in this context means a recombination of related sequences, generally in a either a targeted or random way. It can include "shuffling" as defined and exemplified in U.S. Patent Nos. 5,830,721; 5,811,238; 5,605,793; 5,837,458 and PCT US/19256, all of which are expressly incorporated by reference in their entirety. This set of sequences can also be an artificial set; for example, from a probability table (for example generated using SCMF) or a Monte Carlo set. Similarly, the "family" can be the top 10 and the bottom 10 sequences, the top 100 sequences, etc. This may also be done using error-prone PCR.

Thus, in a preferred embodiment, in silico shuffling is done using the computational methods described therein.

That is, starting with either two libraries or two sequences, random recombinations of the sequences can be generated and evaluated computationally, and then experimental libraries generated.

**PCR with pooled oligos**

Use of pooled oligos for synthetic shuffling is more fully described In U.S. Pat. No. 6,368,861 (see also US6423542; US6376246; US6368861; US6319714; WO0042561A3; WO0042561A2; WO0042560A3; WO0042560A2; WO0042559A1; WO0018906C2; WO0018906A3; and WO0018906A2.)

In a preferred embodiment, PCR using a wild type gene or other gene may be used, as is schematically depicted in Figure 15. In this embodiment, a starting gene is used: the gene may the wild-type gene, the gene encoding the global optimized sequence, or any other sequence of the list. In this embodiment, oligonucleotides are used that correspond to the variant positions and contain the different amino acids of the secondary library. PCR is done using PCR primers at the termini, as is known in the art. PCR provides many benefits namely, fewer oligonucleotides, may result In fewer errors, and if the wild type gene is used, it need not be synthesized. An alternative method for creating members of the library, are ligase chain reaction-based methods, (see Chalmers and Cumow, Biotechniques 30 (2001) 249-252), which in herein expressly incorporated by reference.

In a preferred embodiment, these oligonucleotides are pooled in equal proportions and multiple PCR reactions are performed to create full-length sequences containing the combinations of mutations defined by the secondary library. In a preferred embodiment, the different oligonucleotides are added in relative amounts, e.g. in amounts corresponding to a probability distribution table, an alignment, or other parameters. The multiple PCR reactions thus result in full-length sequences with the desired combinations of mutations in the desired proportions.

**Number of mutations per oligo**

In a preferred embodiment, each overlapping oligonucleotide comprises at least one or more positions to be varied and zero or more positions that are not varied. As may be appreciated by one skilled in the art, the distance between multiple variants may affect the completeness of recombination of all possible library members. That is, each oligo may contain the codon for a single position being mutated, or for more than one position being mutated. For multiple mutating positions on an oligonucleotide, particular combinations of mutations may be included or excluded in the library by including or excluding the oligonucleotide encoding that combination. The total number of oligonucleotides required increases when multiple mutable positions are encoded by a single oligonucleotide. The annealed regions are the ones that remain constant, i.e. have the sequence of the reference sequence.

**Random codons**

In some cases, oligos with random mutations may be used. That is, any amino acid may be represented at a codon position. As known by those skilled in the art, subsets of random codons may be used, where the bias is for or against specific amino acids. By judicial design, certain amino acids may be favored or excluded from the set of possible mutations.

Multiple DNA libraries may be synthesized that code for different subsets of amino acids at certain positions, allowing generation of the amino acid diversity desired without having to fully randomize the codon and thereby waste sequences in the library on stop codons, frameshifts, undesired amino acids, etc. This may be done by creating a library that at each position to be randomized is only randomized at one or two of the positions of the triplet where the position(s) left constant are those that the amino acids to be considered at this position have in common. Multiple DNA libraries may be created to insure that all amino acids desired at each position exist in the aggregate library. Alternatively, shuffling, as is generally known in the art, may be done with multiple libraries. Alternatively, the random peptide libraries may be done using the frequency tabulation and experimental generation methods including, multiplexed PCR, shuffling, and the like.

**Error-prone PCR**

In a preferred embodiment, error-prone amplification methods (e.g. error prone PCR) is done to generate additional members of the secondary library, or the whole library. See U.S. Patent Nos. 5,605,793, 5,811,238, and 5,830,721, all of which are hereby incorporated by reference. This may be done on the optimal sequence or on top members of the library, or some other artificial set or family. Error prone PCR is then performed on the optimal sequence gene in the presence of oligonucleotides that code for the mutations at the variable residue positions of the secondary library (bias oligonucleotides). The addition of the oligonucleotides will create a bias favoring the incorporation of the mutations in the secondary library. Alternatively, only oligonucleotides for certain mutations may be used to bias the library.

In addition to error-prone PCR, mutations could be introduced in specific regions using minor modifications to several other methods, either in vitro or in vivo, including but not limited to "DNA shuffling" (see WO 00/42561 A3; WO 01/70947 A3;), exon shutting (see US 6365 377 B1; Kolkman & Stemmer (2001) Nature Biotechnology 19, 423-428), family shuffling (see Crameri et al. (1998) Nature 391, 288-291; US 6376246 B1), RACHITT™ (Coco et al. (2001) Nature Biotechnology 19, 354- 359; WO 02/06469 A2), STEP and random priming of In vitro recombination ( see Zhao at al., (1998) Nature Biotechnology 16, 258-261; Shao et al (1998) Nucleic Acids Research 26, 681-683; exonuclease mediated gene assembly (US 6352842 B1, US 6361974 B1), Gene Site Saturation Mutagenesis™ (US 6358709 B1), Gene Reassembly™ (US 6358709B1) and SCRATCHY (Lutz et al.(2001), PNAS 98, 11248-11253), DNA fragmentation methods (Kikuchi et al.; Gene 236, 159-167), single-stranded DNA shuffling (Kikuchi et al., (2000) Gene 243, 133-137). Although these methods are intended to introduce random mutations throughout the gene, those skilled in the art will appreciate that specific regions (those defined by computational methods such as PDA™ technology: see WO 01175767) of the gene could be mutated, whilst others could be left untouched, either by isolating and combining the mutated region with the unmodified region (for example, by cassette mutagenesis; see WO 01175767 A2; Kim & Mass, (2000) Biotechniques 28, 196-198; Lanio & Jeltsch (1998) Biotechniques 25, 958- 965; Ge & Rudolph (1997) Biotechniques 22, 28- 30; Ho et al., (1989) Gene 77, 51059), or via *in vitro* or *in vivo* recombination (see for example see WO 02/10183 A1 and Abécassis et al., (2000) Nucleic Acids Research 28, e88 for examples). All of the above-cited references are hereby expressly incorporated by reference. In addition, it should be noted that the computational equivalents of all of these methods can be used as a computational step to generate primary and/or secondary libraries. That is, "in silico" shuffling of a primary library rank-ordered list may be further "shuffled" using experimental procedures.

## Additional methods for gene construction

The creation of members of the secondary library may be performed by several other methods, including, but not limited to, classical site-directed mutagenesis, e.g. Quickchange commercially available from Stratagene, cassette mutagenesis as well as other amplification techniques. Cassette mutagenesis could include the creation of DNA molecules from restriction digestion fragments using nucleic acid ligation, and includes the random ligation of restriction fragments (see Kikuchi et al., (1999), Gene 236, 159-167). Additionally, cassette mutagenesis could also be achieved using randomly-cleaved nucleic acids (see Kikuchi et al., (1999), Gene 236, 133-137), by PCR-ligation PCR mutagenesis (see for example Ali & Steinkasserer (1995), Biotechniques 18, 746-750), by seamless gene engineering using RNA- and DNA- overhang cloning (see Roc & Doc; Coljee et al., (2000) Nature Biotechnology 18, 789-791), by ligation mediated gene construction (U.S.S.N. 60/311,545), by homologous or non-homologous random recombination (see US6,368,861; US6423542; US6376246; US6368861; US6319714; WO0042561A3; WO0042561A2; WO0042560A3; WO0042560A2; WO0042559A1; WO0018906C2; WO0018906A3; and WO0018906A2), or *in vivo* using recombination between flanking sequences (see WO 02/10183 A1 and Abécassis et al., (2000) Nucleic Acids Research 28, e88 for examples). in addition, regions of the gene could be mutated in E. coli lacking correct mismatch repair mechanisms, (e.g. E.coli *XLmutS* strain commercially available from Stratagene), or by using phage display techniques to evolve a library (e.g. Long-McGie et al., (2000), Biotechnol Bioeng 68, 121-125).

In addition to the PCR methods outlined herein, there are other amplification and gene synthesis methods that can be used. For example, the library genes may be "stitched" together using pools of oligonucleotides with polymerases (and optionally or solely) ligases. These resulting variable sequences can then be amplified using any number of amplification techniques, including, but not limited to, polymerase chain reaction (PCR), strand displacement amplification (SDA), nucleic acid sequence based amplification (NASBA), ligation chain reaction (LCR) and transcription mediated amplification (TMA). In addition, there are a number of variations of PCR which may also find use in the invention, including "quantitative competitive PCR" or "QC-PCR", "arbitrarily primed PCR" or "AP-PCR" , "immuno-PCR", "Alu-PCR", "PCR single strand conformational polymorphism" or "PCR-SSCP", "reverse transcriptase PCR" or "RT-PCR", "biotin capture PCR", "vectorette PCR". "panhandle PCR", and "PCR select cDNA subtration", among others. Furthermore, by incorporating the T7 polymerase initiator into one or more oligonucleotides, IVT amplification can be done.

## Experimental Modification of Libraries to Generate Further Libraries

It will be appreciated by those skilled in the art that many of the methods used to construct the secondary libraries can be used in further modifications. For example, cassette mutagenesis could include the creation of DNA molecules from restriction digestion fragments using nucleic acid ligation, and includes the random ligation of restriction fragments (see Kikuchi et al., (1999), Gene 236, 159-167). Additionally, cassette mutagenesis could also be achieved using randomly-cleaved nucleic acids (see Kikuchi et al., (1999), Gene 236, 133-137), by PCR-ligation PCR mutagenesis (see Ali & Steinkasserer (1995), Biotechniques 18, 746-750), by seamless gene engineering using RNA- and DNA- overhang cloning (Roc & Doc; Coljee et al., (2000) Nature Biotechnology 18, 789-791). by ligation mediated gene construction (U.S.S.N. 60/311,545), by homologous or non-homologous random recombination (see US6,368,861; US6423542; US6376246; US6368861; US6319714; WO0042561A3; WO0042561A2; WO0042560A3; WO0042560A2; WO0042559A1; WO0018906C2; WO0018906A3; and W00018906A2).

Tertiary libraries could be created from secondary libraries using any of the techniques outlined herein or one or more of the following, either in a step-wise fashion or in combination: DNA shuffling (see WO 00/42561 A3; WO 01/70947 A3;), exon shuffling (see US 6365 377 B1; Kolkman & Stemmer (2001) Nature Biotechnology 19, 423-428), Family Shuffling (see Crameri et al. (1998) Nature 391, 288-291; US 6376246 B1), RACHITT™ (see Coco et al. (2001) Nature Biotechnology 19, 354- 359; WO 02/06469 A2), STEP and random priming of *in vitro* recombination (see Zhao et al., (1998) Nature Biotechnology 16, 258-261; Shao et al (1998) Nucleic Acids Research 26, 681-683; exonuclease mediated gene assembly (see US 6352842 B1, US 6361974 B1), Gene Site Saturation Mutagenesis™ (see US 6358709 B1), Gene Reassembly™ (see US 6358709B1) and SCRATCHY (see Lutz et al.(2001), PNAS 98, 11248-11253), DNA fragmentation methods (see Kikuchi et al., Gene 236, 159-167), single-stranded DNA shuffling (see Kikuchi et al., (2000) Gene 243, 133-137), *in vitro* or *in vivo* recombination (see WO 02/10183 A1 and Abécassis et al., (2000) Nucleic Acids Research 28, e88 for examples). Additionally, *in vivo* mutagenesis could be performed in strains of *E.coli* that lack correct DNA mismatch repair mechanisms. e.g. E.coli XL *mut*S strain commercially available from Stratagene, or by using phage display techniques to evolve a library (e.g. Long-McGie et al., (2000), Biotechnol Bioeng 68, 121-125).

**Preferred Combinations**

In general, as more fully outlined below, the invention can take on a wide variety of configurations. In general, primary libraries, e.g. libraries of all or a subset of possible proteins are generated computationally. This can be done in a wide variety of ways, including sequence alignments of related proteins, structural alignments, structural prediction models, databases, or (preferably) protein design automation computational analysis. Similarly, primary libraries can be generated via sequence screening using a set of scaffold structures that are created by perturbing the starting structure (using any number of techniques such as molecular dynamics, Monte Carlo analysis) to make changes to the protein (including backbone and sidechain torsion angle changes). Optimal sequences can be selected for each starting structures (or, some set of the top sequences) to make primary libraries.

Some of these techniques result in the list of sequences in the primary library being "scored", or "ranked" on the basis of some particular criteria. In some embodiments, lists of sequences that are generated without ranking can then be ranked using techniques as outlined below.

In a preferred embodiment, some subset of the primary library is then experimentally generated to form a secondary library. Alternatively, some or all of the primary library members are recombined to form a secondary library, e.g. with new members. Again, this may be done either computationally or experimentally or both.

Alternatively, once the primary library is generated, it can be manipulated in a variety of ways. In one embodiment, a different type of computational analysis can be done; for example, a new type of ranking may be done. Alternatively, and the primary library can be recombined, e.g. residues at different positions mixed to form a new, secondary library. Again, this can be done either computationally or experimentally, or both.

As will be appreciated by those in the art, there are a number of specific combinations that can be used with the methods of the present invention. Examples of some preferred combinations are shown in Figures 21A-E.

**Expression Systems**

The library proteins of the present invention are produced by culturing a host cell transformed with nucleic acid, preferably an expression vector, containing nucleic acid encoding a library protein, under the appropriate conditions to induce or cause expression of the library protein. The conditions appropriate for library protein expression will vary with the choice of the expression vector and the host cell, and will be easily ascertained by one skilled in the art through routine e xperimentation. For example, the use of constitutive promoters in the expression vector will require optimizing the growth and proliferation of the host cell, while the use of an inducible promoter requires the appropriate growth conditions for induction. In addition, in some embodiments, the timing of the harvest is important. For example, the baculoviral systems used in insect cell expression are lytic viruses, and thus harvest time selection can be crucial for product yield.

**Examples of expression systems**

As will be appreciated by those in the art, the type of cells used in the present Invention can vary widely. The lists that follow are applicable both to the source of scaffold proteins as well as to host cells in which to produce the variant libraries. A wide variety of appropriate host cells can be used, including yeast, bacteria, archaebacteria, fungi, and insect, plant and animal cells, including mammalian cells. Of particular interest are *Drosophila melanogaster* cells, *Saccharomyces cerevisiae* and other yeasts, *E. coli, Bacillus subtilis*, *Streptococcus cremoris, Streptococcus lividans*, pED (commercially available from Novagen), pBAD and pCNDA (commercially available from invitrogen), pEGEX (commercially available from Amersham Biosciences), pQE (commercially available from Qiagen), SF9 cells, C129 cells, 293 cells, Neurospora, BHK, CHO, COS, and HeLa cells, fibroblasts, Schwanoma cell lines, immortalized mammalian myeloid and lymphoid cell lines, Jurkat cells, mast cells and other endocrine and exocrine cells, and neuronal cells. See the ATCC cell line catalog, hereby expressly incorporated by reference. In one embodiment, the cells may be genetically engineered, that is, contain exogenous nucleic acid, for example, to contain target molecules.

In a preferred embodiment, the library proteins are expressed in mammalian expression systems, including systems in which the expression constructs are introduced into the mammalian cells using virus such as retrovirus or adenovirus. Any mammalian cells may be used, with mouse, rat, primate and human cells being particularly preferred, although as will be appreciated by those in the art, modifications of the system by pseudotyping allows all eukaryotic cells to be used, preferably higher eukaryotes. Accordingly, suitable mammalian cell types include, but are not limited to, tumor cells of all types (particularly melanoma, myeloid leukemia, carcinomas of the lung, breast, ovaries, colon, kidney, prostate, pancreas and testes), cardiomyocytes, endothelial cells, epithelial cells, lymphocytes (T-cells and B cells) , mast cells, eosinophils, vascular intimal cells, hepatocytes, leukocytes including mononuclear leukocytes, stem cells such as haemopoetic, neural, skin, lung, kidney, liver and myocyte stem cells (for use in screening for differentiation and de-differentiation factors), osteoclasts, chondrocytes and other connective tissue cells, keratinocytes, melanocytes, liver cells, kidney cells, and adipocytes. Suitable cells also include known research cells, including, but not limited to, Jurkat T cells, NIH3T3 cells, CHO, Cos, etc. Again, scaffold proteins may be obtained from these sources as well.

In a preferred embodiment, library proteins are expressed in bacterial systems, including bacteria in which the expression constructs are introduced into the bacteria using phage. Bacterial expression systems are well known in the art, and include *Bacillus subtilis, E. coli, Streptococcus cremoris*, and *Streptococcus lividans*

In an alternate embodiment, library proteins are produced in insect cells, including but not limited to *Drosophila melanogaster* S2 cells, as well as cells derived from members of the order Lepidoptera which includes all butterflies and moths, such as the silkmoth *Bombyx mori* and the alphalpha looper *Autographa califomica*. Lepidopteran insects are host organisms for some members of a family of virus, known as baculoviruses (more than 400 known species), that infect a variety of arthropods. (see U.S. 6,090,584).

In an alternate embodiment, library proteins are produced in insect cells. The library can be transfected into SF9 *Spodoptera frugiperda* insect cells to generate baculovirus which are used to infect SF21 or High Five commercially available from Invitrogen, insect cells for high level protein production. Also, transfections into the *Drosophila* Schneider S2 cells will express proteins.

In a preferred embodiment, library protein is produced in yeast cells. Yeast expression systems are well known in the art, and include expression vectors for *Saccharomyces cerevisiae*, *Candida albicans* and C. *maltosa, Hansenula polymorpha*, *Kluyveromyces fragilis* and *K. lactis*, *Pichia guillerimondil* and *P. pastoris, Schizosaccharomyces pombe*, and *Yarrowia lipolytica.*

In one embodiment the library proteins are expressed *in vitro* using cell free translation systems. Several commercial sources are available for this including but not limited to Roche Rapid Translation System, Promega TnT system, Novagen's EcoPro system, Ambion's ProteinScipt-Pro system. *In* vitro translation systems derived from both prokaryotic (e.g. *E. coli*) and eukaryotic (e.g. Wheat germ, Rabbit reticulocytes) cells are available and can be chosen based on the expression levels and functional properties of the protein of interest Both linear (as derived from a PCR amplification) and circular (as in plasmid) DNA molecules are suitable for such expression as long as they contain the gene encoding the protein operably linked to an appropriate promoter. Other features of the molecule that are important for optimal expression in either the bacterial or eukaryotic cells (including the ribosome binding site etc) are also Included in these constructs. The proteins can again be expressed individually or in suitable size pools consisting of multiple library members. The main advantage offered by these *in vitro* systems is their speed and ability to produce soluble proteins. In addition the protein being synthesized can be selectively labeled if needed for subsequent functional analysis.

**Transformation and transfection methods**

The methods of introducing exogenous nucleic acid into host cells is well known in the art, and will vary with the host cell used. Techniques include dextran-mediated transfection, calcium phosphate precipitation, calcium chloride treatment, polybrene mediated transfection, protoplast fusion, electroporation, viral or phage infection, encapsulation of the polynucleotide(s) in liposomes, and direct microinjection of the DNA into nuclei. In the case of mammalian cells, transfection may be either transient or stable.

*Expression Vectors*

A variety of expression vectors may be utilized to express the library proteins. The expression vectors are constructed to be compatible with the host cell type. Expression vectors may comprise self-replicating extrachromosomal vectors or vectors which integrate into a host genome. Expression vectors typically comprise a library member, any fusion constructs, control or regulatory sequences, selectable markers, and/or additional elements.

Preferred bacterial expression vectors include but are not limited to pET, pBAD, bluescript, pUC, pQE, pGEX, pMAL, and the like.

Preferred yeast expression vectors include pPICZ, pPIC3.5K, and pHIL-SI commercially available from Invitrogen.

Expression vectors for the transformation of insect cells, and in particular, baculovirus-based expression vectors, are well known in the art and are described e.g., in O'Reilly et al., *Baculovirus Expression Vectors: A Laboratory Manual* (New York: Oxford University Press, 1994).

A preferred mammalian expression vector system is a retroviral vector system such as is generally described in Mann et al., Cell, 33:153-9 (1993); Pear et al., Proc. Natl. Acad. Sci. U.S.A., 90(18):8392-6 (1993); Kitamura et al., Proc. Natl. Acad. Sci. U.S.A, 92:9146-50 (1995); Kinsella et al., Human Gene Therapy, 7:1405-13; Hofmann et al., Proc. Natl. Acad. Sci. U.S.A., 93:5185-90; Choate et al., Human Gene Therapy, 7:2247 (1996); PCT/US97/01019 and PCT/US97/01048, and references cited therein, all of which are hereby expressly incorporated by reference.

## Inclusion of control or regulatory sequences

Generally, expression vectors include transcriptional and translational regulatory nucleic acid sequences which are operably linked to the nucleic acid sequence encoding the library protein.

The transcriptional and translational regulatory nucleic acid sequences will generally be appropriate to the host cell used to express the library protein, as will be appreciated by those in the art. For example, transcriptional and translational regulatory sequences from *E. coli* are preferably used to express proteins in *E. coli*.

Transcriptional and translational regulatory sequences may include, but are not limited to, promoter sequences, ribosomal binding sites, transcriptional start and stop sequences, translational start and stop sequences, and enhancer or activator sequences. In a preferred embodiment, the regulatory sequences comprise a promoter and transcriptional and translational start and stop sequences.

A suitable promoter Is any nucleic acid sequence capable of binding RNA polymerase and initiating the downstream (3') transcription of the coding sequence of library protein into mRNA. Promoter sequences may be constitutive or inducible. The promoters may be naturally occurring promoters, hybrid or synthetic promoters.

A suitable bacterial promoter has a transcription initiation region which is usually placed proximal to the 5' end of the coding sequence. The transcription initiation region typically includes an RNA polymerase binding site and a transcription initiation site. In *E. coli*, the ribosome-binding site is called the Shine-Dalgamo (SD) sequence and includes an initiation codon and a sequence 3-9 nucleotides in length located 3 - 11 nucleotides upstream of the initiation codon. Promoter sequences for metabolic pathway enzymes are commonly utilized. Examples include promoter sequences derived from sugar metabolizing enzymes, such as galactose, lactose and maltose, and sequences derived from biosynthetic enzymes such as tryptophan. Promoters from bacteriophage, such as the T7 promoter, may also be used. In addition, synthetic promoters and hybrid promoters are also useful; for example, the *tac* promoter is a hybrid of the *trp* and *lac* promoter sequences.

Preferred yeast promoter sequences include the inducible GAL1,10 promoter, the promoters from alcohol dehydrogenase, enolase, glucokinase, glucose-6-phosphate isomerase, glyceraldehyde-3-phosphate-dehydrogenase, hexokinase, phosphofructokinase, 3-phosphoglycerate mutase, pyruvate kinase, and the acid phosphatase gene.

A suitable mammalian promoter will have a transcription initiating region, which is usually placed proximal to the 5' end of the coding sequence, and a TATA box, usually located 25-30 base pairs upstream of the transcription initiation site. The TATA box is thought to direct RNA polymerase II to begin RNA synthesis at the correct site. A mammalian promoter will also contain an upstream promoter element (enhancer element), typically located within 100 to 200 base pairs upstream of the TATA box. Typically, transcription termination and polyadenylation sequences recognized by mammalian cells are regulatory regions located 3' to the translation stop codon and thus, together with the promoter elements, flank the coding sequence. The 3' terminus of the mature mRNA is formed by site-specific post-translational cleavage and polyadenylation. Examples of transcription terminator and polyadenylation signals Include those derived from SV40. An upstream promoter element determines the rate at which transcription is initiated and can act in either orientation. Of particular use as mammalian promoters are the promoters from mammalian viral genes, since the viral genes are often highly expressed and have a broad host range. Examples include the SV40 early promoter, mouse mammary tumor virus LTR promoter, adenovirus major late promoter, herpes simplex virus promoter, and the CMV promoter.

### Inclusion of a selectable marker

In addition, In a preferred embodiment, the expression vector contains a selection gene or marker to allow the selection of transformed host cells containing the expression vector. Selection genes are well known in the art and will vary with the host cell used.

For example, a bacterial expression vector may include a selectable marker gene to allow for the selection of bacterial strains that have been transformed. Suitable selection genes include genes which render the bacteria resistant to drugs such as ampicillin, chloramphenicol, erythromycin, kanamycin, neomycin and tetracycline.

Yeast selectable markers include the biosynthetic genes ADE2, HIS4, LEU2, and TRP1 when used in the context of auxotrophe strains; ALG7, which confers resistance to tunicamycin; the neomycin phosphotransferase gene, which confers resistance to G418; and the CUP1 gene, which allows yeast to grow in the presence of copper ions.

Suitable mammalian selection markers include, but are not limited to, those that confer resistance to neomycin (or its analog G418). blasticidin S, histinidol D, bleomycin, puromycin, hygromycin B, and other drugs. Selectable markers conferring survivability in a specific media include, but are not limited to Blasticidin S Deaminase, Neomycin phophotranserase II, Hygromycin B phosphotranserase, Puromycin N-acetyl transferase, Bleomycin resistance protein (or Zeocin resistance protein, Phleomycin resistance protein, or phleomycin/zeocin binding protein), hypoxanthine guanosine phosphoribosyl transferase (HPRT), Thymidylate synthase, xanthine-guanine phosphorldosyl transferase, and the like.

### Inclusion of additional elements

In addition, the expression vector may comprise additional elements. In a preferred embodiment, the vector contains a fusion protein, as discussed below. In another embodiment, the expression vector may have two replication systems, thus allowing it to be maintained In two organisms, for example in mammalian or insect cells for expression and in a prokaryotic host for cloning and amplification. Furthermore, for integrating expression vectors, the expression vector contains at least one sequence homologous to the host cell genome, and preferably two homologous sequences which flank the expression construct. The integrating vector may be directed to a specific locus in the host cell by selecting the appropriate homologous sequence for inclusion in the vector. Such vectors may include *cre-lox* recombination sites, or *att*R, *att*B, *att*P, and *att*L sites. Constructs for integrating vectors and appropriate selection and screening protocols are well known in the art and are described in e.g., Mansour et al., *Cell*, 51:503 (1988) and Murray, *Gene Transfer and Expression Protocols, Methods in Molecular Biology, Vol. 7* (Clifton: Humana Press, 1991). In a preferred embodiment, the expression vector contains a RNA splicing sequence upstream or downstream of the gene to be expressed in order to increase the level of gene expression.. (See Barret et al., Nucleic Acids Res. 1991; Groos et al., Mol. Cell. Biol. 1987; and Budiman et al., Mol. Cell. Biol. 1988.)

### Fusion Constructs

The library protein may also be made as a fusion protein, using techniques well known in the art. For example, fusion partners such as targeting sequences can be used which allow the localization of the library members into a subcellular or extracellular compartment of the cell. Purification tags may be fused with a library, allowing the purification or isolation of the library protein. Rescue sequences can be used to enable the recovery of the nucleic acids encoding them. Other fusion sequences are possible, such as fusions which enable utilization of a screening or selection technology.

### Targeting or signal sequences

The expression vector may also include a signal peptide sequence that directs library protein and any associated fusions to a desired cellular location or to the extracellular media. Suitable targeting sequences include, but are not limited to, binding sequences capable of causing binding of the expression product to a predetermined molecule or class of molecules while retaining bioactivity of the expression product, (for example by using enzyme inhibitor or substrate sequences to target a dass of relevant enzymes); sequences signaling selective degradation, of itself or co-bound proteins; and signal sequences capable of constitutively localizing the candidate expression products to a predetermined cellular locale, including a) subcellular locations such as the Golgi, endoplasmic reticulum, nucleus, nucleoli, nudear membrane, mitochondria, chloroplast, secretory vesicles, lysosome, and cellular membrane; and b) extracellular locations via a secretory signal. Target sequences also may be used in conjunction with cell surface display technology as discussed below.

Particularly preferred is localization to either subcellular locations or to the outside of the cell via secretion. For example some targeting sequences enable secretion of library protein in bacteria. The signal sequence typically encodes a signal peptide comprised of hydrophobic amino acids which direct the secretion of the protein from the cell, as is well known in the art. This method may be useful for gram-positive bacteria or gram-negative bacteria. The protein can be either secreted into the growth media or into the periplasmic space, located between the inner and outer membrane of the cell.

## Purification tags

In a preferred embodiment, the library member comprises a purification tag operably linked to the rest of the library peptide or protein. A purification tag is a sequence which may be used to purify or isolate the candidate agent, for detection, for immunoprecipitation, for FACS (fluorescence-activated cell sorting), or for other reasons. Thus, for example, purification tags include purificatio n sequences such as polyhistidine, including but not limited to His$_6$, or other tag for use with Immobilized Metal Affinity Chromatography (IMAC) systems (e.g. Ni$^{+2}$ affinity columns), GST fusions, MBP fusions, Strep-tag, the BSP biotinylation target sequence of the bacterial enzyme BirA, and epitope tags which are targeted by antibodies. Suitable epitope tags include but are not limited to c-myc (for use with the commercially available 9E10 antibody), flag tag, and the like.

## Rescue fusions

A rescue fusion is a fusion protein which enables recovery of the nucleic acid encoding the library protein. In a preferred embodiment, such a rescue fusion would enable screening or selection of library members. Such fusion proteins may include but are not limited to, rep proteins, viral VPg proteins, transcription factors including but not limited to zinc fingers, RNA and DNA binding proteins, and the like. Attachment can be covalent or noncovalent

Alternatively, the rescue sequence may be a unique oligonucleotide sequence that serves as a probe target site to allow the quick and easy isolation of the retroviral construct, via PCR, related techniques, or hybridization.

in an alternate embodiment, rescue sequences could also be based upon in vivo recombination systems, such as the cre-lox system, the Invitrogen Gateway system, forced recombination systems in yeast, mammalian, plant, bacteria or fungal cells (see WO 02/10183 A1), or phage display systems.

In an alternate embodiment, display technologies are utilized. For example, in phage display (see Kay, BK et al, eds. Phage display of peptides and proteins: a laboratory manual (Academic Press, San Diego, CA, 1996); Lowman HB, Bass SH, Simpson N, Wells JA (1991) Selecting high-affinity binding proteins by monovalent phage display. Bioechemistry 30:10832-10838; Smith GP (1985) Filamentous fusion phage: novel expression vectors that display cloned antigens on the virion surface. Science 228:1315-1317.) library proteins can be fused to the gene III protein. Cell surface display (Witrrup KD, Protein engineering by cell-surface display. Curr. Opin. Biotechnology 2001, 12:395-399.) may also be useful for screening. This includes but is not limited to display on bacteria (see Georgiou G, Poetschke HL, Stathopoulos C, Francisco JA, Practical applications of engineering gram-negative bacterial cell surfaces. Trends Biotechnol. 1993 Jan;11(1):6-10; Georgiou G, Stathopoulos C, Daugherty PS, Nayak AR, Iverson BL, and Curtiss RR (1997) Display of heterologous proteins on the surface of microorganisms: from the screening of combinatorial libraries to live recombinant vaccines. Nature Biotechnol. 15, 29-34; Lee JS, Shin KS, Pan JG, Kim CJ. Surface-displayed viral antigens on Salmonella carrier vaccine. Nature Biotechnology, 2000, 18:645-648; Jun et al, 1998), yeast (see Boder ET, Wittrup KD: Yeast surface display for screening combinatorial polypeptide libraries. Nat Biotechnol 1997, 15: 553-557 Boder ET and Wittrup KD. Yeast surface display for directed evolution of protein expression, affinity, and stability. Methods Enzymol 2000, 328:430-44.), and mammalian cells (see Whitehorn EA, Tate E, Yanofsky SD, Kochersperger L, Davis A, Mortensen RB, Yonkovich S, Bell K, Dower WJ, and Barrett RW 1995. A generic method for expression and use of "tagged" soluble versions of cell surface receptors. Bio/technology, 13, 1215-1219.).

## Additional fusions that allow for screening or selection

In an alternate embodiment, a protein fragment complementation assay is used (see Johnsson N & Varshavsky A. Split Ubiquitin as a sensor of protein interactions in vivo. 1994 Proc Natl Acad Sci USA, 91: 10340-10344; Pelletier JN, Campbell-Valois FX, Michnick SW. Oligomerization domain-directed reassembly of active dihydrofolate reductase from rationally designed fragments. 1998. Proc Natl Acad Sci USA 95:12141-12146.) Other fusion methods which may allow screening include but are not limited to periplasmic expression and cytometric screening (see Chen G, Hayhurst A, Thomas JG, Harvey BR, Iverson BL, Georgiou G: Isolation of high-affinity ligand-binding proteins by periplasmic expression with cytometric screening (PECS). Nat Biotechnol 2001, 19: 537-542.), and the yeast two hybrid screen (see Fields S, Song O: A novel genetic system to detect protein-protein interactions. Nature 1989, 340:245-246.)

## Other fusions

Additional fusion partners may also be utilized. For example, library protein may be made as a fusion protein to increase expression, Increase solubility, confer stability or protection from degradation, and/or confer other properties. For example, when raising monoclonal antibodies to a small epitope, the library protein may be fused to a carrier protein to form an immunogen. According to Varshavsky's N-End Rule, susceptibility to ubiquitination and subsequent degradation can be minimized by the incorporation of glycines after the initiation methionine (MG or MGG), thus conferring long half-life in the cytoplasm. Similarly, adding two prolines to the C-terminus confers resistance to carboxypeptidase action.

## Linkers

Linker sequences may be used to connect the library protein to its fusion partner or tag. The linker sequence will generally comprise a small number of amino acids, typically less than ten. However, longer linkers may also be used. As will be appreciated by those skilled in the art, any of a wide variety of sequences may be used as linkers. Typically, linker sequences are selected to be flexible and resistant to degradation. A common linker sequence comprises the amino acid sequence GGGGS. The preferred linker between a protein and C-terminal PP tag consists of two glycines.

## Labels

In one embodiment, the library nucleic acids, proteins and antibodies of the invention are labeled. In general, labels fall into three classes: a) immune labels, which may be an epitope incorporated as a fusion constructs may which is recognized by an antibody as discussed above, isotopic labels, which may be radioactive or heavy isotopes, and c) small molecule labels which may include fluorescent and colorimetric dyes or molecules such as biotin which enable the use of other labeling techniques. Labels may be incorporated into the compound at any position and may be incorporated *in vivo* during protein or peptide expression or *in vitro*.

## Protein Purification

In a preferred embodiment, the library protein is purified or isolated after expression. Library proteins may be isolated or purified in a variety of ways known to those skilled in the art depending on what other components are present in the sample. The degree of purification necessary will vary depending on the use of the library protein. In some instances no purification will be necessary. For example in one embodiment, if library proteins are secreted, screening or selection can take place directly from the media.
Standard purification methods include electrophoretic, molecular, immunological and chromatographic techniques, including ion exchange, hydrophobic, affinity, size exclusion chromatography, and reversed-phase HPLC chromatography, as well as precipitation, dialysis, and chromatofocusing techniques. Purification can often be facilitated by the inclusion of purification tag, as described above. For example, the library protein may be purified using glutathione resin if a GST fusion is employed, Immobilized Metal Affinity Chromatography (IMAC) if a His or other tag is employed, or immobilized anti-flag antibody if a flag tag is used. Ultrafiltration and diafiltration techniques, in conjunction with protein concentration, are also useful. For general guidance in suitable purification techniques, (see Scopes, R., Protein Purification: Principles and Practice 3rd Ed., Springer-Verlag, NY (1994).), hereby expressly incorporated by reference.
In a preferred embodiment, the libraries are used in any number of display techniques. For example, the libraries may be displayed using phage or enveloped virus systems, bacterial systems, yeast two hybrid systems or mammalian systems.
In a preferred embodiment, the libraries are displayed using a phage or enveloped virus system. For example, a library of viruses, each carrying a distinct peptide sequence as part of the coat protein, can be produced by Inserting random oligonucleotides sequences into the coding sequence of viral coat or envelope proteins. Several different viral systems have been used to display peptides, as described in Smith, G.P, (1985) Science, 228:1315-1317; Santini, C., et al., (1998) J. Mol. Biol., 282:125-135; Sternberg, N. and Hoess, R.H. (1995) Proc. Natl. Acad. Sci. USA, 92: 1609-1613; Maruyama, I.N., et al. (1994) Proc. Natl. Acad. Sci. USA, 91:8273-8277; Dunn, I.S., (1995) J. Mol. Biol., 248:497-506; Rosenberg, A., et al. (1996) Innovations 6:1-6); Ren, Z.J., et al. (1996) Protein Sci., 5:1833-1843; Efimov, V.P., et al. (1995) Virus Genes 10:173-177; Duibecco, R., U.S. Patent No. 4,593,002; Ladner, R.C.., et al., U.S. Patent No. 5,837,500; Ladner, R.C., et al., U.S. Patent No. 5,223,409; Dower, et al., U.S. Patent No. 5,427,908; Russell et al., U.S. Patent No. 5,723,287; U U.S. Patent No. 6,190,856; and the application entitled "METHODS AND COMPOSITIONS FOR THE CONSTRUCTION AND USE OF ENVELOPE VIRUSES AS DISPLAY PARTICLES", filed August 2, 2001, serial number not yet assigned, all of which are expressly incorporated by reference.

In a preferred embodiment, the libraries are displayed on the surface of a bacterial cell as is described in WO 97/37025, which is expressly incorporated by reference in its entirety. In this embodiment, surface anchoring vectors are provided for the surface expression of genes encoding proteins of interest. At a minima, the vector includes a gene encoding an ice nucleation protein, a secretion signal a targeting signal and a gene of interest. Preferably, the bacterial host is a gram negative bacterium belonging to the genera Escherichia, Acetobacter, Pseudomonas, Xanthomonas, Erwinia, and Xymomonas. Advantages to using the ice nucleation protein as the surface anchoring protein are the high level of expression of the ice nucleation protein on the surface of the bacterial cell and its stable expression during the stationary phase of bacterial cell growth.

In a preferred embodiment, the libraries are displayed using yeast two hybrid systems as is described in Fields and Song (1989) Nature 340:245, which is expressly incorporated herein by reference. Yeast-based two-hybrid systems utilize chimeric genes and detect protein-protein interactions via the activation of reporter-gene expression. Reporter-gene expression occurs as a result of reconstitution of a functional transcription factor caused by the association of fusion proteins encoded by the chimeric genes. Preferably, the yeast two-hybrid system commercially available from Clontech is used to screen libraries for proteins that interact with a candidate proteins. See generally, Ausubel et al., Current Protocols in Molecular Biology, John Wiley & Sons, pp.13.14.1-13.14.14, which is expressly incorporated herein by reference.

In a preferred embodiment, the libraries are displayed using mammalian systems. For example, a cell-based display can be used to display large cDNA libraries in mammalian cells as described in Nolan, et al., U.S. Patent No. 6,153,380; Shioda , et al. U.S. Patent No. 6,251,676, both of which are expressly incorporated herein by reference.

**Screening of Libraries**

**High-throughput screening technology**

Fully robotic or microfluidic systems include automated liquid-, particle-, cell- and organism-handling including high throughput pipetting to perform all steps of experimental library generation, protein expression, and library screening. This includes liquid, particle, cell, and organism manipulations such as aspiration, dispensing, mixing, diluting, washing, accurate volumetric transfers; retrieving, and discarding of pipette tips; and repetitive pipetting of identical volumes for multiple deliveries from a single sample aspiration. These manipulations are cross-contamination-free liquid, particle, cell, and organism transfers. This instrument performs automated replication of microplate samples to filters, membranes, and/or daughter plates, high-density transfers, full-plate serial dilutions, and high capacity operation.

In addition, as will also be appreciated by those in the art, biochips may be part of the HTS system utilizing any number of components such as biosensor chips with protein arrays to measure protein-protein interactions or DNA-sensor chips to measure protein-DNA interactions. Microfluidic chip arrays (e.g., those commercially available from Caliper) may also be utilized in the context of automated HTS screening.

The automated HTS system used can include a computer workstation comprising a microprocessor programmed to manipulate a device selected from the group consisting of a thermocycler, a multichannel pipetter, a sample handler, a plate handler, a gel loading system, an automated transformation system, a gene sequencer, a colony picker, a bead picker, a cell sorter, an incubator, a light microscope, a fluorescence microscope, a spectrofluorimeter, a spectrophotometer, a luminometer, a CCD camera and combinations thereof.

**_in vivo_ screening**

In a preferred embodiment, the library is screened using _in vivo_ assay systems, including cell-based, tissue-based, or whole-organism assay systems. Cells, tissues, or organisms may be exposed to individual library members or pools containing several library members. Alternatively, host cells can be transformed or transfected with DNA encoding the library proteins and analyzed for phenotypic alterations.

To screen the library, experimental systems are developed in which the activity for the library protein of interest is coupled to an observable property. Typical observable properties include changes in absorbance, fluorescence, or luminescence. Screens may also monitor changes in properties such as cell morphology or viability.

For example, cell death or viability can be measured using dyes or immuno-cytochemical reagents (e.g. Caspase staining assay for apoptosis, Alamar blue for cell vitality) that specifically recognize either viable or inviable cells.

In an alternate cell death or viability assay, the cells are transformed or transfected with a receptor or binding partner protein responsive to the ligand represented by the library. The receptor may be coupled to a signaling pathway that causes cell death, allows cell survival, or triggers expression of a reporter gene. These readout modalities can be measured using dyes or immuno-cytochemical reagents that indicate cell death, cell vitality (e.g. Caspase staining assay for apoptosis, Alamar blue for cell vitality).

Alternatively, readout can be via a reporter construct. Reporter constructs may be proteins that are intrinsically fluorescent or colored, or proteins that modify the spectral properties of a substrate or binding partner. Common reporter constructs include the proteins luciferase, green fluorescent protein, and beta-galactosidase.

The assays described can also be performed by measuring morphological changes of the cells as a response to the presence of a library variant. These morphological changes can be registered using microscopic image analysis systems (e.g. Cellomics ArrayScan technology) such as those now available commercially.

*in vitro* screening

In a preferred embodiment, different physical and functional properties of the library members are screened in an *in vitro* assay. Properties of library members that may be screened include, but are not limited to, various aspects of stability (including pH, thermal, oxidative/reductive and solvent stability), solubility, affinity, activity and specificity. Multiple properties can be screened simultaneously (e.g. substrate specificity in organic solvents, receptor-ligand binding at low pH) or individually.

Protein properties can be assayed and detected in a wide variety of ways. Typical readouts include, but are not limited to, chromogenic, fluorescent, luminescent, or isotopic signals. These detection modalities are utilized in several assay methods Including, but not limited to, FRET (fluorescence resonance energy transfer) and BRET (bioluminescence resonance energy transfer) based assays, AlphaScreen (Amplified Luminescent Proximity Homogeneous Assay), SPA (scintillation proximity assay), ELISA (enzyme-linked immunosorbent assays), BIACORE (surface plasmon resonance), or enzymatic assays. *In vitro* screening may or may not utilize a protein fusion or a label.

**Selection of Libraries**

In an alternatively preferred embodiment, a selection method is used to select for desired library members. This is generally done on the basis of desired phenotypic properties, e.g. the protein properties defined herein. This is enabled by any method which couples phenotype and genotype, i.e. protein function with the nucleic acid that codes for it. In some cases this will be a "trans" effect rather than a "cis" effect In this way, isolation of library protein variants simultaneously enables isolation of its coding nucleic acid. Once isolated, the gene or genes encoding library protein can be purified ("rescued") and/or amplified. This process of isolation and amplification can be repeated, allowing favorable protein variants in the library to be enriched. Nucleic acid sequencing of the selected library members ultimately allows for identification of library members with desired properties.

Isolation of library protein can be accomplished by a number of methods. In some embodiments, only cells containing library protein variants with desired protein properties are allowed to survive or replicate. In alternate embodiments, the library protein and its genetic material are obtained by binding the library protein to another protein, RNA aptamer, or other molecule.

In one embodiment, the selection method is based on the use of specific fusion constructs. For example, if phage display is used, the library members are fused to the phage gene III protein.

In one embodiment selection is accomplished using a rescue fusion sequence, which forms a covalent or noncovalent link between the library member (phenotype) and the nucleic acid that encodes the library member (genotype). For example, in a preferred embodiment the rescue fusion protein binds to a specific sequence on the expression vector (see U.S.S.N. 09/642,574; PCT/US00/22906; U.S.S.N. 10/023,208; PCT/US01/49058; U.S.S.N. 09/792,630; U.S.S.N. 10/080,376; PCT/US02/04852; U.S.S.N. 09/792,626; PCT/US02/04853; U.S.S.N. 10/082,671; U.S.S.N. 09/953,351; PCTUS01/28702; U.S.S.N. 10/097,100; and PCT/US02/07466). and envelope virus (see U.S. S.N. 09/922,503 and PCT/US01/24535).

In an alternate embodiment, selection is accomplished using a display technology including, but not limited to phage display, in which the library members are fused to a protein such as the phage gene III protein, (see Kay, BK et al, eds. Phage display of peptides and proteins: a laboratory manual (Academic Press, San Diego, CA, 1996); Lowman HB, Bass SH, Simpson N, Wells JA (1991) Selecting high-affinity binding proteins by monovalent phage display. Bioechemistry 30:10832-10838; Smith GP Filamentous fusion phage: novel expression vectors that display cloned antigens on the virion surface. (1985) Science 228:1315-1317.) and its derivatives such as selective phage infection (see Malmborg AC, Soderlind E, Frost L, Borrebaeck CASelective phage infection mediated by epitope expression on F pilus. (1997) J Mol Biol 273:544-551.), selectively infective phage (see Krebber C, Spada S, Desplanq D, Krebber A, Ge L, Pluckthun A Selectively infective phage (SIP): a mechanistic dissection of a novel method to select for protein-ligand interactions. (1997) J Mol Biol 268:619-630.), and delayed infectivity panning (see Benhar I, Azriel R, Nahary L, Shaky S, Berdichevsky Y, Tamarkin A, Wels W (2000) Highly efficient selection of phage antibodies mediated by display of antigen as Lpp-OmpA' fusions on live bacteria. J Mol Biol 301:893-904.). Other display technologies, which could be used, include but are not limited to cell surface display (see Witrrup KD, Protein engineering by cell-surface display. Curr. Opin. Biotechnology 2001, 12:395-399) such as display on bacteria (see Georgiou G, Poetschke HL, Stathopoulos C, Francisco JA, Practical applications of engineering gram-negative bacterial cell surfaces. Trends Biotechnol. 1993 Jan;11(1):6-10; Georgiou G, Stathopoulos C, Daugherty PS, Nayak AR, Iverson BL, and Curtiss RR (1997) Display of heterologous proteins on the surface of microorganisms: from the screening of combinatorial libraries to live recombinant vaccines. Nature Biotechnol. 15, 29-34; Lee JS, Shin KS, Pan JG, Kim CJ. Surface-displayed viral antigens on Salmonella carrier vaccine. Nature Biotechnology, 2000, 18:645-648; Jun HC, Lebeault JM, Pan JG. Surface display of *Zymomonas mobilis levansucrase* by using the ice-nucleation protein of Pseudomonas syringae. Nat Biotechnol 1998, 16:576-80.), yeast (see Boder ET and Wittrup KD. Yeast surface display for directed evolution of protein expression, affinity, and stability. Methods Enzymol 2000, 328:430-44.; Boder ET, Wittrup KD: Yeast surface display for screening combinatorial polypeptide libraries. Nat Biotechnol 1997, 15:553-557), and mammalian cells (see Whitehom EA, Tate E, Yanofsky SD, Kochersperger L, Davis A, Mortensen RB, Yonkovich S, Bell K, Dower WJ, and Barrett RW (1995). A generic method for expression and use of "tagged" soluble versions of cell surface receptors. Bio/technology, 13, 1215-1219.), as well as *in vitro* display technologies such as polysome display (see Mattheakis LC, Bhatt RR, Dower WJ, Proc. Natl Acad Sci USA 1994, 91: 9022-9026; Hanes J and Pluckthun A Proc Natl Acad Sci USA 1997, 94:4937-4942.), ribosome display (see Hanes J and Pluckthun A Proc Natl Acad Sci USA 1997, 94:4937-4942), mRNA display (Roberts RW and Szostak JW Proc Natl Acad Sci USA 1997, 94, 12297-12302; Nemoto N, Miyamoto-Sato E, Husimi Y, Yanagawa H FEBS Lett. 1997, 414:405-408), and ribosome-inactivation display system (see Zhou J, Fujita S, Warashina M, Baba, T, Taira K J Am Chem Soc (2002), 124, 538-543.)

In an alternate embodiment, *in vitro* selection methods that do not rely on display technologies are used. These methods include but are not limited to periplasmic expression and cytometric screening (see Chen G, Hayhurst A, Thomas JG, Harvey BR, Iverson BL, Georgiou G: Isolation of high-affinity ligand-binding proteins by periplasmic expression with cytometric screening (PECS). Nat Biotechnol 2001, 19: 537-542), protein fragment complementation assay (see Johnsson N & Varshavsky A. Split Ubiquitin as a sensor of protein interactions in vivo. (1994) Proc Natl Acad Sci USA, 91: 10340-10344.) and the yeast two hybrid screen (see Fields S, Song 0: A novel genetic system to detect protein-protein interactions. Nature 1989, 340:245-246.) used in selection mode (see Visintin M, Tse E, Axelson H, Rabbitts TH, Cattaneo A: Selection of antibodies for intracellular function using a two-hybrid in vivo system. Proc Natl Acad Sci USA 1999, 96: 11723-11728.).

In an alternative embodiment, *in vivo* selection can occur if expression of the library protein imparts some growth, reproduction, or survival advantage to the cell. For example, if host cells transformed with a library comprising variants of an essential enzyme are grown in the presence of the corresponding substrate; only clones with a functional variant of the enzyme will survive. Alternatively, an advantage may be conferred if the library member comprises a growth or survival factor and the host cell expresses the appropriate receptor.

**Additional Characterization**

In a preferred embodiment, a library member or members isolated using some screening or selection method are further characterized. The library member(s) may be subjected to further biological, physical, structural, kinetic, and thermodynamic analysis. Thus, for example, a selected library variant may be subjected to physical-chemical characterization using gel electrophoresis, reversed-phase HPLC, SEC-HPLC, mass spectrometry (MS) including but not limited to LC-MS, LC-MS peptide mapping and the like, ultraviolet absorbance spectroscopy, fluorescence spectroscopy, circular dichroism spectroscopy, isothermal titration calorimetry, differential scanning calorimetry, surface plasmon resonance, analytical ultra-centrifugation, proteolysis, and cross-linking. Structural analysis employing X-ray crystallographic techniques and nuclear magnetic resonance spectroscopy are also useful. As is known to those skilled in the art, several of the above methods can also be used to determine the kinetics and thermodynamics of binding and enzymatic reactions. The biological properties of one or more library members, including pharmacokinetics and toxicity, can also be characterized in cell, tissue, and whole organism experiments.

**Expression vectors**

Using the nucleic acids of the present invention, which encode library members, a variety of expression vectors are made. The expression vectors may be either self-replicating extrachromosomal vectors or vectors which integrate into a host genome.

Nucieic acid is operably iinked when it is placed into a functional relationship with another nucleic acid sequence. For example, DNA for a presequence or secretory leader is operably linked to DNA for a polypeptide if it is expressed as a preprotein that participates in the secretion of the polypeptide; a promoter or enhancer is operably linked to a coding sequence if it affects the transcription of the sequence; or a ribosome binding site is operably linked to a coding sequence if it is positioned so as to facilitate translation However, enhancers do not have to be contiguous.

**Inclusion of control or regulatory sequences**

Generally, these expression vectors include transcriptional and translational regulatory nucleic acid operably linked to the nucleic acid encoding the library protein.

The transcriptional and translational regulatory nucleic acid will generally be appropriate to the host cell used to express the library protein, as will be appreciated by those in the art; for example, transcriptional and translational regulatory nucleic acid sequences from *Bacillus* are preferably used to express the library protein in *Bacillus*. Numerous types of appropriate expression vectors, and suitable regulatory sequences are known in the art for a variety of host cells.

In general, the transcriptional and translational regulatory sequences may include, but are not limited to, promoter sequences, ribosomal binding sites, transcriptional start and stop sequences, translational start and stop sequences, and enhancer or activator sequences. In a preferred embodiment, the regulatory sequences include a promoter and transcriptional start and stop sequences.

Promoter sequences include constitutive and inducible promoter sequences. The promoters may be naturally occurring promoters, hybrid or synthetic promoters. Hybrid promoters, which combine elements of more than one promoter, are also known in the art, and are useful in the present invention.

**Inclusion of a selectable marker(s)**

In addition, in a preferred embodiment, the expression vector contains one or more selectable genes or parts of selectable marker genes to allow the selection of transformed host cells containing the expression vector, and particularly in the case of mammalian cells, ensures the stability of the vector, since cells which do not contain the vector will generally die. Selection genes are well known in the art and will vary with the host cell used.

The bacterial expression vector may also include at least one selectable marker gene(s) to allow for the selection of bacterial strains that have been transformed. Suitable selectable gene(s) or parts of selectable marker genes, include genes, which render the bacteria resistant to drugs such as ampicillin, chloramphenicol, erythromycin, kanamycin, neomycin and tetracycline. Selectable markers also include biosynthetic genes, such as those in the histidine, tryptophan and leucine biosynthetic pathways.

**Inclusion of additional elements**

In a preferred embodiment, the expression vector contains a RNA splicing sequence upstream or downstream of the gene to be expressed in order to increase the level of gene expression. See Barret et al., Nucleic Acids Res. 1991; Groos et al., Mol. Cell. Biol. 1987; and Budiman et al., Mol. Cell. Biol. 1988.

In addition, the expression vector may comprise additional elements. For example, the expression vector may have two replication systems, thus allowing it to be maintained in two organisms, for example in mammalian or insect cells for expression and in a prokaryotic host for cloning and amplification. Furthermore, for integrating expression vectors, the expression vector contains at least one sequence homologous to the host cell genome, and preferably two homologous sequences which flank the expression construct. The integrating vector may be directed to a specific locus in the host cell by selecting the appropriate homologous sequence for inclusion in the vector. Such vectors may include *cre-lox* recombination sites, or *att*R, *att*B, *att*P, and *att*L sites. Constructs for integrating vectors and appropriate selection and screening protocols are well known in the art and are described in e.g., Mansour et al., *Cell,* 51:503 (1988) and Murray, *Gene Transfer and Expression Protocols, Methods in Molecular Biology, Vol. 7* (Clifton: Humana Press, 1991).

<u>Constructs</u>

**Targeting or signal sequences**

The expression vector may also include a signal peptide sequence that provides for secretion of the library protein in bacteria. The signal sequence typically encodes a signal peptide comprised of hydrophobic amino acids which direct the secretion of the protein from the cell, as is well known in the art. The protein is either secreted Into the growth media (gram-positive bacteria) or into the periplasmic space, located between the inner and outer membrane of the cell (gram-negative bacteria).

Thus, suitable targeting sequences include, but are not limited to, binding sequences capable of causing binding of the expression product to a predetermined molecule or class of molecules while retaining bioactivity of the expression product, (for example by using enzyme inhibitor or substrate sequences to target a class of relevant enzymes); sequences signaling selective degradation, of itself or co-bound proteins; and signal sequences capable of constitutively localizing the candidate expression products to a predetermined cellular locale, including a) subcellular locations such as the Golgi, endoplasmic reticulum, nucleus, nucleoli, nuclear membrane, mitochondria, chloroplast, secretory vesicles, lysosome, and cellular membrane; and b) extracellular locations via a secretory signal. Particularly preferred is localization to either subcellular locations or to the outside of the cell via secretion.

## ID (Purification) Tags

In a preferred embodiment, the library member comprises a rescue sequence operably linked to the rest of the peptide or protein. A rescue sequence is a sequence which may be used to purify or isolate either the candidate agent or the nucleic acid encoding it. Thus, for example, peptide rescue sequences include purification sequences such as polyhistidines, including but not limited to the $His_6$, and the like or other tag for use with $Ni^{+2}$ affinity columns and epitope tags for detection, immunoprecipitation or FACS (fluorescence-activated cell sorting). Suitable epitope tags include c-myc (for use with the commercially available 9E10 antibody), the BSP biotinylation target sequence of the bacterial enzyme BirA, flu tags, IacZ, and GST.

A rescue sequence could also be a nucleic acid sequence operably linked to an epitope in a covalently attached protein, or a protein that specifically recognizes the nucleic acid. Such sequences include, but are not limited to, most sequence specific RNA and DNA binding proteins, preferably those that recognize specific sequences or structures, and the like.

Alternatively, the rescue sequence may be a unique oligonucleotide sequence that serves as a probe target site to allow the quick and easy isolation of the construct, via PCR, related techniques, or hybridization.

In a preferred embodiment, rescue sequences could also be based upon in vivo recombination systems, such as the cre-lox system, the Invitrogen Gateway™ system, forced recombination systems in yeast, mammalian, plant, bacteria or fungal cells (for example WO 02/10183 A1), or phage display systems.

## Fusion constructs

The library protein may also be made as a fusion protein, using techniques well known in the art. Thus, for example, for the creation of monoclonal antibodies, if the desired epitope is small, the library protein may be fused to a carrier protein to form an immunogen. Alternatively, the library protein may be made as a fusion protein to increase expression, or for other reasons. For example, when the library protein is a library peptide, the nucleic acid encoding the peptide may be linked to other nucleic acid for expression purposes. Similarly, other fusion partners may be used, such as targeting sequences which allow the localization of the library members into a subcellular or extracellular compartment of the cell, rescue sequences or purification tags which allow the purification or isolation of either the library protein or the nucleic acids encoding them; stability sequences, which confer stability or protection from degradation to the library protein or the nucleic acid encoding it, for example resistance to proteolytic degradation, or combinations of these, as well as linker sequences as needed.

In a preferred embodiment, the fusion partner is a stability sequence to confer stability to the library member or the nucleic acid encoding it. Thus, for example, peptides may be stabilized by the incorporation of glycines after the initiation methionine (MG or MGG0), for protection of the peptide to ubiquitination as per Varshavsky's N-End Rule, thus conferring long half-life in the cytoplasm. Similarly, two prolines at the C-terminus impart peptides that are largely resistant to carboxypeptidase action. The presence of two glycines prior to the prolines impart both flexibility and prevent structure initiating events in the di-proline to be propagated into the candidate peptide structure. Thus, preferred stability sequences are as follows: $MG(X)_nGGPP$, where X is any amino acid and n is an integer of at least four.

## Labeling (isotopic, fluorescent, affinity)

In one embodiment, the library nucleic acids, proteins and antibodies of the invention are labeled. By "labeled" herein is meant that nucleic acids, proteins and antibodies of the invention have at least one element, isotope or chemical compound attached to enable the detection of nucleic acids, proteins and antibodies of the invention. In general, labels fall into three classes: a) isotopic labels, which may be radioactive or heavy isotopes; b) affinity labels, which may be antibodies or antigens; and c) colored or fluorescent dyes. The labels may be incorporated into the compound at any position.

## Expression systems

The library proteins of the present invention are produced by culturing a host cell transformed with nucleic acid, preferably an expression vector, containing nucleic acid encoding an library protein, under the appropriate conditions to induce or cause expression of the library protein. As outlined below, the libraries may be the basis of a variety of display techniques, including, but not limited to, phage and other viral display technologies, yeast, bacterial, and mammalian display technologies. The conditions appropriate for library protein expression will vary with the choice of the expression vector and the host cell, and will be easily ascertained by one skilled in the art through routine experimentation. For example, the use of constitutive promoters in the expression vector will require optimizing the growth and proliferation of the host cell, while the use of an inducible promoter requires the appropriate growth conditions for induction. In addition, in some embodiments, the timing of the harvest is important. For example, the baculoviral systems used in insect cell expression are lytic viruses, and thus harvest time selection may be crucial for product yield.

As will be appreciated by those in the art, the type of cells used in the present invention may vary widely. Basically, a wide variety of appropriate host cells may be used, including yeast, bacteria, archaebacteria, fungi, and insect and animal cells, including mammalian cells. Of particular interest are *Drosophila melanogaster* cells, *Saccharomyces cerevisiae* and other yeasts, *E. coli*, *Bacillus subtilis*, SF9 cells, C129 cells, 293 cells, Neurospora, BHK, CHO, COS, and HeLa cells, fibroblasts, Schwanoma cell lines, immortalized mammalian myeloid and lymphoid cell lines, Jurkat cells, mast cells and other endocrine and exocrine cells, and neuronal cells. See the ATCC cell line catalog, hereby expressly incorporated by reference. In addition, the expression of the secondary libraries in phage display systems, such as are well known in the art, are particularly preferred, especially when the secondary library comprises random peptides. In one embodiment, the cells may be genetically engineered, that is, contain exogenous nucleic acid, for example, to contain target molecules.

**Mammalian expression systems**

In a preferred embodiment, the library proteins are expressed in mammalian cells. Any mammalian cells may be used, with mouse, rat, primate and human cells being particularly preferred, although as will be appreciated by those in the art, modifications of the system by pseudotyping allows all eukaryotic cells to be used, preferably higher eukaryotes. As is more fully described below, a screen will be set up such that the cells exhibit a selectable phenotype in the presence of a random library member. As is more fully described below, cell types implicated in a wide variety of disease conditions are particularly useful, so long as a suitable screen may be designed to allow the selection of cells that exhibit an altered phenotype as a consequence of the presence of a library member within the cell.

Accordingly, suitable mammalian cell types include, but are not limited to, tumor cells of all types (particularly melanoma, myeloid leukemia, carcinomas of the lung, breast, ovaries, colon, kidney, prostate, pancreas and testes), cardiomyocytes, endothelial cells, epithelial cells, lymphocytes (T-cell and B cell) , mast cells, eosinophils, vascular intimal cells, hepatocytes, leukocytes including mononuclear leukocytes, stem cells such as haemopoietic, neural, skin, lung, kidney, liver and myocyte stem cells (for use in screening for differentiation and de-differentiation factors), osteoclasts, chondrocytes and other connective tissue cells, keratinocytes, melanocytes, liver cells, kidney cells, and adipocytes. Suitable cells also include known research cells, including, but not limited to, Jurkat T cells, NIH3T3 cells, CHO, COS, etc. See the ATCC cell line catalog, hereby expressly incorporated by reference.

Mammalian expression systems are also known in the art, and include retroviral systems. A mammalian promoter is any DNA sequence capable of binding mammalian RNA polymerase and initiating the downstream (3') transcription of a coding sequence for library protein into mRNA. A promoter will have a transcription-initiating region, which is usually placed proximal to the 5' end of the coding sequence, and a TATA box, usually located 25-30 base pairs upstream of the transcription initiation site. The TATA box is thought to direct RNA polymerase II to begin RNA synthesis at the correct site. A mammalian promoter will also contain an upstream promoter element (enhancer element), typically located within 100 to 200 base pairs upstream of the TATA box. An upstream promoter element determines the rate at which transcription is initiated and may act in either orientation. Of particular use as mammalian promoters are the promoters from mammalian viral genes, since the viral genes are often highly expressed and have a broad host range. Examples include the SV40 early promoter, mouse mammary tumor virus LTR promoter, adenovirus major late promoter, herpes simplex virus promoter, and the CMV promoter.

Typically, transcription termination and polyadenylation sequences recognized by mammalian cells are regulatory regions located 3' to the translation stop codon and thus, together with the promoter elements, flank the coding sequence. The 3' terminus of the mature mRNA is formed by site-specific post-translational cleavage and polyadenylation. Examples of transcription terminator and polyadenylation signals include those derived from SV40.

The methods of introducing exogenous nucleic acid into mammalian hosts, as well as other hosts, is well known in the art, and will vary with the host cell used. Techniques include dextran-mediated transfection, calcium phosphate precipitation, polybrene mediated transfection, protoplast fusion, electroporation, viral infection, encapsulation of the polynucleotide(s) in liposomes, and direct microinjection of the DNA into nuclei.

**Bacterial expression systems**

In a preferred embodiment, library proteins are expressed in bacterial systems. Bacterial expression systems are well known in the art.

A suitable bacterial promoter is any nucleic acid sequence capable of binding bacterial RNA polymerase and initiating the downstream (3') transcription of the coding sequence of library protein into mRNA. A bacterial promoter has a transcription initiation region which is usually placed proximal to the 5' end of the coding sequence. This transcription initiation region typically includes an RNA polymerase binding site and a transcription initiation site. Sequences encoding metabolic pathway enzymes provide particularly useful promoter sequences. Examples include promoter sequences derived from sugar metabolizing enzymes, such as galactose, lactose and maltose, and sequences derived from biosynthetic enzymes such as tryptophan. Promoters from bacteriophage may also be used and are known in the art. In addition, synthetic promoters and hybrid promoters are also useful; for example, the *tac* promoter is a hybrid of the *trp* and *lac* promoter sequences. Furthermore, a bacterial promoter may include naturally occurring promoters of non-bacterial origin that have the ability to bind bacterial RNA polymerase and initiate transcription.

In addition to a functioning promoter sequence, an efficient ribosome-binding site is desirable. In *E. coli*, the ribosome-binding site is called the Shine-Dalgarno (SD) sequence and includes an initiation codon and a sequence 3-9 nucleotides in length located 3 - 11 nucleotides upstream of the initiation codon.

**Baculovirus expression system**

In one embodiment, library proteins are produced in insect cells. Expression vectors for the transformation of insect cells, and in particular, baculovirus-based expression vectors, are well known in the art and are described e. g., in O'Reilly et al., *Baculovirus Expression Vectors: A Laboratory Manual* (New York: Oxford University Press, 1994).

**Yeast expression systems**

In a preferred embodiment, library protein is produced in yeast cells. Yeast expression systems are well known in the art, and include expression vectors for *Saccharomyces cerevisiae*, *Candida albicans* and *C. maltosa*, *Hansenula polymorpha*, *Kluyveromyces fragilis* and K *lactis*, *Pichia guillerimondii* and *P. pastoris*, *Schizosaccharomyces pombe,* and *Yarrowia lipolytica*. Preferred promoter sequences for expression in yeast include the inducible GAL1,10 promoter, the promoters from alcohol dehydrogenase, enolase, glucokinase, glucose-6-phosphate isomerase, glyceraldehyde-3-phosphate-dehydrogenase, hexokinase, phosphofructokinase, 3-phosphoglycerate mutase, pyruvate kinase, and the acid phosphatase gene. Yeast selectable markers include ADE2, HIS4, LEU2, TRP1, and ALG7, which confers resistance to tunicamycin; the neomycin phosphotransferase gene, which confers resistance to G418; and the CUP1 gene, which allows yeast to grow in the presence of copper ions.

**In Vitro Expression systems**

In one embodiment, the library proteins are expressed in vitro using cell-free translation systems. Several commercial sources are available for this system including but not limited to Roche Rapid Translation System, Promega TnT system, Novagen's EcoPro system, Ambion's ProteinSci pt-Pro system. In vitro translation systems derived from both prokaryotic (e.g. *E. coli*) and eukaryotic (e.g. Wheat germ, Rabbit reticulocytes) cells are available and may be chosen based on the expression levels and functional properties of the protein of interest. Both linear (as derived from a PCR amplification) and circular (as in plasmid) DNA molecules are suitable for such expression as long as they contain the gene encoding the protein operably linked to an appropriate promoter. Other features of the molecule that are important for optimal expression in either the bacterial or eukaryotic cells (including the ribosome binding site etc) are also included in these constructs. The proteins may again be expressed individually or in suitable size pools consisting of multiple library members. The main advantage offered by these in vitro systems is their speed and ability to produce soluble proteins. In addition the protein being synthesized may be selectively labeled if needed for subsequent functional analysis.

**Protein purification**

In a preferred embodiment, the library protein is purified or isolated after expression. Library proteins may be isolated or purified in a variety of ways known to those skilled in the art depending on what other components are present in the sample. Standard purification methods include electrophoretic, molecular, immunological and chromatographic techniques, including ion exchange, hydrophobic, affinity, and reverse-phase HPLC chromatography, and chromatofocusing. For example, the library protein may be purified using a standard anti-library antibody column. Ultrafiltration and diafiltration techniques, in conjunction with protein concentration, are also useful. For general guidance in suitable purification techniques, see Scopes, R., Protein Purification, Springer- Verlag, NY (1982). The degree of purification necessary will vary depending on the use of the library protein. In some instances no purification will be necessary.

Screening of library members

Library members may be screened using a variety of assays, including but not limited to in vitro assays, and in vivo assays such as cell-based, tissue-based, and whole-organism assays. Automation and high-throughput screening technologies may be utilized in the screening procedures.

**Cell-based assays — eukaryotic and prokaryotic**

In a preferred embodiment, the library is screened using ceii-based assay systems.

**In vivo selection of library variants**

Host cells transformed with a library representing variants of an enzyme or resistance factor of interest are grown in the presence of the corresponding substrate or antibiotic. Only clones with a functional variant of the enzyme or resistance factor will survive.

**Screening based on cell survival, cell death or expression of reporter genes in cells**

Cells are exposed to individual variants or pools of variants belonging to a library to be assayed. The cells are transformed or transfected either transiently or stably with the corresponding receptor responsive to the ligand represented by the library. The receptor is coupled to a signaling pathway that either causes cell death, cell survival, or triggers expression of a reporter gene. These readout modalities may be measured using dyes or immuno-cytochemical reagents that indicate cell death, cell vitality (e.g. Caspase staining assay for apoptosis, Alamar blue for cell vitality), or in case of the reporter constructs enzymes that convert dyes and cause them to be luminescent (e.g. luciferase) or shift their absorbance or fluorescent properties to wavelengths different from their properties before conversion.

**Screening based on cell survival of individual clones or clone pools**

Host cells are transformed or transfected with library DNA representing variants of a ligand or receptor of interest. The cells are also transformed or transfected either transiently or stably with the corresponding receptor responsive to the ligand represented by the library or in case of a receptor library with ligand signaling through the receptor represented by the library. The receptor is coupled to a signaling pathway that causes cell survival. If the sequence of the variant causing cell survival is not pre-identified, surviving cell clones may be used to identify the sequence identity of the corresponding variant.

**Screening based morphological changes of cells**

All of the above described assay readouts rely on changes that may be measured using absorbance, fluorescence or luminescence readers. The assays described may also be read measuring morphological changes of the cells as a response to the presence of a library variant. These morphological changes may be registered using microscopic image analysis systems (e.g. Cellomics ArrayScan technology) now avaiiabie commercially.

**Screening based on candidate bioactive agents**

Candidate agents are obtained from a wide variety of sources, as will be appreciated by those in the art, including libraries of synthetic or natural compounds. As will be appreciated by those in the art, the present invention provides a rapid and easy method for screening any library of candidate agents, including the wide variety of known combinatorial chemistry-type libraries.
In a preferred embodiment, candidate agents are synthetic compounds. Any number of techniques are available for the random and directed synthesis of a wide variety of organic compounds and biomolecules, including expression of randomized oligonucleotides. See for example WO 94/24314, hereby expressly incorporated by reference, which discusses methods for generating new compounds, including random chemistry methods as well as enzymatic methods. As described in WO 94/24314, one of the advantages of the present method is that it is not necessary to characterize the candidate bioactive agents prior to the assay; only candidate agents that bind to the target need be identified. In addition, as is known in the art, coding tags using split synthesis reactions may be done, to essentially identify the chemical moieties on the beads.

Alternatively, a preferred embodiment utilizes libraries of natural compounds in the form of bacterial, fungal, plant and animal extracts that are available or readily produced, and can be attached to beads as is generally known in the art

Additionaily, natural or synthetically produced libraries and compounds are readily modified through conventional chemical, physical and biochemical means. Known pharmacological agents may be subjected to directed or random chemical modifications, including enzymatic modifications, to produce structural analogs.

In a preferred embodiment, candidate bioactive agents include proteins, nucleic acids, and chemical moieties.

In a preferred embodiment, the candidate bioactive agents are proteins. In a preferred embodiment, the candidate bioactive agents are naturally occurring proteins or fragments of naturally occurring proteins. Thus, for example, cellular extracts containing proteins, or random or directed digests of proteinaceous cellular extracts, may be attached to beads as is more fully described below. In this way libraries of procaryotic and eucaryotic proteins may be made for screening against any number of targets. Particularly preferred in this embodiment are libraries of bacterial, fungal, viral, and mammalian proteins, with the latter being preferred, and human proteins being especiaiiy preferred.

In a preferred embodiment, the candidate bioactive agents are peptides of from about 2 to about 50 amino acids, with from about 5 to about 30 amino acids being preferred, and from about 8 to about 20 being particularly preferred. The peptides may be digests of naturally occurring proteins as is outlined above, random peptides, or "biased" random peptides. By"randomized" or grammatical equivalents herein is meant that each nucleic acid and peptide consists of essentially random nucleotides and amino acids, respectively. Since generally these random peptides (or nucleic acids, discussed below) are chemically synthesized, they may incorporate any nucleotide or amino acid at any position. The synthetic process can be designed to generate randomized proteins or nucleic acids, to allow the formation of all or most of the possible combinations over the length of the sequence, thus forming a library of randomized candidate bioactive proteinaceous agents. In addition, the candidate agents may themselves be the product of the invention; that is, a library of proteinaceous candidate agents may be made using the methods of the invention.

## High-throughput screening technology

Fully robotic or microfluidic systems include automated liquid-, particle-, cell- and organism-handling including high throughput pipetting to perform all steps of gene targeting and recombination applications. This includes liquid, particle, cell, and organism manipulations such as aspiration, dispensing, mixing, diluting, washing, accurate volumetric transfers; retrieving, and discarding of pipette tips; and repetitive pipetting of identical volumes for multiple deliveries from a single sample aspiration. These manipulations are cross-contamination-free liquid, particle, cell, and organism transfers. This instrument performs automated replication of microplate samples to filters, membranes, and/or daughter plates, high-density transfers, full-plate serial dilutions, and high capacity operation.

In addition, as will also be appreciated by those in the art, biochips may be part of the HTS system utilizing any number of components such as biosensor chips with protein arrays to measure protein-protein interactions or DNA-sensor chips to measure protein-DNA interactions. Microfluidic chip arrays (e.g., technology developed by Caliper) may also be utilized in the context of automated HTS screening.

The automated HTS system used may include a computer workstation comprising a microprocessor programmed to manipulate a device selected from the group consisting of a thermocycler, a multichannel pipetter, a sample handler, a plate handler, a gel loading. system, an automated transformation system, a gene sequencer, a colony picker, a bead picker, a cell sorter, an incubator, a light microscope, a fluorescence microscope, a spectrofluorimeter, a spectrophotometer, a luminometer, a CCD camera and combinations thereof.

## In vitro assays

in a preferred embodiment, different physical and functional properties of the library members are screened in an in vitro assay. In vitro assays allow a broader dynamic range for screening protein properties of interest that are not limited by cellular viability of the cells expressing the library members or library members acting upon other cells to exert its effects. Properties of library members that may be screened include, but are not limited to, various aspects of stability (including pH, thermal, oxidative/reductive and solvent stability), solubility, affinity, activity and specificity. Multiple properties may be screened simultaneously (e.g. substrate specificity in organic solvents, receptor-ligand binding at low pH) or Individually.

Protein properties may be assayed and detected in a wide variety of ways. Modality of detection could include, but are not limited to, chromogenic, fluorescent, luminescent, or isotopic substrates for protein library members. Any of these detection modalities are utilized in several assay methods including, but not limited to, FRET (fluorescence resonance energy transfer) and BRET (bioluminescence resonance energy transfer) based assays, AlphaScreen (Amplified Luminescent Proximity Homogeneous Assay), SPA (scintillation proximity assay), ELISA (enzyme-linked immunosorbent assays), or enzymatic assays.

## Additional characterization

In a preferred embodiment, a library member or members isolated from a cell positively selected for any number of protein properties by in-vivo or in-vitro screening methods well known to those in the art, are further characterized for said properties by aforementioned screens or other methods including physical, structural, kinetic, and thermodynamic analysis. Thus, for example, a selected library variant may be subjected to physical characterization through gel electrophoresis, reverse-phase HPLC, MS, LC-MS, RP-HPLC, SEC-HPLC, LC-MS peptide mapping, CD, analytical ultra-centrifugation, and proteolysis. Structural analysis employing X-ray crystallographic techniques, NMR, and cross-linking are also useful. In addition, thermodynamic and kinetic characterization of proteinaceous moieties are well known in the art.

## EXAMPLES

The following examples serve to more fully describe the manner of using the above-described invention, as well as to set forth the best modes contemplated for carrying out various aspects of the invention. it is understood that these examples in no way serve to limit the true scope of this invention, but rather are presented for illustrative purposes. All references cited herein are incorporated by reference.

## Example 1

## Computational Prescreening on β-lactamase TEM-1

Experiments were performed on the β-lactamase gene TEM-1. Brookhaven Protein Data Bank entry 1 BTL was used as the starting structure. All water molecules and the $SO_4^{2-}$ group were removed and explicit hydrogens were generated on the structure. The structure was then minimized for 50 steps without electrostatics using the conjugate gradient method and the Dreiding II force field. These steps were performed using the BIOGRAF program commercially available from Molecular Simulations, Inc., San Diego, CA. This minimized structure served as the template for all the protein design calculations.

## Computational Screening

Computational screening of sequences was performed using PDA™ technology. A 4 Å sphere was drawn around the heavy side chain atoms of the four catalytic residues (S70, K73, S130, and E166) and all amino acids having heavy side chain atoms within this distance cutoff were selected. This yielded the following 7 positions: F72, Y105, N132, N136, L169, N170, and K234. Two of these residues, N132 and K234, are highly conserved across several different β-lactamases and were therefore not included in the design, leaving five variable residue positions (F72, Y105, N136, L169, N170). These designed positions were allowed to change their identity to any of the 20 naturally occurring amino acids except proline, cysteine, and glycine (a total of 17 amino acids). Proline is usually not allowed since it is difficult to define appropriate rotamers for proline, cysteine is excluded to prevent formation of disulfide bonds, and glycine is excluded because of conformational flexibility.
Additionally, a second set of residues within 5 Å of the five residues selected for PDA™ technology design were floated (their amino acid identity was retained as wild type, but their conformation was allowed to change). The heavy side chain atoms were again used to determine which residues were within the cutoff. This yielded the following 28 positions: M68, M69, S70, T71, K73, V74, L76, V103, E104, S106, P107, I127, M129, S130, A135, L139. L148, L162, R164, W165, E166, P167, D179, M211, D214, V216, S235, I247. The two prolines, P107 and P167, were excluded from the floated residues, as were positions M69, R164, and W165, since their crystal structures exhibit highly strained rotamers, leaving 23 floated residues from the second set. Also, A248 was included instead of I247. The conserved residues N132 and K234 from the first sphere (4 Å) were also floated, resulting in a total of 25 floated residues.
The potential functions and parameters used in the PDA™ technology calculations were as follows. The van der Waals scale factor was set to 0.9, and the electrostatic potential was calculated using a distance dependent dielectric of ε=40 R. The well depth for the hydrogen bond potential was set to 8 kcal/mol with a local and remote backbone scale factor of 0.25 and 1.0 respectively. The solvation potential was only calculated for designed positions classified as core (F72, L169, M68, T71, V74, L76, I127, A135, L139, L148, L162, M211 and A248). Type 2 solvation was used (Street and Mayo, 1998). The non-polar exposure multiplication factor was set to 1.6, the non-polar burial energy was set to 0.048 kcal/mol/$A^2$, and the polar hydrogen burial energy was set to 2.0 kcal/mol.

The Dead End Elimination (DEE) optimization method (see reference) was used to find the lowest energy, ground state sequence. DEE cutoffs of 50 and 100 kcal/mol were used for singles and doubles energy calculations, respectively.

Starting from the DEE ground state sequence, a Monte Carlo (MC) calculation was performed that generated a list of the 1000 lowest energy sequences. The MC parameters were 100 annealing cycles with 1,000,000 steps per cycle. The non-productive cycle limit was set to 50. In the annealing schedule, the high and low temperatures were set to 5000 and 100 K respectively.

The following probability distribution was then calculated from the top 1000 sequences in the MC list (see Table 31 below). It shows the number of occurrences of each of the amino acids selected for each position (the 5 variable residue positions and the 25 floated positions).

Table 1:

| Monte Carlo analysis (amino acids and their number of occurrences at the designed positions resulting from the MC list of the 1000 lowest energy ranked sequences. | | | | | | | | |
|---|---|---|---|---|---|---|---|---|
| **POSITION** | **AMINO ACID: OCCURRENCES** | | | | | | | |
| 69 | M:1000 | | | | | | | |
| 70 | S:1000 | | | | | | | |
| 71 | T:1000 | | | | | | | |
| 72 | Y:591 | F:365 | V:35 | E:8 | L:1 | | | |
| 73 | K:1000 | | | | | | | |
| 74 | V:1000 | | | | | | | |
| 76 | L:1000 | | | | | | | |
| 103 | V:1000 | | | | | | | |
| 104 | E:1000 | | | | | | | |
| 105 | M:183 | Q:142 | I:132 | N:129 | E:126 | S:115 | D:97 | A:76 |
| 106 | S:1000 | | | | | | | |
| 127 | I:1000 | | | | | | | |
| 129 | M:1000 | | | | | | | |
| 130 | S:1000 | | | | | | | |
| 132 | N:1000 | | | | | | | |
| 135 | A:1000 | | | | | | | |
| 136 | D:530 | M:135 | N:97 | V:68 | E:66 | S:38 | T:33 | A:27 | Q:6 |
| 139 | L:1000 | | | | | | | |
| 148 | L:1000 | | | | | | | |
| 162 | L:1000 | | | | | | | |
| 166 | E:1000 | | | | | | | |
| 169 | L:698 | E:156 | M:64 | S:37 | D:23 | A:21 | Q:10 | | |
| 170 | M:249 N:24 | L:118 F:21 | E:113 K:15 | D:112 Y:9 | T:90 H:9 | Q:87 V:8 | S:66 | R:44 | A:35 |
| 179 | D:1000 | | | | | | | |
| 211 | M:1000 | | | | | | | |
| 214 | D:1000 | | | | | | | |
| 216 | V:1000 | | | | | | | |
| 234 | K:1000 | | | | | | | |
| 235 | S:1000 | | | | | | | |
| 248 | A:1000 | | | | | | | |

This probability distribution was then transformed into a rounded probability distribution (see Table 2). A 10% cutoff value was used to round at the designed positions and the wild type amino acids were forced to occur with a probability of at least 10%. An E was found at position 169 15.6% of the time. However, since this position is adjacent to another designed position, 170, its closeness would have required a more complicated oligonucleotide library design; E was therefore not included for this position when generating the sequence library (only L was used).

Table 2:

| PDA™ technology probability distribution for the designed positions of β-lactamase (rounded to the nearest 10%). | | | | | |
|---|---|---|---|---|---|
| POSITION | 72 | 105 | 136 | 169 | 170 |
| RESIDUE/PROBABILITY | Y 50% | M 20% | D 70% | L 100% | M 30% |
| | F 50% | Q 20% | M 20% | | L 20% |
| | I 20% | N 10% | | E 20% | |
| | N 10% | | | D 20% | |
| | E 10% | | | N 10% | |
| | S 10% | | | | |
| | Y 10% | | | | |

As seen from Table 2, the computational pre-screening resulted in an enormous reduction in the size of the problem. Originally, 17 different amino acids were allowed at each of the 5 designed positions, giving $17^5$ = 1,419,857 possible sequences. This was pared down to just 210 possible sequences - a reduction of nearly four orders of magnitude.

**Generation of Sequence Library**

Overlapping oligonucleotides corresponding to the full length TEM-1 gene for β-lactamase and all desired mutations were synthesized and used in a PCR reaction as described previously **(Figure 1),** resulting in a sequence library containing the 210 sequences described above.

Synthesis of mutant TEM-1 genes

To allow the mutation of the TEM-1 gene, pCR2.1 (commercially available from Invitrogen) was digested with Xbal and EcoRI, blunt ended with T4 DNA polymerase, and religated. This removes the HindIII and Xhol sites within the polylinker. A new Xhol site was then introduced into the TEM-1 gene at position 2269 (numbering as of the original pCR2.1) using a Quickchange Site-Directed Mutagenesis Kit as described by the manufacturer (commercially available from Stratagene). Similarly, a new HindIII site was Introduced at position 2674 to give pCR-Xen1.

To construct the mutated TEM-1 genes, overlapping 40-mer oligonucleotides were synthesized corresponding to the sequence between the newly introduced Xhol and HindIll sites, designed to allow a 20-nucleotide overlap with adjacent oligonucleotides. At each of the designed positions (72, 105, 136 and 170) multiple oligonucleotides were synthesized, each containing a different mutation so that all the possible combinations of mutant sequences (210) could be made in the desired proportions as shown in Table 3. For example, at position 72, two sets of oligonucleotides were synthesized, one containing an F at position 72, the other containing a Y. Each oligonucleotide was resuspended at a concentration of 1μg/μl, and equal molar concentrations of the oligonudeotides were pooled.

At the redundant positions, each oligonucleotide was added at a concentration that reflected the probabilities in Table 3. For example, at position 72 equal amounts of the two oligonucleotides were added to the pool, while at position 136, twice as much M-encoding oligonucleotide was added compared to the N-containing oligonucleotide, and seven times as much D-containing oligonucleotide was added compared to the N-containing oligonucleotide.

DNA library assembly

For the first round of PCR, 2 μl of pooled oligonucleotides at the desired probabilities (Table 3) were added to a 100 μl reaction that contained 2 μl 10 mM dNTPs, 10 μl 10x Taq buffer (commercially available from Qiagen), 1 μl of Taq DNA polymerase (5 units/μl: commercially available from Qiagen) and 2 μl Pfu DNA polymerase (2.5 units/μl: commercially available from Promega). The reaction mixture was assembled on ice and subjected to 94°C for 5 minutes, 15 cycles of 94°C for 30 seconds, 52°C for 30 seconds and 72°C for 30 seconds, and a final extension step of 72°C for 10 minutes.

Isolation of full-length oligonucleotides

For the second round of PCR, 2.5 µl of the first round reaction was added to a 100 µl reaction containing 2 µl 10 mM dNTPs, 10 µl of 10x Pfu DNA polymerase buffer commercially available from Promega, 2 µl Pfu DNA polymerase (2.5 units/µl: commercially available from Promega), and 1 µg of oligonucleotides corresponding to the 5' and 3' ends of the synthesized gene. The reaction mixture was assembled on ice and subjected to 94°C for 5 minutes, 20 cycles of 94DC for 30 seconds, 52°C for 30 seconds and 72°C for 30 seconds, and a final extension step of 72°C for 10 minutes to isolate the full length oligonucleotides.

Purification of DNA library

The PCR products were purified using a QIAquick PCR Purification Kit commercially available from Qiagen, digested with Xho1 and HindIII, electrophoresed through a 1.2 % agarose gel and re-purified using a QIAquick Gel Extraction Kit commercially available from Qiagen.

Verification of Sequence Library Identity

The PCR products containing the library of mutant TEM-1 β-lactamase genes were then cloned between a promoter and terminator in a kanamycin resistant plasmid and transformed into E. *coli.* An equal number of bacteria were then spread onto media containing either kanamycin or ampicillin. All transformed colonies will be resistant to kanamycin, but only those with active mutated β-lactamase genes will grow on ampicillin. After overnight incubation, several colonies were observed on both plates, indicating that at least one of the above sequences encodes an active β-lactamase. The number of colonies on the kanamycin plate far outnumbered those on the ampicillin plate (roughly a 5:1 ratio) suggesting that either some of the sequences destroy activity, or that the PCR introduces errors that yield an inactive or truncated enzyme.
To distinguish between these possibilities, 60 colonies were picked from the kanamycin plate and their plasmid DNA was sequenced. This gave the distribution shown in Table 3.

Table 3:

| Percentages predicted by PDA™ technology vs. those observed from experiment for the designed positions. | | | | | | | |
|---|---|---|---|---|---|---|---|
| Wild Type | PDA™ Technology Residues (Predicted Percentage/Observed Percentage) | | | | | | |
| 72F | Y50/50 | F50/50 | | | | | |
| 105Y | M20/27 | Q20/18 | I20/21 | N 10/7 | E 10/7 | S10/10 | Y10/10 |
| 136N | D70/72 | M20/17 | N10/11 | | | | |
| 170N | M 30/34 | L 20/21 | E 20/21 | D 20/17 | N 10/7 | | |

Note that the observed percentages of each amino acid at all four positions closely match the predicted percentages. Sequencing also revealed that only one of the 60 colonies contained a PCR error, a G to C transition.
This small test demonstrates that multiple PCR with pooled oligonucleotides may be used to construct a sequence library that reflects the desired proportions of amino acid changes.

**Experimental Screening of Sequence Library**

The purified PCR product containing the library of mutated sequences was then ligated into pCR-Xen1 that had previousiy been digested with Xho1 and HindIII and purified. The ligation reaction was transformed into competent TOP10 E. *coli* cells (Invitrogen). After allowing the cells to recover for 1 hour at 37°C, the cells were spread onto LB plates containing the antibiotic cefotaxime at concentrations ranging from 0.1 µg/ml to 50 µg/ml and selected for increasing resistance.
A triple mutant was found that improved enzyme function by 35-fold in only a single round of screening **(see Figure 4).** This mutant (Y105Q, N136D, N170L) survived at 50 µg/ml cefotaxime.

**Example 2**

**Secondary Library generation of a Xylanase**

PDA™ technology Screening Leads to Enormous Reduction in Number of Possible Sequences

To demonstrate that computational screening is feasible and will lead to a significant reduction in the number of sequences that have to be experimentally screened, calculations for the *B. circulans* xylanase with and without the substrate were performed. The PDB structures 1XNB of free *B. circulans* xylanase and 1 BCX for the enzyme substrate complex were used. 27 residues inside the binding site were visually identified as belonging to the active site. 8 of these residues were regarded as absolutely essential for the enzymatic activity. These positions were floated (see Figure 2). This means that they could change their side chain conformation but not their amino acid identity.

Three of the 20 naturally occurring amino acids were not considered (cysteine, proline, and glycine). Therefore, 17 different amino acids were still possible at the remaining 19 positions; the problem yields $17^{19}$ = 2.4 x$10^{23}$ different amino add sequences. This number is 10 orders of magnitude larger than what may be handled by state of the art directed evolution methods. Clearly these approaches cannot be used to screen the complete dimensionality of the problem and consider all sequences with multiple substitutions. Therefore PDA™ technology calculations were performed to reduce the sequence space. Starting from the PDA™ technology ground state a list of 10,000 low energy sequences was created by Monte Carlo and the probability for each amino acid at each position was determined (see Table 4).

Table 4:

| | Probability of amino acids at the designed positions resulting from the PDA™ technology calculation of the wild type (WT) enzyme structure. Only amino acids with a probability greater than 1 % are shown. | | | | | | |
|---|---|---|---|---|---|---|---|
| wr | PDA™ Technology Probability Distribution | | | | | | |
| 5 Y | W7.2% | F5.8% | Y2.9% | H4.0% | | | |
| 7 Q | E9.1% | L0.2% | | | | | |
| 11 D | I1.2% | D0.7% | V0.1% | M7.9% | L6.4% | E5.3% | T4.2% |
| | | Q3.8% | Y2.6% | F2.1% | N1.9% | S1.9% | A1.1% |
| 37 V | D9.9% | M9.4% | V1.4% | S2.8% | I4.1% | E1.0% | |
| 39 G | A9.8% | | | | | | |
| 63 N | W1.2% | Q6.7% | A1.4% | | | | |
| 65 Y | E1.7% | L4.9% | M3.4% | | | | |
| 67 T | E1.0% | D2.3% | L3.9% | A1.7% | | | |
| 71 W | V7.8% | F5.5% | W8.5% | M6.0% | D5.8% | E4.3% | I1.0% |
| 80 Y | M2.4% | L1.5% | F9.0% | I5.9% | Y5.7% | E3.7% | |
| 82 V | V8.6% | D1.0% | | | | | |
| 88 Y | N1.1% | K6.6% | W1.3% | | | | |
| 110 T | D9.9% | | | | | | |
| 115 A | A5.6% | Y7.8% | T4.4% | D10.2% | S9.2% | F2.6% | |
| 118 E | E2.2% | D2.6% | I2.0% | A1.7% | | | |
| 125 F | F9.4% | Y1.8% | M7.3% | L1.5% | | | |
| 129 W | E1.3% | S8.6% | | | | | |
| 168 V | D8.1% | A1.0% | | | | | |
| 170 A | A8.7% | S7.6% | D3.7% | | | | |

If we consider all the amino acids obtained from the PDA™ technology calculation, including those with probabilities less than 1%, we obtain $4.1 \times 10^{15}$ different amino acid sequences. This is a reduction by 7 orders of magnitude. If one only considers those amino acids that have at least a probability of more than 1% as shown in Table 1 (1% criterion), the problem is decreased to $6.6 \times 10^9$ sequences. If one neglects all amino acids with a probability of less than 5% (5% criterion) there are only $4.0 \times 10^8$ sequences left. This is a number that may be easily handled by screening and gene shuffling techniques. Increasing the list of low energy sequences to 100,000 does not change these numbers significantly and the effect on the amino acids obtained at each position is negligible. Changes occur only among the amino acids with a probability of less than 1%. Including the substrate in the PDA™ technology calculation further reduced the number of amino acids found at each position. If we consider those amino acids with a probability higher than 5%, we obtain $2.4 \times 10^6$ sequences (see Table 5).

Table 5:

| Probability of amino acids at the designed positions resulting from the PDA™ technology calculation of the enzyme substrate complex. Only those amino acids with a probability greater than 1% are shown. | | | | | | | | |
|---|---|---|---|---|---|---|---|---|
| WT | PDA™ | TECHNOLOGY PROBABILITY DISTRIBUTION | | | | | | |
| 5Y | Y69.2% | W17.0% | H 7.3% | F 6.0 % | | | | |
| 7Q | Q78.1% | E18.0% | L 3.9% | | | | | |
| 11D | D97.1% | | | | | | | |
| 37V | V50.9% | D33.9% | S5.4% | A 1.2% | L1.0% | | | |
| 39G | S80.6% | A19.4% | | | | | | |
| 63N | W92.2% | D 3.9% | Q 2.9% | | | | | |
| 65Y | E91.1% | L 8.7% | | | | | | |
| 67T | E92.8% | L 5.2% | | | | | | |
| 71W | W62.6% | E13.3% | M11.0% | S 6.9% | D4.0% | | | |
| 80Y | M66.4% | F13.6% | E10.7% | I 6.0% | L1.3% | | | |
| 82V | V86.0% | D12.8% | | | | | | |
| 88Y | W55.1% | Y15.9% | N11.4% | F 9.5% | K1.9% | Q1.4% | D1.4% | M1.4% |
| 5Y | Y69.2% | W17.0% | H 7.3% | F 6.0% | | | | |
| 110T | D99.9% | | | | | | | |
| 115A | D46.1% | S27.8% | T17.1% | A 7.9% | | | | |
| 118E | I47.6% | D43.0% | E 3.6% | V 2.5% | | A1.4% | | |
| 125F | Y51.1% | F43.3% | L 3.4% | M2.0% | | | | |
| 129W | L63.2% | M28.1% | E 7.5% | | | | | |
| 168V | D98.2% | | | | | | | |
| 170A | T92.3% | A 5.9% | | | | | | |

These calculations show that PDA™ technology may significantly reduce the dimensionality of the problem and may bring it into the scope of gene shuffling and screening techniques (see Figure 3).

**Example 3**

**Protocol for TNFα Library Expression and Purification**

Overnight culture preparation:

Competent Tuner(DE3)pLysS cells in 96 well-PCR plates were transformed with 1 ul of TNFa library DNAs and spread on LB agar plates with 34 g/ml chloramphenicol and 100 μg/ml ampicillin. After an overnight growth at 37°C, a colony was picked from each plate in 1.5 ml of CG media with 34 μg/ml chloramphenicol and 100 μg/ml ampicillin kept in 96 deep well block. The block was shaken at 250 rpm at 37°C overnight.

Expression:

Colonies were picked from the plate into 5 ml CG media (34 μg/ml chloramphenicol and 100 μg/ml ampicillin) in 24-well block and grown at 37°C at 250 rpm until OD600 0.6 were reached, at which time IPTG was added to each

well to 1μM concentration. The culture was grown 4 extra hours

Lysis:

The 24-well block was centrifuged at 3000 rpm for 10 minutes. The pellets were resuspended in 700 ul of lysis buffer (50 mM $NaH_2PO_4$, 300 mM NaCl, 10 mM imidazole). After freezing at -80°C for 20 minutes and thawing at 37°C twice, $MgCl_2$ was added to 10 mM, and DNase I to 75 μg/ml. The mixture was incubated at 37°C for 30 minutes.

$Ni^{2+}$ NTA column purification:

Purification was carried out following Qiagen Ni NTA spin column purification protocol for native condition. The purified protein was dialyzed against 1 X PBS for 1 hour at 4°C four times. Dialyzed protein was filter sterilized, using Millipore multiscreenGV filter plate to allow the addition of protein to the sterile mammalian cell culture assay later on.

Quantification:

Purified protein was quantified by SDS PAGE, followed by Coomassie stain, and by Kodak digital image densitometry.

TNFα Activity Assay:

The activity of variant TNFα protein samples were tested using Vybrant Assay Kit and Caspase Assay kit. Sytox Green nucleic acid stain is used to detect TNF-induced cell permeability in Actinomycin-D sensitized cell line. Upon binding to cellular nucleic acids, the stain exhibits a large fluorescence enhancement, which is then measured. This stain is excluded from live cells but penetrates cells with compromised membranes.
Caspase assay is a fluorimetric assay, which may differentiate between apoptosis and necrosis in the cells. This kit measures the caspase activity, triggered during apoptosis of the cells.
WEHI cells (Var-13 Cell Line from ATCC) were plated at $2.5 \times 10^5$ cells/mL, 24 hrs prior to the assay (100 μL/ well for the Sytox assay and 50 μL/well for the Caspase assay).

Table 6. Activity Assay Results for Sytox vs. Caspase

| Oligo trunk name | Conc. (ng/ul) | Clone # | (Sytox);01.25.01 % Activity Based on Std. Pt. | | | | Caspase 01.25.01 % Activity Based on Std. Pt. | | | |
|---|---|---|---|---|---|---|---|---|---|---|
| | | | Neat** | 1:10 | 1:100 | 1:1000 | Neat** | 1:10 | 1:100 | 1:1000 |
| N30D | 67.12 | 1 | 135 | 94 | 47 | 22 | 117 | 121 | 71 | 39 |
| K65E | 0.00 | 2 | 30 | 14 | 15 | 13 | 36 | 22 | 23 | 24 |
| G66Q | 16.55 | 3 | 122 | 35 | 19 | 15 | 107 | 50 | 28 | 25 |
| Q67W | 0.00 | 4 | 15 | 14 | 14 | 14 | 23 | 20 | 22 | 22 |
| K112D | 11.97 | 5 | 16 | 14 | 14 | 14 | 36 | 20 | 21 | 20 |

| | | | | | | | | | | |
|---|---|---|---|---|---|---|---|---|---|---|
| D143E | 23.46 | 6 | 21 | 13 | 14 | 13 | 21 | 17 | 16 | 17 |
| D143N | 0.00 | 7 | 16 | 13 | 13 | 13 | 38 | 31 | 26 | 27 |
| D143Q | 0.00 | 8 | 14 | 14 | 14 | 14 | 28 | 23 | 23 | 24 |
| D143S | 0.00 | 9 | 23 | 15 | 14 | 14 | 32 | 23 | 24 | 23 |
| A145R | 4.22 | 10 | 15 | 14 | 13 | 14 | 31 | 23 | 21 | 21 |
| A145K | 15.53 | 11 | 14 | 13 | 14 | 13 | 27 | 21 | 16 | 16 |
| A145E | 49.89 | 12 | 16 | 14 | 13 | 14 | 25 | 21 | 15 | 8 |
| E146K | 34.38 | 13 | 13 | 13 | 12 | 13 | 48 | 29 | 26 | 22 |
| E146R | 0.00 | 14 | 17 | 13 | 14 | 14 | 31 | 24 | 22 | 22 |
| K65E/D143K | 0.00 | 15 | 14 | 13 | 13 | 13 | 27 | 24 | 20 | 21 |
| K65E/D143R | 0.00 | 16 | 14 | 13 | 13 | 13 | 25 | 23 | 20 | 21 |
| WT1 | 17.10 | 17 | 129 | 101 | 58 | 27 | 86 | 94 | 59 | 36 |
| A84V | 34.60 | 18 | 14 | 12 | 13 | 14 | 35 | 18 | 15 | 17 |
| WT2 | 60.36 | 19 | 127 | 95 | 67 | 30 | 103 | 122 | 105 | 36 |
| WT3 | 38.54 | 20 | 133 | 97 | 61 | 28 | 98 | 109 | 84 | 34 |
| WT4 | 54.16 | 21 | 130 | 93 | 48 | 23 | 84 | 94 | 49 | 28 |
| WT5 | 31.68 | 22 | 133 | 96 | 69 | 30 | 94 | 93 | 69 | 33 |

**Neat\*\*: Normalized to 500 ng/mL**

Table 7.

| Activity Assay Results for Sytox | | | | | |
|---|---|---|---|---|---|
| WEHI TNF-a Activity Assay(%); (Sytox); 1.18.01 | | | | | |
| **Oligo Name** | **Conc.(ng/ul)** | **% Activity** | | | |
| | | **10** | **100** | **1000** | **10000** |
| N30Df | 51.40 | 108 | 100 | 99 | 70 |
| **K65Ef** | **0.00** | **85** | **60** | **31** | **45** |
| G66Qf | 50.99 | 90 | 88 | 57 | 58 |
| **Q67Wf** | **0.00** | **94** | **50** | **27** | **68** |
| K112Df | 14.06 | 17 | 15 | 15 | 42 |
| D143Ef | 35.86 | 25 | 15 | 13 | 21 |
| D143Nf | 38.52 | 14 | 13 | 14 | 26 |
| D143Qf | 19.28 | 14 | 13 | 14 | 18 |
| D143Sf | 45.91 | 19 | 14 | 12 | 75 |
| A145Rf | 41.18 | 14 | 11 | 11 | 15 |
| A145Kf | 55.47 | 13 | 12 | 12 | 81 |
| A145Ef | 53.29 | 20 | 13 | 12 | 64 |
| E146Kf | 33.28 | 12 | 12 | 11 | 17 |
| E146Rf | 32.22 | 12 | 11 | 12 | 21 |
| K65E/D143K | 8.39 | 34 | 18 | 15 | 19 |
| K65E/D143R | 9.97 | 16 | 15 | 14 | 81 |
| WT | 53.92 | 130 | 117 | 112 | 19 |
| A84V | 27.84 | 15 | 15 | 14 | 33 |

Table 8. Activity Assay Results Sytox vs. Caspase

| Oligo Name | Conc. (ng/ul) | WEHI TNF-a Activity Assay(%); (Sytox); 02.01.01 | | | | WEHI TNF-a Activity Assay(%); (Caspase); 02.01.01 | | | |
|---|---|---|---|---|---|---|---|---|---|
| | | Neat** | 1:10 | 1:100 | 1:1000 | Neat** | 1:10 | 1:100 | 1:1000 |
| G66Q | 29.18 | 122 | 61 | 30 | 18 | 90 | 48 | 35 | 30 |
| D143N | 18.92 | 13 | 11 | 13 | 12 | 37 | 25 | 25 | 24 |
| D143Q | 0.00 | 12 | 13 | 12 | 12 | 27 | 23 | 24 | 27 |
| D143S | 13.39 | 12 | 12 | 12 | 12 | 39 | 26 | 25 | 25 |
| A145R | 39.62 | 14 | 13 | 13 | 12 | 38 | 23 | 23 | 23 |
| A145K | 23.67 | 13 | 11 | 12 | 13 | 34 | 24 | 23 | 22 |
| E146R | 34.11 | 14 | 14 | 14 | 13 | 49 | 31 | 28 | 28 |
| K65E/D143K | 0.00 | 12 | 12 | 13 | 12 | 31 | 24 | 24 | 22 |
| K65E/D143R | 0.00 | 12 | 13 | 14 | 13 | 32 | 27 | 26 | 23 |
| WT | 0.00 | 149 | 116 | 68 | 32 | 96 | 80 | 39 | 27 |

*Neat**: Normalized to 500 ng/mL*

Figure __

Sytox Vs. Caspase; % Activitiy Based on Highest Std. Pt.

Data Analysis: Fluorescence vs. TNFα standard concentration was plotted to make a standard curve. Compare the fluorescence obtained from the highest point on the standard curve (5 ng/mL) to the fluorescence obtained from the unknown samples, to determine the % activity of the samples. The data may be analyzed using a four-parameter fit program to determine the 50% effective concentration for TNF ($EC_{50}$). % Activity of unknown samples = (Fluor. Of unknown samples/fluor. of 5 ng/mL std. Point) x 100.

**Example 4**

**PDA™ Technology Calculations for soluble TNF-R (p55)**

Using publicly available protein three-dimensional structures for the p55 TNFR (Protein Data Bank codes 1ext, 1ncf, 1 nr) both alone and complexed with its ligand, PDA™ technology was used to design optimized soluble p55 receptors as TNF-$\alpha$ antagonists. For the library shown below, the sequences shown were generated using PDA™ technology relative to the Protein Data Bank 1 ext numbering scheme. Amino acid residues known from the structure of the receptor-TNF$\alpha$ complex to be critical for p55 binding to TNF$\alpha$ were designed around. The results shown in Table 1 are an example of a library in which 15 positions from the wild-type p55 receptor were used for PDA™ technology design. Four of the positions chosen were nonpolar, 7 of the positions were charged, and 4 were polar. The library shown in Table 1 was pooled from five independent designs, and a 15% cutoff was applied for each position in the library. The size of the library for a single mutation is 78 and the entire library is 1.5 x $10^{10}$ sequences. The wild-type (WT) sequence is shown in the first line of the table. The mutation pattern for soluble p55 receptors at given position is shown in the remainder of the table.

Table 9.

|    | 54 | 56 | 57 | 59 | 62 | 65 | 67 | 68 | 69 | 70 | 95 | 97 | 98 | 101 | 103 |
|----|----|----|----|----|----|----|----|----|----|----|----|----|----|-----|-----|
| WT | N  | H  | L  | H  | S  | K  | R  | K  | E  | M  | H  | W  | S  | L   | Q   |
|    | V  | H  | L  | A  | A  | K  | V  | R  | A  | A  | K  | F  | S  | L   | I   |
|    |    |    |    | T  |    |    | L  | K  |    |    |    | A  |    | K   |     |
|    | E  |    | K  | E  | R  | R  | R  | D  | K  | M  | E  | T  | E  | F   |     |
|    |    |    |    | D  | Q  | Q  | K  |    | H  | D  | H  | D  | H  | R   | Y   |
|    |    |    |    | Q  |    |    | E  |    | W  |    |    |    |    |     | K   |
|    | N  | W  |    | R  | L  |    |    |    |    | E  |    |    |    |     |     |
|    |    | R  |    | Y  |    |    |    |    | S  |    |    | W  | W  |     |     |
|    |    | K  | F  | K  | N  |    |    |    |    |    |    |    |    |     | R   |
|    | F  | F  |    | F  |    |    |    |    | T  | L  |    | T  |    | Q   |     |
|    |    |    |    |    |    |    |    |    |    |    |    | K  |    |     |     |
|    |    |    |    |    |    |    |    |    |    |    |    | Q  |    |     |     |
|    |    |    |    | G  |    | Q  |    |    |    |    |    |    |    |     |     |
|    |    |    |    | S  |    |    |    |    |    |    |    |    |    |     |     |
|    |    |    |    | H  |    |    |    |    | E  |    |    |    |    |     | Q   |

**EXAMPLE 5**

**Computational Stabilization of Human Growth Hormone (hGH)**

Human Growth Hormone (hGH) was computationally redesigned to improve its thermostability. The computational design was performed using a previously developed combinatorial optimization algorithm based on the dead-end elimination theorem. The algorithm uses an empirical free energy function form scoring designed sequences. This function was augmented with a term that accounts for the loss of backbone and side chain conformational entropy. The weighting factors for this term, the electrostatic interaction term, and the polar hydrogen burial term were optimized by minimizing the number of mutations designed by the algorithm relative to wild-type. Forty-five residues in the core of the protein were selected for optimization with the modified potential function. The proteins designed using the developed scoring function contained six to ten mutations, showed enhancement in the melting temperature of up to 16°C, and were biologically active in cell proliferation studies. (See Filikov, et al, Computational stabilization of human growth hormone, Protein Science (2002), 11: 1452-1461, Cold Spring Harbor Laboratory Press, hereby expressly incorporated by reference in its entirety.)

**EXAMPLE 6**

**DEVELOPMENT OF A CYTOKINE ANALOG WITH ENHANCED STABILITY USING COMPUTATIONAL ULTRAHIGH THROUGHPUT SCREENING**

An ultra high throughput, computational screening method was used to improve the physico-chemical characteristics of Granulocyte-Colony Stimulating Factor (G-CSF). Residues in the buried core were selected for optimization to minimize changes to the surface, thereby maintaining the active site and limiting the designed protein's potential for antigenicity. Using a structure that was homology modeled from bovine G-CSF, core designs of 25-34 residues were completed, corresponding to $10^{21}$ - $10^{28}$ sequences screened. The optimal sequence from each design was selected for biophysical characterization and experimental testing; each having 10-14 mutations. The designed proteins showed enhanced thermal stabilities of up to 13 °C, displayed 5- to 10-fold improvements in shelf life, and were biologically active in cell proliferation assays and in a neutropenic mouse model. (See Luo, et al, Development of a cytokine analog with enhanced stability using ultrahigh computational throughput screening, Protein Science (2002), 11: 1218-1226, Cold Spring Harbor Laboratory Press, hereby expressly incorporated by reference in its entirety.)

**Claims**

1. A method executed by a computer under the control of a program, said computer including a memory for storing said program, said method comprising the steps of:

   (a) receiving a scaffold protein with residue positions;
   (b) selecting a collection of variable residue positions from said residue positions;
   (c) importing a set of coordinates for a scaffold protein, said scaffold protein comprising amino acid positions;
   (d) generating a set of optimized variant protein sequences comprising one or more variant amino acids; and,
   (e) applying a clustering algorithm to cluster said set into a plurality of subsets.

2. The method according to claim 1, further comprising applying a taboo search.

3. The method according to claim 1, wherein said clustering algorithm comprises a single-linkage clustering algorithm or a complete linkage clustering algorithm or an average linkage clustering algorithm.

4. The method according to claim 1, wherein said subsets are clustered according to sequence similarity or said subsets are clustered according to energetic similarity.

5. The method according to claim 1, wherein DNA shuffling is applied with said subsets to generate a library of optimized protein sequences.

6. The method according to claim 1, wherein said protein design cycle comprises protein design automation technology or said protein design cycle comprises the sequence prediction algorithm or said protein design cycle comprises a force field calculation.

7. The method according to any one of the preceding claims, wherein said subsets are used to generate secondary libraries comprising related sequences.

8. The method according to any one of the preceding claims, wherein after step (e) a library of said optimized protein sequences is generated.

9. The method according to claim 7 or claim 8, further comprising physically generating at least one member of said set of optimized protein sequences and experimentally testing said sequences for a desired function.

FIG._1

$E_{calc}$

PDA

Sequences

**FIG._2A**

$E_{calc}$

Sequences

**FIG._2B**

$E_{calc}$

Sequences

**FIG._3A**

$E_{true}$

Sequences

**FIG._3B**

$E_{calc}$

1st Simulation

*FIG._4A*

Sequences

$pE_{calc}$

2nd Simulation

*FIG._4B*

Sequences

Nearest Neighbor
(Single Linkage)

*FIG._5A*

Furthest Neighbor
(Complete Linkage)

*FIG._5B*

Average
(Only Shown
for 2 Cases)

*FIG._5C*

**Cluster Number**

A set of 300 protein sequences/structures was clustered using *single-linkage hierarchical clustering* and a root mean squared deviation (r.m.s.d) of energy matrix as the similarity criterion. In this case, the user chose to separate the sequences into twelve clusters. The energy of each sequence is displayed. Quality is represented by the homology of the designed sequence relative to natural WW proteins, using a PSSM derived from a multiple sequence alignment of natural WW sequences.

*FIG._6*

EP 1 510 959 A2

| Sequence # | Cluster (Energy Matrix r.m.s.d) | Sequence |
|---|---|---|
| 14 | 0 | SGDNGWTDKESADSRYKFYFNDQTNESTSSMPTN |
| 18 | 0 | SGPNGWMMMMLDSSNYWYYYDLLTNLSTSSEPAS |
| 54 | 0 | SLPAGWTDMELDSSNYKYYFNMLYQQSTETMPTS |
| 66 | 0 | SGDNGWTLMMLDSSNVMYYYNKETQLSTMSQPTS |
| 138 | 0 | SLPNGWMLMELNSARYMYYYNMLYNLSTNSMPTN |
| 156 | 0 | SGPNGWMLMEMDSSSYMFYYNMETQESTWSRPTS |
| | | |
| 112 | 1 | SGPAGWTWEMLQNADFWYTFNKIYEQMLEQMPTS |
| 119 | 1 | NGPSGWTWKMMDSSNVWYTFNDSTNTSTMSMPTN |
| 127 | 1 | SGPSGWMWMMLDNSQFYYTFNNETNTSLEQMPTN |
| 129 | 1 | SGPDGWTWKMMNDANYWYTFNMATQTATASMPND |
| 137 | 1 | NGPSGWTWKMMDSADFWYTFNDATNTMLEEMPTS |
| 141 | 1 | SGPPGWTWKMMDSADFWYTYNMATNEMTESMPND |
| 157 | 1 | SGPSGWAWKMLDNAQYWYTFNDLYQQSTSSMPND |
| 169 | 1 | SGPSGWMWMMAQSANFWFTYNLLTNTSLEQMPTS |
| 176 | 1 | SGPDGWTWMMLDSANFWYTFNKETQLALEQMPTN |
| 182 | 1 | SGPDGWMWKMMDSADFWFTFNDQTNTSLEQMPTS |
| 184 | 1 | NGPDGWAWMMLQNANYWYTYNNETNESTQEMPTS |
| 186 | 1 | SGPDGWTWKMMDNAQFWYTFNMATNTAQESMPND |
| 189 | 1 | SGPDGWLWKMLQNANYWYTFNDLTQLATESMPTN |
| 191 | 1 | NGPSGWNWMMMDNAQFWYTFNTIYQQMLEQMPTS |
| 193 | 1 | SGPPGWTWMMMNDANVWFTFNKLTEEATMSMPTN |
| 196 | 1 | SGPNGWTWMMLDSANYWYTYNMMYNLATASMPTS |
| 199 | 1 | AGPSGWTWMMLDSANYWFTYNMATNTATETMPTS |
| 203 | 1 | SGPDGWTWMMMQSANYWYMFNMATQETTASMPTS |
| 208 | 1 | SGPDGWAWMMLDNAQFWFMFNNETNTAQESMPTD |
| 211 | 1 | NGPDGWMWMKLDNADYWYTFNLLTNTATETMPTN |
| 213 | 1 | SGPSGWTWMMMQSANFWYTFNMATNTMLEQMPTS |
| 215 | 1 | NGPPGWTWMMLQSADYWYTFNMMSNTATASMPTS |
| 218 | 1 | SGPSGWNWMMLQSANYWFTFNTLYQQAQQEMPND |
| 220 | 1 | NGPSGWTWKMMDSAQFWYTFNDATDTMQEQMPTS |
| 222 | 1 | SGPDGWTWMKLDSADYWFTFNKLTNTAQASMPND |
| 224 | 1 | NGPDGWNWMMMDNANFWYTFNLATNTAQEEMPTS |
| 228 | 1 | SGPSGWAWMMLDNANFWFTFNNETNTSQSEMPND |
| 235 | 1 | SGPNGWMWMMLQNANFWYTFNNMYQQAQEQMPTN |
| 238 | 1 | SGPDGWTWMMLDNAQFWFTFNMETNTAQESMPND |
| 240 | 1 | SGPDGWMWKMMDNANYWYTFNNETNTMQAEMPTS |
| 242 | 1 | SGPDGWMWMMAQSADFWYTFNLATNTSQEQMPTS |
| 245 | 1 | NGPDGWTWMMLDNANFWFTFNMATNTSQESMPTS |
| 248 | 1 | SGPSGWTWMMMQSANFWYTFNMATQESTTSMPTS |
| 253 | 1 | SGPDGWTWMMADNADYWFTFNKLTNTAQAEMPTS |
| 257 | 1 | SGPPGWMWMMMQSANFWYTFNMATNESTEQMPTS |
| 259 | 1 | NGPSGWTWMMLQSANYWYTFNMLYQQMQAEMPTN |
| 262 | 1 | SGPDGWTWMMLDNADFWFTFNKLTNTAQESMPND |
| 269 | 1 | SGPPGWTWKMMDSANYWYTFNDLTNTAQASMPND |

## FIG. 74

| Sequence # | Cluster (Energy Matrix r.m.s.d) | Sequence |
|---|---|---|
| 271 | 1 | NGPSGWMWMMLDNANFWFTFNLATNTAQEQMPTS |
| 277 | 1 | SGPDGWTWMMMQSANFWYTFNMATNTMQEQMPTN |
| 279 | 1 | SGPSGWTWMMLQSANFWYTFNMATNTSQESMPND |
| 280 | 1 | NGPDGWMWKMMDNADFWYTFNNETNTAQESMPTS |
| 281 | 1 | SGPDGWTWMMLQSANYWYTFNMLTNTAQAEMPTS |
| 283 | 1 | SGPDGWMWKMMDNANFWYTFNDATNTAQESMPND |
| 285 | 1 | SGPDGWMWMMLQNANYWYTFNLATNTAQAEMPTS |
| 290 | 1 | SGPDGWMWMMLQNANFWYTFNLLTNTAQESMPND |
| 299 | 1 | SGPDGWMWMMLQNANFWYTFNLLTNTAQESMPTS |
| 300 | 1 | SGPDGWTWMMLQNANFWYTFNMLTNTAQESMPTS |
|  |  |  |
| 4 | 2 | SGPDGWTWMMANSSQLQYTFNKETNTSTMENPTN |
| 8 | 2 | NGPDGWMWMKLDSSSEEYTYNMATNESTKEEPTS |
| 10 | 2 | AGPNGWTWLEANSSQYKFTYDMLYQQSTSSEPTS |
| 31 | 2 | SLPSGWMWMMASDAQLEYTYNMMYNLATDQMPTS |
| 34 | 2 | SGPNGWTWMKNNSSEYWYTYDNETQQSTESNPTN |
| 44 | 2 | NGPDGWTWEMDQSSNRYYTFDKATEQATDTEPTS |
| 51 | 2 | AGPPGWTWLMLDNSDVLYTYNLETQTATMEEPTS |
| 56 | 2 | SGPDGWTWAKADSSELLYTYNNETQEATMSEPAS |
| 77 | 2 | SGPSGWTWMESNDSQTLYMFNNETQQTTMTMPTS |
| 79 | 2 | NLPSGWSWLKMDSASYEYTYNMSSNTATDSEPTS |
| 85 | 2 | SGPSGWTWEMTNNSQDMYMFNKSTMTSTKSMPTS |
| 88 | 2 | SGPAGWTMAMLQNADLRFAFNDATNTTTESDPAN |
|  |  |  |
| 1 | 3 | NGPDGWTWMMWQNASYEYTFNMLTNTAQWSMPTS |
| 121 | 3 | SGPPGWTWEMWQNADYWYTFNKMTEFWLWEMPTS |
| 135 | 3 | SGPSGWAWMMWQSANYWYTYNNETNTATWSMPND |
| 139 | 3 | SGPDGWTWEMWQNAQYYYTFNKMTNTMTWTMPTS |
| 143 | 3 | SGPSGWTWMMWQSANYWYTFNKIYEQMTWTMPTS |
| 155 | 3 | NGPSGWTWMMWQSANYWYTFNMATNTAQWEMPTS |
| 159 | 3 | AGPSGWNWMMWQSANYYYVFNTIYQWWQWSMPTS |
| 161 | 3 | SGPDGWTWMMMDSAQYWYTFNMLTDTMLWEMPTS |
| 163 | 3 | NGPAGWLWMMWQNANLWYTFNLATNTWTWSEPTS |
| 167 | 3 | NGPDGWTWMKWNSADYYYTFNKATNTMTWSMPTS |
| 180 | 3 | SGPPGWTWMMWDNANLWYTFNKATNTMTWTMPTS |
| 197 | 3 | NGPSGWMWKMWENADYYYTFNDLTNTMLWEEPTS |
| 201 | 3 | SGPSGWTWMMWDSADYWYTFNKMYEQMQWSMPND |
| 206 | 3 | SGPDGWTWKMWDSANYWYTFNDATNTWTWTMPTS |
| 230 | 3 | SGPNGWMWMMWDNAQYWYTFNMMTNTMQWSQPTS |
| 232 | 3 | SGPSGWMWMMWDNANYWYTFNLETNTMTWSMPND |
| 246 | 3 | SGPSGWNWMMWQSASYWYMFNTLYQQMTWTMPTS |
| 250 | 3 | NGPNGWMWMMWDSANYWYTFNKDTQQMTWTMPTN |
| 255 | 3 | SGPNGWTWMMWQSANYWYTFNMDTQQMTWSMPTS |
| 263 | 3 | NGPSGWTWMMWDSANYWYTFNMATNTMTWSMPTS |
| 265 | 3 | SGPDGWTWEMWQSANYWYTFNKATNTMTWTMPTS |

FIG. 7B

| Sequence # | Cluster (Energy Matrix r.m.s.d) | Sequence |
|---|---|---|
| 286 | 3 | SGPDGWTWMMWDNAQYWYTFNKLTNTMQWSMPND |
| 294 | 3 | SGPDGWTWMMWDNANYWYTFNMATNTMQWSMPTS |
| 298 | 3 | NGPDGWMWMMWDNANYWYTFNLLTNTMQWSMPTS |
|  |  |  |
| 93 | 4 | AGPNGWMWKMWDSASYEYTFNDQTDTATWSEPAS |
| 151 | 4 | NGPDGWTWMMMQNASVEYTFNMATNTATMSEPTS |
| 187 | 4 | NGPPGWTWEMWQSASYEYTFNKMTEQMLWEEPTS |
|  |  |  |
| 16 | 5 | NGPDGWTMEMMQNSQFMYAFNKMYEQTQSSDPTS |
| 21 | 5 | NGPPGWTMLMMQSAQVMYAYDMTTQQSTMSMPTN |
| 32 | 5 | SGPDGWLMMELDNSREMYAFNLLTQQSQWSMPTS |
| 47 | 5 | SGPSGWTMAMMDNADLWYSFNNETMLAQMEMPTS |
| 52 | 5 | SGPDGWLMMRMQSAETDYAFNDQTNETTDSMPTN |
| 68 | 5 | SGPDGWTMAELSNSQFKYAFNNMSQQSQSAMPTS |
| 82 | 5 | SGPNGWTMAELDNSRVMYAFNNETQQSTMTMPTS |
| 91 | 5 | NGPDGWNMMKMNSAEFMYAFNTMYEQTQEQQPTS |
| 100 | 5 | NGPNGWLMMMADSANYMYAYNDMYNLAQSSMPTS |
|  |  |  |
| 2 | 6 | SGPNGWMDEMMDSSNVKYYFNKDTQQTTMTMPTS |
| 5 | 6 | SLPSGWTMLMKQNADEWYYYNMMSNTSQWSEPAS |
| 7 | 6 | NGPAGWADKEMADARVKFYFNDQTQLATMTMPTN |
| 23 | 6 | SGPSGWADMELSSANYKYYFNNETNTTTDQMPTS |
| 28 | 6 | SGPDGWTDLMLDNSEYRYYYNMLYNWSQSSMPTS |
| 30 | 6 | NGPNGWTKEMMQSSDYWYYFNKASEQSTASEPAS |
| 33 | 6 | AGPSGWTDAMLDSANFKFYFNDSTMTTTESEPTS |
| 39 | 6 | SGPSGWTDLESENANYKYYYNLETQQSQSSMPAS |
| 43 | 6 | DGPSGWNLMEKSDAREMYYFNNETDTSQWSKPND |
| 48 | 6 | SGPDGWADMMAQSANYRFYYNDMYQQSTSSMPTS |
| 58 | 6 | SGPNGWTDMELDNSRYKYYFNMDTQQTTSSRPTS |
| 60 | 6 | NGPDGWAMMMLQSLNYWYYFNDATNESTWSEPAS |
| 64 | 6 | SGPDGWMNEEMQNSRFKYYFNKDTQQTQESMPTN |
| 74 | 6 | NGPNGWTDLELDNARYKFYYNKATNLSQSSMPTS |
| 76 | 6 | AGPDGWMNEMLQSAEVRYYYNALTNESTMSQPTS |
| 84 | 6 | SGPDGWMDMMLQSANFKYYFNMLTEQTQSAMPND |
| 86 | 6 | AGPNGWTKKKLDSAEYEYYYNMMSNESTWSEPTS |
| 89 | 6 | SGPPGWMDMELDSSNVKYYYNLLSNTSTMSMPTN |
| 94 | 6 | SGPEGWMDEMAQSSNYKYYYNKLYNLSQSARPTS |
| 96 | 6 | NGPPGWAMMMLNSSQFWFYFNNMTNESTESMPTN |
| 101 | 6 | SLPDGWMDMEWNSSQYKYYFNKDTQTMTWTMPTN |
| 105 | 6 | NLPPGWTDMEWPDARLKYYFNMMTNTAKWERPTS |
| 107 | 6 | SGPSGWMLMMLDSANVMYYYNMETQESTMTMPTS |
| 109 | 6 | DLPSGWLDMEKSNASEKYYFNLDTQQTTWSRPTN |
| 111 | 6 | NGPDGWTLKQMDSSQYMYYFNDLTNTMQWAEPTS |
| 113 | 6 | SLPSGWNDMELNSASYKYYFNTAYQLATSSMPTS |
| 118 | 6 | SLPDGWMDMELDNASFKFYYNLLYNWAQESMPTS |

FIG. 7C

72

| Sequence # | Cluster (Energy Matrix r.m.s.d) | Sequence |
|---|---|---|
| 120 | 6 | AGPNGWTDMMLESAEYRYYFNMDTQETTSSQPTS |
| 122 | 6 | DGPDGWADMELDSSQFKFYYNNETQQAQEAKPND |
| 124 | 6 | NLPNGWTLKMLDNANYMYYFNMATQQSTNSMPTN |
| 128 | 6 | NGPNGWNKMMWNSASYEYYYNMMTQEWTWSEPTS |
| 130 | 6 | SGPEGWMDMELDNSRFKFYFNMLYNLTQSEMPTS |
| 134 | 6 | SGPNGWTDMELDSASYKYYFNMLYQESTSSRPTS |
| 136 | 6 | SGPDGWMDMMMDNSEVRFYFNKDTQQTQMENPTS |
| 140 | 6 | AGPAGWMNMRADSSNYDFYFNNETQQAQSSQPTS |
| 144 | 6 | SGPNGWMLKKMDSSEVMFYFNNETNLATMSEPTS |
| 148 | 6 | SLPDGWTDMEADNSRYKYYFNKATEQSTSSMPTN |
| 154 | 6 | SGPDGWLDMEASNASYKYYFNLLTQLTTSSMPTS |
| 158 | 6 | NGPDGWTKMKLNSADVDYYFNKATNTTTMTEPTS |
| 160 | 6 | SGPNGWMDKEMDNSRFKFYYNNETNQATESMPTN |
| 162 | 6 | DGPPGWMLMKLNSADYMYYFNMMYEQTQSSKPND |
| 168 | 6 | DGPPGWADMELDNSRYKYYFNNMYQLAQSSKPND |
| 179 | 6 | NGPDGWMDMESSDARYKYYFNKDTQEATASRPTS |
| 181 | 6 | SGPSGWNKMMMQSASVEYYFNTLYEQSQMSEPTS |
| 185 | 6 | AGPSGWMLMKADSADYMYYFNLLYQQTTSSQPTS |
| 188 | 6 | SGPNGWMDMEMDSSSVKFYFNNETNESTMTRPTS |
| 200 | 6 | SGPDGWADMESSNAQYKYYFNLLTQESTSSMPTS |
| 209 | 6 | SGPSGWTDMEANSASYKYYFNMATQTSTASMPTS |
| 223 | 6 | SGPSGWTDAESSDAQYKYYFNNETQESTSSQPTS |
| 225 | 6 | AGPPGWMDMELQSASYKYYFNMMYEQTTSSRPTS |
| 229 | 6 | NGPDGWTDEEMQSARYKYYFNKLTEQSTSSMPTS |
|  |  |  |
| 3 | 7 | SGPSGWLNMELDSASFMFMYNLATNESTESMPTS |
| 6 | 7 | SGPPGWNWMKKDSSEYLYMFNTLYEQTTASMPTS |
| 12 | 7 | NGPDGWMKMMMDNSSVEYMYNLATNLSTMTMPTS |
| 19 | 7 | SGPDGWNNMRANDADYDYMFNNNTQESTASMPND |
| 20 | 7 | SGPSGWTWKEMDNSRFLYTFNDSSNTAQESNPTN |
| 24 | 7 | SGPDGWTWMKLDNSEFQYMYNKLYNLSQSSMPTS |
| 25 | 7 | NGPSGWMNEMAQSADYWYTMNTATETTTESEPAS |
| 35 | 7 | SGPPGWTNERMQSADVDYMFNKASNEATMTMPND |
| 36 | 7 | NGPDGWNWMMADNSSYEFTFNTLYQLTQAERPTD |
| 37 | 7 | SGPSGWMDMMLPDAQFMYVYNMMTNTSTSSMPTS |
| 40 | 7 | NGPDGWAKMMMQSSSVEYMFNDATQLATMTMPTS |
| 46 | 7 | SGPNGWTWMELNSSSFLYTFNLLTQESTTSNPAN |
| 50 | 7 | SGPEGWTNEESNDAQFKYMFNKLYQWSQSSMPND |
| 71 | 7 | SGPSGWTWKKADNADYWYTYNDLYNWAQAENPAN |
| 75 | 7 | SGPPGWLWKMADSSNFWYTFNDMYMFALEQMPND |
| 80 | 7 | SGPDGWMKMMLQSSNYWFMYNLLTNESTASMPAS |
| 83 | 7 | NGPSGWAWMKANSADYLFTYNDAYNLATESEPTS |
| 92 | 7 | SGPSGWTNMELDNSSYKFMYNKETNLSTASMPTS |
| 98 | 7 | SGPDGWTDEEMQSASYKYMFNKLTEQTTSSMPTS |
| 99 | 7 | NGPSGWTWMKLDNSEFYFTFNMATNQSTSEEPTS |

FIG. 7D

| Sequence # | Cluster (Energy Matrix r.m.s.d) | Sequence |
|---|---|---|
| 103 | 7 | SGPSGWNNMRLQSANYDYMYNLLYNTSTASMPTS |
| 106 | 7 | SGPNGWAWKMLQNSQFEYTFNDLYQLSTESMPTS |
| 116 | 7 | SGPNGWAKMKMNSADYDYMFNNMTQQSTASMPND |
| 125 | 7 | SGPSGWTWMKMDSSDVWFTYNLIYNLATMSMPTS |
| 152 | 7 | SGPEGWTNEMLNDSQYWYMFNKIYEQTQASMPND |
| 164 | 7 | SGPDGWTDMEMDSSSVKFMYNMLTNEATMTMPTN |
| 173 | 7 | SGPDGWTDMELNSSSYKFMYNMLTNESTESMPTN |
| 178 | 7 | SGPNGWMWMMADSSQYYFTYNLMYNQSQSEMPND |
|  |  |  |
| 9 | 8 | SGPSGWTMEMLQNANFMYAFNKETDTTQESMPND |
| 13 | 8 | SGPPGWNWMMLQSANFWYTFNTLTQTSQEQNPAN |
| 15 | 8 | SGPSGWTWMKLESAEYLYTYNMESQFMLWEMPTS |
| 17 | 8 | SGPSGWAWMMMDSASVEFTFNNATNTATMTQPTS |
| 27 | 8 | AGPAGWTWKMMDSANVYYTFNDQTNEATMSQPTS |
| 29 | 8 | SGPSGWNWMESNSASFLFTFNNETDTSLEQMPTN |
| 41 | 8 | AGPPGWMWKMLDSADFWFTFNNLSNTSLEQQPTN |
| 42 | 8 | SGPNGWTDMKWDNSEYRYMYNMMTNESTWSMPTS |
| 49 | 8 | NGPSGWTWMKKDSSEEYYTFNMASNTSTWSEPTS |
| 55 | 8 | NGPSGWNNMMMENAQFWYMFNTATNTTTESMPTS |
| 59 | 8 | SGPPGWMWMKNNSASFEFTYNMLTNFALEQMPTS |
| 61 | 8 | AGPSGWMWMEMDNASVLYTFNLESMTSQMEQPTS |
| 63 | 8 | SGPSGWNWMMLNDAQEYFTFNTLTNTATKENPTN |
| 65 | 8 | NGPAGWMWKKWDSADYWYTFNDATDTSLWQMPTS |
| 69 | 8 | SGPPGWNWMMKQSANELYTFNLATNTSTWTQPTS |
| 72 | 8 | SGPDGWAMMMLQSASYMYAFNNETQQSTWSMPTS |
| 87 | 8 | NGPDGWNWMMMDSSSEWYTFDNETQQAQKEMPTS |
| 90 | 8 | SGPSGWTWMMKSDAQELYTFNMDTQEATWTMPTS |
| 95 | 8 | SGPDGWTWMKMDNADVDYTFDMASQTTTMTMPTS |
| 97 | 8 | SGPSGWTWLMLNDASDEYTYNLETQTAQKSMPND |
| 102 | 8 | SGPAGWTWKMMENADFWYTFNNETNEMTEQEPAS |
| 104 | 8 | AGPDGWTWMMMDSSSYWFTFNKATEQSQWSNPND |
| 108 | 8 | SGPDGWTWKKMDSSDFWYTFNMATNTAQSEMPTN |
| 110 | 8 | SGPNGWMWMMWNSANYYFTYNNMTNQWTWSMPND |
| 117 | 8 | SGPSGWTWEMWQSLQYYYTFNKASNTMTWTQPTS |
| 123 | 8 | SGPAGWMWMKMNSASYEYTFNMLSNTSTETMPTS |
| 131 | 8 | AGPSGWLWMMLQSADYWYTYNLDTNTSTETQPTS |
| 132 | 8 | NGPDGWTNMKWESAEYMYMFNKATEQSTWSMPTN |
| 133 | 8 | SGPPGWMWKMMDNSQFYYTFNDMTNTMQEQNPTN |
| 145 | 8 | SGPDGWTWMMMPNASFEYTFNMATQTSLEQMPTS |
| 147 | 8 | NGPPGWNWMMLQSANYWYTYNMMTNEATASMPTS |
| 149 | 8 | SGPSGWLWMKNNSADFWFTYNLETNTSQEEMPTS |
| 153 | 8 | NGPPGWMWKKMDSADFYFTFNNMTNTSTEQEPTS |
| 165 | 8 | NGPSGWMWKMKDSAQYYYTFNDSTNTEQAEEPTS |
| 171 | 8 | NGPSGWTWMMMDNASVEYTFNKETNTTTMTMPTS |
| 174 | 8 | NGPAGWNWMMMENADYWYTFNTATEQSTQEEPTS |

FIG. 7E

EP 1 510 959 A2

| Sequence # | Cluster (Energy Matrix r.m.s.d) | Sequence |
|---|---|---|
| 194 | 8 | NGPNGWAWMKAQNADFYYVFNNETQQTTESMPTS |
| | | |
| 11 | 9 | SGPSGWLDMRWDSADYNYYFNNMYNTATWARPTD |
| 78 | 9 | SGPNGWNDMMWPNADYKYYFNTATETSQWENPTS |
| 115 | 9 | NGPPGWMMKMKDNANFWYYFNDLTDTEQSERPTS |
| 126 | 9 | SGPDGWTDEEWQSASYKYYFNKLTEQMQWENPAS |
| 142 | 9 | NGPDGWLDMELSNASYKYYFNLLTDTSQSERPTS |
| 146 | 9 | DGPEGWTDAMWDSAQYKYYFNDLYNWWQWSKPND |
| 150 | 9 | AGPNGWMLKMWDSANYMYYFNDLYQLMTWTQPTS |
| 166 | 9 | SGPNGWMMEMMQNANFWYYFNKDTQQSTESMPTN |
| 170 | 9 | SGPDGWTLKMWDSAQYMYYFNMATEQWTWSQPTS |
| 172 | 9 | DGPNGWMNEMLQSANFWYYFNKDYQLAQSSKPND |
| 175 | 9 | SGPSGWMDKEWDSASYKYYFNDSSNTWQWAMPTS |
| 177 | 9 | AGPSGWTLEKWQNADYMYYFNMLTNTSTWSQPTS |
| 183 | 9 | SGPNGWTDMEWNSSQYKYYFNMDTQLAQWSMPND |
| 190 | 9 | SGPAGWTNMRWNSADYDYYYNMASNTWQWSMPND |
| 192 | 9 | SGPDGWMLKELDSASYMYYFNDQTNTSQSENPTS |
| 195 | 9 | SGPDGWMDMEWDSSQYKYYFNMATNTWQWSMPND |
| 198 | 9 | SGPDGWLDMEMQSASFKYYFNNETQQSQESRPTS |
| 202 | 9 | NGPNGWMDKELDNSSFKFYFNNETNTAQSSQPTS |
| 204 | 9 | SGPPGWMLMKLNSADFMYYFNMLTNTAQEQMPTN |
| 205 | 9 | AGPNGWMDMELDNAQYKYYFNKDTQQSTSSQPTS |
| 207 | 9 | NGPSGWTDEMMQSADYKYYFNKLYEQTQSENPTS |
| 210 | 9 | DGPNGWTLEMWQSANYMYYFNKMYEQSQWSQPTS |
| 212 | 9 | SGPNGWADMELDSAQYKYYFNNETQQSQSSRPTS |
| 214 | 9 | DGPDGWMDKEWDNASYKFYFNLLTELWQWSKPND |
| 216 | 9 | SGPNGWMDEMMDNAQFKYYFNKDTQQSQESMPTS |
| 217 | 9 | SGPDGWMLMEADSARYMYYFNMATNTSTSSQPTS |
| 219 | 9 | SGPDGWMDMEWDNASYKYYFNNETNEMTWSRPTS |
| 221 | 9 | SGPNGWTLMMWQNANYMYYFNMDTQQMTWTMPTS |
| 226 | 9 | SGPNGWTLKMWDNAQYMYYFNDLTNTWQWSMPTN |
| 227 | 9 | SGPDGWMDMMMQSADVKYYFNLATQEATMSQPTS |
| 231 | 9 | SGPDGWTDLELNSASFKFYFNMATQQAQESNPTS |
| 233 | 9 | NGPDGWTLEMMQSADYMYYFNKLYEQSQSSMPTS |
| 234 | 9 | SGPPGWNDMEWDSAQYKYYFNMATNTMTWSRPTS |
| 236 | 9 | AGPDGWTDKEKDSASYKYYFNDLTNTEQSSQPTS |
| 237 | 9 | NGPSGWMNMMMQSANYWYYFNLATQESTSSMPTS |
| 239 | 9 | SGPSGWTDEEWQSASYKYYFNKLYEQMTWSQPTS |
| 241 | 9 | NGPNGWTDMELDSAQYKFYFNMDTQQATSSRPTS |
| 243 | 9 | SGPPGWTLEMWQNANYMYYFNKLYEQMQWSMPND |
| 244 | 9 | AGPSGWMMKMMDSAQYWYYFNDSTNTATASQPTS |
| 247 | 9 | SGPDGWMDMELDNAQYKYYFNNETNTAQSSRPTS |
| 249 | 9 | SGPDGWLLMKLDNADYMFYFNLLTNTAQSSMPND |
| 251 | 9 | AGPDGWTDKEMDNAQFKYYFNDATNTMQESQPTS |
| 252 | 9 | NGPNGWMMMMLQSANYWYYFNNETQESTSSEPTS |

FIG. 7F

75

| Sequence # | Cluster (Energy Matrix r.m.s.d) | Sequence |
|---|---|---|
| 254 | 9 | SGPSGWMDKEMDSASFKYYFNDATNTAQESKPND |
| 256 | 9 | NGPDGWLDMELDNAQYKFYFNLLTNTAQSSQPTS |
| 258 | 9 | SGPDGWMLKMWDNADYMYYFNNETNTAQWSMPND |
| 260 | 9 | SGPDGWMDKEMDNAQFKYYFNDATNTAQESQPTS |
| 261 | 9 | SGPNGWMNMMWQSANYWYYFNNETQQMTWTMPTS |
| 264 | 9 | SGPDGWMDMELDNAQYKYYFNLLTQQSQSSQPTS |
| 266 | 9 | SGPSGWMDMELDNASFKYYFNNMYQQAQESMPTN |
| 267 | 9 | NGPDGWTMMMQSANYWYYFNMLTNTSQSSEPTS |
| 268 | 9 | SGPDGWMDMELDNAQFKFYFNLATNTAQESRPTS |
| 270 | 9 | AGPDGWMDMMLQNADYKYYFNNETQQSQSSQPTS |
| 272 | 9 | SGPDGWTDEMWQSAQYKYYFNKLTNTMQWSMPTS |
| 273 | 9 | SGPDGWTMMMLQNANYWYYFNMATQQSQSSQPTS |
| 274 | 9 | NGPDGWMDKEMDSASFKYYFNDLTNTAQESMPTS |
| 275 | 9 | SGPSGWMDMMLQNAQYKYYFNLLYQQTQSSRPTS |
| 276 | 9 | NGPDGWMKMMWDNANYWYYFNNETNTAQWSEPTS |
| 278 | 9 | AGPDGWTDMELDNAQYKFYFNKLTNTAQSSQPTS |
| 282 | 9 | NGPSGWMDMELDNAQYKYYFNLLYQQSQSSQPTS |
| 284 | 9 | SGPDGWTDMMLQSADYKYYFNMLTNTSQSSMPTS |
| 287 | 9 | NGPDGWADMMLQSANFKYYFNNETNTAQESQPTS |
| 288 | 9 | SGPSGWNMMMWDNANYWYYFNTLYQQMQWSQPTS |
| 289 | 9 | SGPDGWTDMELDNAQYKYYFNMATNTSQSSMPTN |
| 291 | 9 | SGPDGWMDMEWDNASYKYYFNLATNTMQWSQPTS |
| 292 | 9 | NGPDGWTMMMLQNANYWYYFNMLTNTAQSSEPTS |
| 293 | 9 | SGPDGWTDEMLQNAQFKYYFNKLTNTAQESMPTS |
| 295 | 9 | SGPDGWMDMELDNAQYKYYFNLLTNTSQSSQPTS |
| 296 | 9 | NGPDGWMMMMLQNANFWYYFNMLTNTAQESEPTS |
| 297 | 9 | SGPDGWTDMMLQNANFKYYFNMATNTAQESQPTS |
| | | |
| 26 | 10 | SGPNGWMMMQKADSQEMFSFDNMTQQSTWTMPND |
| 62 | 10 | SGPNGWTDLMADSSEDRYMYNMMYNLSTKTMPND |
| | | |
| 22 | 11 | NGPNGWMWMMWNSLSYEFTFNKDTQFSLWEEPTS |
| 38 | 11 | SGPNGWTWMKWDSSEYMYTFNMDTNQSTWSEPTS |
| 45 | 11 | SGPAGWMMMMMDSAQYQFAYNKDSNTSTWTMPTS |
| 53 | 11 | SGDNGWMMWMMWNSASYEFTYNMMTNTSQWEQPTS |
| 57 | 11 | SGPSGWTMEMWQSADYRYAFNKSSDTSQWADPTN |
| 67 | 11 | AGPSGWTWLMVNSASLEFTYNMLYNTATQSEPAS |
| 70 | 11 | NGPNGWMDMKMDSSEYRFMFNMQTMLTTWSMPTN |
| 73 | 11 | SGPSGWMWMMMNSSQLEYTFNRDTNTTTMSEPTS |
| 81 | 11 | SGPPGWTWMMMDSAQFQYTFNKMTDTSQEENPTN |
| 114 | 11 | AGPDGWTWMKWDSSELEFTYNMETNESKWEEPTS |
| | | |
| | | |
| | | |
| | | |
| | | |

**FIG. 7G**

76

**1**         **3**         **9**

These two views of representative structures from cluster **1**, **3**, and **9** illustrate the relationship between clusters and couplings of amino acids.  In the upper set, it is clear that clusters **1** and **3** share a coupling pattern that is distinct from that of cluster **9**.  However, the bottom set reveals that on the opposite side of the protein, cluster **1** and **3** have a different coupling pattern.

*FIG._8*

**Designed Sequence Subfamilies**
300 Designed sequences for the SH3 template are clustered by comparing differences in interaction patterns. Note that the clusters have varying degrees of homology to the wild type SH3.

*FIG._9*

EP 1 510 959 A2

EP 1 510 959 A2

```
   KELVLALYDYQEKSPREVTMKKGDILTLLNSTNKDWWKVEVNDRQGFVPAAYVKKL

50 PEKALALSDYNRDADEKTSSQSGLDVDLLDISNNSYWRIKTNNAEGYKEAQLLNKL
50 AEQADAQANYSASANEKTTSYQGLMVTLLDISDNSFWIKTDENEGYKDAIYMAKN
50 KPKANALSNYNADSDQKASSQAGKDVDLLETSNESYWKIKIDNAEGYKDAQKMEPR
50 AAQALALSNYSSDANDKASAQSGLKVTLLDTSNDSYWKIQIEAREGFKDAIYLAPD

51 AEQADALADYSSDSDEKTSAQRGKIITLLETSNNTMIKVKVDEREGYKVADYTNPL
51 SEQALALSNFESNLNIMASAASGKVITLLDTSDNSWIKVKIDSLEGFISADYTNAL

52 QPLALADFDASAETETSMYAGLMVVLLNTENNEYIMIQTDTNQGYTLAILNDPK
52 PALALALSNYSSNTDIAASMAQGLVVVLLNTENNSYIKIKINDLEGFVPADLTNPL

53 PAKALALADYNASDNVKASSQSGLDIDLLDTQNNSYIKVKIDEREGYKDADKDRPD
53 NPTANAQADFQRSSDVEATAYQGLDIRLLDTSNNEYIRVEIDNRTGYKFAIYTATL

   KEKVNSLANFNASADIETSTYQGKDNDLLNKENNEWWKIKTDDDEGYTLAILLDLL
   PEKVNSLADYEASDNIKTSTYQGKDNDLLDKSNNEYWKIRTDNNEGFTEARLLDLL
54 PEKVLSLSNYNASDNIKTSTQQGKDNDLLNKENETLWKIRTDEREGFTEANLLRLD
54 PEKVNSLSNYNASDNIKTSTYQGKDNDLLDKSNNSYWKIKTDNNEGYTEARLLDLR
54 PEKVNSLSNYNASADIKTSTAQGKDNDLLDKSNDSYWKIRTDNNEGFTDANKLALI
54 SEQVDSQSNYNASLEEKTSTAAGKKNTLLDKSNNSYWKIQTDERQGYTDADKLALD
54 AEQVDSLSNYNADLDEKTSTQAGKLNTLLNAENQAMWKIRTDEREGYTEANLLNKI
54 KPKVNSLSYFNANSSAETSTQQGKDNDLLLASNEAWWMIKTENNEGYTLAILLPI
54 REMINSLSNYDRTADIMTSTAQGQNNVLLDKSNQAWWKIQTESLQGYVPANLTEPL
54 PQMIDSLSNFESSLNEETSTASGQKNVLLDKSNETMWKIKTENNEGYVSASLTNPL
```

FIG._10A

*FIG._10B*

FIG._10C

```
67  A Q Q V M S L S N F D R S S D I E T S T A Q G K L L T L L N A E D E S L A M V R V D S N E G F T S K S I L D L H
67  A E K V L A Q A N Y S A S A S D M A S S Q A G A D L D L L N K E D Q S Y A K V K I N D L E G F K S M S Y L A L N

68  E P K V N A E A D F E R S S D I E T S A Q R G K D L K L L D P Q N E A F I K V Q V D S D T G F K S A S F D R P R
68  S L Q V L A L S D Y E E S D N I K T T S Q S G A K L V A L Q T T N N S F I M V Q V D S D T G F K D A I Y T A A D
68  S L Q V K A Q S D Y N A D S E T K T S S Q S G A M L T A L D S S N E D Y T K V K V E N R E G Y K E K I L L A F D
68  P L Q V E A L A N Y N R S S D E M T T A Y A G Q M L T T L N S E N S D M T R V K V E S D E G Y K E M A L L R P V

69  K P K V N A D S Y Y N A S E E T A T T S E Q G A N L D L L N P E N Q D L I K V K V N D N E G Y K E A N L T S L I
69  N Q K V N A L A D Y S R N E D I A S S S Q S G A D L D L L E A S D Q T L I I V S I N N D T G K K S A T L T D P I

70  A E K V L A L A D Y D R S A D E K T S S Y Q G K D L D L L N K E N E T L W K V R V D S D E G F K E A N L L D L L
70  P E K V L A L A D Y E A S D N I K T S S A Q G K D L D L L D K S N N T L W K V R T D N N E G Y K E A I L L D L L
70  K E K V L A L A D Y N A S D N I K T S S Q Q G K D L D L L D K S N Q D Y W K V K V D N D E G F K E A I L L D L L
)   A E Q V L A L A D F D R K A D I E T S S Y Q G K D L T L L N K E N E S W W K V R V D S D E G F K L A E L L D L I
)   P E K V R A E A D F E A T A D I E T S S Q R G K D L D L L N K E N Q E W W M V Q T D E R Q G Y K L A I L L R L I
)   A Y L V L A Q S Y F D R S D S K E T P S Q Q G A L L I L L N A E D N S W W M V E V N D N Q G Y K L S E L L R L D
)   P E K V N S L S D F Q A S S E E T S S A A G Q D L D L L E A S S D S M W K V Q V N E R Q G F K A A E F L S P I
70  A P L V N A L A D Y D R T S E I E T T S Y S G Q N L V L L N A E N Q D L W K V Q V D Q R T G F K S A T F M D L I
70  Q P L V D A L S D F E A T S S E E T S S Q A G A N L V L L L P S N N S L W K V K V N D R T G Y K S A D Y M N P L
70  T E K V N A L S N Y D R S A D T M A T A Y S G Q D L A L L D P S N Q D M W K V R I N N L E G Y I E A A L L L L I

71  N E Q A L A L S D F N R S A N E M T S A Q Q G K V L K L L N I E N S S W W K V E V E N R T G F K S A S F L R K D
71  S L K V L A L S D Y S R D S D A K T T A Q A G K D L A A L N E E N A D Y W K V K V D N D E G Y K E A N L L N P I

72  N E K I N A L A D Y S A D A D E K T S S Q Q G Q D M E L L D K S N N S M W K V K V N D R E G F K E A R L L T D L L
72  K Q K I D S L S D Y D R S D D I K T T T Y Q G Q N N D L L L A S N N T L W K V K T E D R D G Y I E A N R A N L L
72  S Q K I K A D S D Y D R S A D E K T S A Y A G K D M I L L N A E N S D F W K V K V D N D E G F K E A I L T A P I
```

*FIG._10D*

FIG._10E

FIG._11

Structural Views of 7 Coupled Positions
Visual Inspection of Representative Cluster Members Reveal the Structural Basis for Coupling Patterns

Subfamily 54

Subfamily 66

Native

Subfamily 57

FIG._12

FIG._13

FIG._14

OLIGOS FOR POSITIONS   OLIGOS FOR POSITIONS   OLIGOS FOR POSITIONS

PCR PRIMER

WILD TYPE GENE OR GENE FOR GLOBAL MINIMA

PCR PRIMER

FIG. 16

EP 1 510 959 A2

WT

PDAScreen
35x increase

0.1 µg/ml          10 µg/ml          50 µg/ml

*FIG._15*

BLACK BOX = REGION TO BE MUTATED

F1     F2     F3     TEMPLATE DNA

R1     R2     R3

R4

STEP 1: SET UP 3 PCR REACTIONS:

PRODUCTS:

TUBE 1:

TUBE 2:

TUBE 3:

STEP 2: SET UP PCR REACTION WITH PRODUCTS OF TUBE 1 + PRODUCTS TUBE 2 + F1 + R4.

HEAT + ANNEAL PHASE OF PCR,

F1

R4

SYNTHESIS PHASE OF PCR,

AMPLIFICATION PHASE DURING SUBSEQUENT CYCLES USING F1 + R4.

F1

R1

STEP 3: REPEAT STEP 2 USING PRODUCT FROM STEP 2 + PRODUCT FROM STEP 1, TUBE 3 + PRIMERS F1 + R3.

## FIG._17

STEP 1: SET UP 3 PCR REACTIONS:

TUBE 1:

TUBE 2:

TUBE 3:

STEP 2: DIGEST PRODUCTS FROM STEP 1 WITH SUITABLE RESTRICTION ENDONUCLEASES.

STEP 3: LIGATE DIGESTED PRODUCT FROM STEP 2, TUBE 2 WITH DIGESTED PRODUCT FROM STEP 2, TUBE 1.

STEP 4: AMPLIFY VIA PCR LIGATED PRODUCTS OF STEP 3 WITH F1 + R4.

STEP 5: DIGEST AMPLIFIED PRODUCT OF STEP 4 WITH RESTRICTION ENDONUCLEASE #2.

STEP 6: LIGATE PRODUCT FROM STEP 5 WITH PRODUCT FROM STEP 2, TUBE 1.

STEP 7: AMPLIFY PRODUCT FROM STEP 6 WITH F1 + R3.

*FIG._18*

DIAGRAM 3

*FIG. 19*

FIG._20A

Amplification    Scheme & Math    Amplification Scheme Based on M13 Single Stranded Template

Primer 1

M13 ssDNA

PCR Cycle1

Primer 2

Undesired Product (1)

Undesired Residual Template + Product (1)

PCR Cycle2

Undesired Product (2)

Desired Product (1)

Undesired Residual Template + Product (1)

PCR Cycle3

Undesired Product (3)

Desired Product (4)

Undesired Residual Template + Product (1)

PCR Cycle4

EP 1 510 959 A2

## Amplification Scheme Based on M13 Single Stranded Template

Undesired Product (4)

Desired Product (11)

PCR Cycle5

Undesired Residual Template + Product (1)

### Numerical Progression of Desired Product with Increasing PCR Cycles

| PCR Cycles | Desired Product | Undesired Products and Residual Template | Percent Desired Product in Total Product |
|---|---|---|---|
| 1 | | 1 | |
| 2 | 0 | 2 | 0.00% |
| 3 | 1 | 3 | 25.00% |
| 4 | 4 | 4 | 50.00% |
| 5 | 11 | 5 | 68.75% |
| 6 | 26 | 6 | 81.25% |
| 7 | 57 | 7 | 89.06% |
| 8 | 120 | 8 | 93.75% |
| 9 | 247 | 9 | 96.48% |
| 10 | 502 | 10 | 98.05% |
| 11 | 1013 | 11 | 98.93% |
| 12 | 2036 | 12 | 99.41% |
| 13 | 4083 | 13 | 99.68% |
| 14 | 8178 | 14 | 99.83% |
| 15 | 16369 | 15 | 99.91% |
| 16 | 32752 | 16 | 99.95% |
| 17 | 65519 | 17 | 99.97% |
| 18 | 131054 | 18 | 99.99% |
| 19 | 262125 | 19 | 99.99% |
| 20 | 524268 | 20 | 100.00% |

*FIG._20B*

EP 1 510 959 A2

# FIG._21A  Computational Primary Library Followed by Experimental Secondary Library

| Starting Scaffold Protein | Wild-type Protein(s) | Wild-type Protein (s) | Wild-type Protein (s) | Wild-type Protein (s) | Wild-type Protein (s) | Wild-type Protein (s) | Wild-type Protein (s) | Wild-type Protein (s) | Wild-type Protein (s) |
|---|---|---|---|---|---|---|---|---|---|
| Primary Library Generation | PDA$^{TM}$ ± Homology | Homology Modeling / Alignment: a) Sequence b) Structure | SPA | SPA with Taboo Constraints ± Clustering | SPA with Clustering Algorithm | PDA$^{TM}$ with Taboo Constraints ± Clustering | PDA$^{TM}$ with Clustering Algorithm | SCMF and / or Probability Table | In Silico Shuffling |
| Secondary Library Generation | Gene Assembly Procedure | Gene Assembly Procedure | Gene Assembly Procedure | Gene Assembly Procedure | Gene Assembly Procedure | Gene Assembly Procedure | Gene Assembly Procedure | Gene Assembly Procedure | Gene Assembly Procedure |

# FIG._21B  Computational Primary Library Followed by Experimental Secondary Library

| Starting Scaffold Protein | Variant or Chimeric Protein(s) | Variant or Chimeric Protein(s) | Variant or Chimeric Protein(s) | Variant or Chimeric Protein(s) | Variant or Chimeric Protein(s) | Variant or Chimeric Protein(s) | Variant or Chimeric Protein(s) | Variant or Chimeric Protein(s) | Variant or Chimeric Protein |
|---|---|---|---|---|---|---|---|---|---|
| Primary Library Generation | PDA$^{TM}$ ± Homology | Homology Modeling / Alignment: a) Sequence b) Structure | SPA | SPA with Taboo Constraints ± Clustering | SPA with Clustering Algorithm | PDA$^{TM}$ with Taboo Constraints ± Clustering | PDA$^{TM}$ with Clustering Algorithm | SCMF and / or Probability Table | In Silico Shuffling |
| Secondary Library Generation | Gene Assembly Procedure | Gene Assembly Procedure | Gene Assembly Procedure | Gene Assembly Procedure | Gene Assembly Procedure | Gene Assembly Procedure | Gene Assembly Procedure | Gene Assembly Procedure | Gene Assembly Procedure |

EP 1 510 959 A2

## FIG._21C Computational Primary Library Followed by Computational Secondary Library

| Starting Scaffold Protein | Wild-type Protein(s) | Wild-type Protein(s) | Wild-type Protein(s) | Wild-type Protein(s) | Wild-type Protein(s) | Wild-type Protein(s) | Wild-type Protein(s) | Wild-type Protein(s) | Wild-type Protein(s) |
|---|---|---|---|---|---|---|---|---|---|
| Primary Library Generation | PDA$^{TM}$ ± Homology | Homology Modeling: a) Sequence b) Structure | SPA | SPA with Taboo Constraints ± Clustering | SPA with Clustering Algorithm | PDA$^{TM}$ with Taboo Constraints ± Clustering | PDA$^{TM}$ with Clustering Algorithm | SCMF and / or Probability Table | PDA$^{TM}$ |
| Secondary Library Generation | PDA$^{TM}$ | PDA$^{TM}$ | PDA$^{TM}$ | PDA$^{TM}$ | PDA$^{TM}$ | PDA$^{TM}$ | PDA$^{TM}$ | PDA$^{TM}$ | In Silico Shuffling |
| Optional Tertiary Library Generation | Gene Assembly Procedure | Gene Assembly Procedure | Gene Assembly Procedure | Gene Assembly Procedure | Gene Assembly Procedure | Gene Assembly Procedure | Gene Assembly Procedure | Gene Assembly Procedure | Gene Assembly Procedure |

## FIG._21D Computational Primary Library Followed by Computational Secondary Library

| Starting Scaffold Protein | Variant or Chimeric Protein(s) | Variant or Chimeric Protein(s) | Variant or Chimeric Protein(s) | Variant or Chimeric Protein(s) | Variant or Chimeric Protein(s) | Variant or Chimeric Protein(s) | Variant or Chimeric Protein(s) | Variant or Chimeric Protein(s) | Variant or Chimeric Protein(s) |
|---|---|---|---|---|---|---|---|---|---|
| Primary Library Generation | PDA$^{TM}$ ± Homology | Homology Modeling: a) Sequence b) Structure | SPA | SPA with Taboo Constraints ± Clustering | SPA with Clustering Algorithm | PDA$^{TM}$ with Taboo Constraints ± Clustering | PDA$^{TM}$ with Clustering Algorithm | SCMF and / or Probability Table | PDA$^{TM}$ |
| Secondary Library Generation | PDA$^{TM}$ | PDA$^{TM}$ | PDA$^{TM}$ | PDA$^{TM}$ | PDA$^{TM}$ | PDA$^{TM}$ | PDA$^{TM}$ | PDA$^{TM}$ | In Silico Shuffling |
| Optional Tertiary Library Generation | Gene Assembly Procedure | Gene Assembly Procedure | Gene Assembly Procedure | Gene Assembly Procedure | Gene Assembly Procedure | Gene Assembly Procedure | Gene Assembly Procedure | Gene Assembly Procedure | Gene Assembly Procedure |

EP 1 510 959 A2